# EUROPEAN PATENT SPECIFICATION

(11) **EP 0 915 832 B1**
(45) Date of publication and mention of the grant of the patent: **02.10.2002**
(21) Application number: 97935178.0
(22) Date of filing: 24.07.1997
(51) Int. Cl.: C07C 233/49, G01N 33/53, C07C 259/10, C07F 5/02

(54) **BORONIC COMPOUND COMPLEXING REAGENTS AND HIGHLY STABLE COMPLEXES**
KOMPLEXBILDNER FÜR BORONVERBINDUNG UND HOCHSTABILE KOMPLEXE
REACTIFS PERMETTANT DE COMPLEXER DES COMPOSES DE BORE ET COMPLEXES D'UN GRANDE STABILITE

(30) Priority: 05.08.1996 US 691930; 05.08.1996 US 689283
(43) Date of publication of application: 19.05.1999
(73) Proprietor: PROLINX, INC., Bothell, WA 98021 (US)
(72) Inventor: STOLOWITZ, Mark, L., Woodinville, WA 98072 (US); KAISER, Robert, J., Bothell, WA 98011 (US); LUND, Kevin, P., Lynnwood, WA 98037 (US)
(74) Representative: Gros, Florent
(86) International application number: US9713314
(87) International publication number: WO98005627

(56) References cited:
- WO-A-95/20591
- US-A- 3 293 252

## Description

### FIELD OF THE INVENTION

The present invention relates to the field of bioconjugate preparation, and more particularly, to a class of boronic compound complexing reagents useful for the conjugation of biological macromolecules, and the method of making and using such reagents.

### BACKGROUND OF THE INVENTION

Bioconjugation is a descriptive term for the joining of two or more different molecular species by chemical or biological means, in which at least one of the molecular species is a biological macromolecule. This includes, but is not limited to, conjugation of proteins, peptides, polysaccharides, hormones, nucleic acids, liposomes and cells, with each other or with any other molecular species that add useful properties, including, but not limited to, drugs, radionuclides, toxins, haptens, inhibitors, chromophores, fluorophores, ligands, etc. Immobilization of biological macromolecules is also considered a special case of bioconjugation in which the macromolecule is conjugated, either reversibly or irreversibly, to an insoluble support. Bioconjugation is utilized extensively in biochemical, immunochemical and molecuar biological research. Major applications of bioconjugation include; detection of gene probes, enzyme-linked immuno solid-phase assay, monoclonal antibody drug targeting and medical imaging.

Bioconjugates are generally classified as either direct or indirect conjugates. Direct conjugates encompass those in which two or more components are joined by direct covalent chemical linkages. Alternatively, indirect conjugates encompass those in which two or more components are joined via an intermediary complex involving a biological macromolecule. The system described herein is the first to enable the formation of indirect conjugates without dependence upon an intermediary biological macromolecule.

### AVIDIN-BIOTIN SYSTEM

Although numerous methods of indirect bioconjugate preparation have been described, a significant number of those reported in the literature have been prepared by exploiting the Avidin-Biotin system, in which, the binding specificity of the protein Avidin (purified from egg white), or Streptavidin (purified from the bacterium *Streptomyces avidinii*), toward the cofactor Biotin (vitamin H) is utilized to bridge an Avidin conjugated macromolecule with a biotinylated macromolecule. Both Avidin and Streptavidin possess four Biotin binding sites of very high affinity (K = 10¹⁵ mol⁻¹).

The Avidin-Biotin system has been utilized extensively for enzyme-linked immuno solid-phase assay (ELISA), in which an enzyme-Avidin conjugate (useful for detection by reaction with the enzyme's substrate to afford a colored or chemiluminescent product) is employed to detect the presence of a biotinylated antibody, after first binding the antibody to an immobilized antigen or hapten. Applications of the Avidin-Biotin system number in the hundreds, and have recently been reviewed (Wilchek, M. and Bayer, E. A., (1990) *Methods in Enzymology*, **184**).

Although utilized extensively, several limitations are known to be associated with the Avidin-Biotin system, which include nonspecific binding generally attributed to the basicity of the Avidin molecule, nonspecific binding attributed to the presence of carbohydrate residues on the Avidin molecule, and background interference associated with the presence of endogenous Biotin, which is ubiquitous in both eukaryotic and prokaryotic cells.

### DIGOXIGENIN α-DIGOXIGENIN SYSTEM

An alternative indirect bioconjugation system designed to overcome some of the limitations associated with the Avidin-Biotin system has recently been developed for the detection of gene probes by ELISA (Kessler, C., Hôltke, H.-J., Seibl, R., Burg, J. and Mühlegger, K., (1990) *Biol. Chem. Hoppe-Seyler,* **371,** 917-965. This system involves the use of the steroid hapten Digoxigenin, an alkaloid occuring exclusively in Digitalis plants, and Fab fragments derived from polyclonal sheep antibodies against Digoxigenin (α-Digoxigenin). The high specificity of the various α-Digoxigenin antibodies affords low backgrounds and eliminates the non-specific binding observed in Avidin-Biotin systems. Digoxigenin-labeled DNA and RNA probes can detect single-copy sequences in human genomic Southern blots. The development of the Digoxigenin α-Digoxigenin system has recently been reviewed (Kessler, C. (1990) in Advances in Mutagenesis Research (Obe, G. ed.) pp. 105-152, Springer-Verlag, Berlin/Heidelberg). The Digoxigenin α-Digoxigenin system is the most recent representative of several hapten-antibody systems now utilized for bioconjugation.

### IMMOBILIZED PHENYLBORONATES

Phenylboronic acids are known to interact with a wide range of polar molecules having certain requisite functionalities. Complexes of varying stability, involving 1,2-diols, 1,3-diols, 1,2-hydroxy acids, 1,3-hydroxy acids, 1,2-hydroxylamines, 1,3-hydroxylamines, 1,2-diketones and 1,3-diketones, are known to form with either neutral phenylboronic acid or phenylboronate anion. Consequently, immobilized phenylboronic acids have been exploited as chromatographic supports to selectively retain, from diverse biological samples, those molecular species having the requisite functionalities. Many important biological molecules including carbohydrates, catecholamines, prostaglandins, ribonucleosides, and steroids contain the requisite functionalities, and have been either analyzed or purified in this manner. The use of phenylboronic acid chromatographic media for the isolation and separation of biological molecules has been discussed in several reviews (Singhal, R. P. and DeSilva, S. S. M. (1992) *Adv. Chromatog.,* **31,** 293-335; Mazzeo, J. R. and Krull, I. S. (1989) *BioChromatog*., **4,** 124-130; and Bergold, A. and Scouten, W. H. (1983) in Solid Phase Biochemistry (Scouten, W. H. ed.) pp. 149-187, John Wiley & Sons, New York ).

Phenylboronic acid, like boric acid, is a Lewis acid, and ionizes not by direct deprotonation, but by hydration to give the tetrahedral phenylboronate anion (pKₐ = 8.86). Phenylboronic acid is three times as strong an acid as boric acid. Ionization of phenylboronic acid is an important factor in complex formation, in that, upon ionization, boron changes from trigonal coordination (having average bond angles of 120° and average bond lengths of 1.37 ^{∼}) to the tetrahedrally coordinated anion (having average bond angles of 109° and average bond lengths of 1.48 ^{∼}).

Molecular species having cis or coaxial 1,2-diol and 1,3-diol functionalities, and particularly carbohydrates, are known to complex with immobilized phenylboronate anion, to form cyclic esters under alkaline aqueous conditions (Lorand, J. P. and Edwards, J. O. (1959) *J. Org. Chem*., **24,** 769).

Acidification of 1,2-diol and 1,3-diol complexes to neutral pH is know to release the diol containing species, presumably due to hydrolysis of the cyclic ester. Coplanar aromatic 1,3-diols, like 1,8-dihydroxynaphthalene, are known to complex even under acidic conditions due to the hydrolytic stability of six-membered cyclic boronic acid esters (Sienkiewicz, P. A. and Roberts, D. C. (1980) *J. Inorg. Nucl. Chem*., **42,** 1559-1571). Molecular species having pendant 1,2-hydroxylamine, 1,3-hydroxylamine, 1,2-hydroxyamide, 1,3-hydroxyamide, 1,2-hydroxy-oxime and 1,3-hydroxyoxime functionalities are also known to reversibly complex with phenylboronic acid under alkaline aqueous conditions similar to those associated with the retention of diol containing species (Tanner, D. W. and Bruice, T. C. (1967) *J. Amer. Chem. Soc*., **89,** 6954).

### PHENYLBORONATE BIOCONJUGATES

Ortho-substituted acetamidophenylboronic acids have been proposed as potential linkers for selective bioconjugation via the vicinal diol moieties of the carbohydrate residues associated with glycoproteins (Cai, S. X. and Keana, J. F. W. (1991) *Bioconjugate Chem.,* **2,** 317-322). Phenylboronic acid bioconjugates derived from 3-isothiocyanatophenylboronic acid have been successfully utilized for appending radioactive technetium dioxime complexes to monoclonal antibodies for use in medical imaging (Linder, K. E., Wen, M. D., Nowotnik, D. P., Malley, M. F., Gougoutas, J. Z., Nunn, A. D. and Eckelman, W. C. (1991) *Bioconjugate Chem.,* **2,** 160-170; Linder, K. E., Wen, M. D., Nowotnik, D. P., Ramalingam, K., Sharkey, R. M., Yost, F., Narra, R. K. and Eckelman, W. C. (1991) *Bioconjugate Chem*., **2,** 407-414).

3-Aminophenylboronic acid has been covalently appended to proteins by a variety of chemical methods and the resulting phenylboronic acid bioconjugates tested for their binding of D-sorbitol, D-mannose and glycated hemoglobin (GHb). The interactions proved to be reversible and of very low affinity rendering the bioconjugates of very limited practical use. Similarly, an alkaline phosphatase phenylboronic acid bioconjugate used in an attempted enzyme-linked assay for the detection of GHb failed to detect the presence of glycated protein (Frantzen, F., Grimsrud, K., Heggli, D. and Sundrehagen, E. (1995) *Journal of Chromatography B*, **670,** 37-45).

Certain phenylboronic acid reagents and boronic compound complexing reagents. their conjugates and bioconjugates as well as methods for their preparation have been described in International Application No. PCT/US/95/01004, and corresponding United States Patent Nos. 5594111, 5623055, 5668258, 5648470, 5594151, 5668257, 5677431, 5688928, 5852178, and 6008406. Representatively, International Application No. PCT/US/95/01004 describes a bioconjugate complex having a compound of general formula: BAS-L-Bc-L'-(Bc'-L")n-BAS', wherein BAS and BAS' are bioactive species (which may be the same or different): L, L', and L" are linkers (which may be the same or different); Bc and Bc' are boronic acid complexes (which may be the same or different) of formula D-E or E-D wherein D is a boronic acid moiety and E is a boronic acid complexing moiety, and n is 0 or 1.

U.S. Patent No. 3,293,252 describes cyclic boron compounds represented by the structural formula: 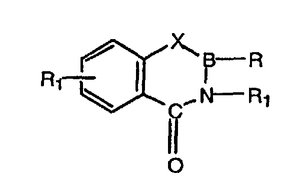 wherein R represents hydroxy, alkyl, cycloalkyl, aryl and arylalkyl groups; R₁ represents aryl and aralkyl groups; R₂ represents hydrogen, halogen, nitro, amino, NH-acyl, sulfonamido, lower alkyl, lower alkoxy and haloalkyl; and x represents oxygen, sulfur, or N-H or N-substituted moieties.

### SUMMARY OF THE INVENTION

The present invention relates to a novel class of boron compound complexing reagents useful for the preparation of bioconjugates, and the method of making and using such reagents. In one embodiment, the boron compound is phenylboronic acid, or derivatives thereof, which complex with the complexing reagents of the present invention. Unless otherwise noted, the phrase phenylboronic acid complexing reagent is used herein to include the broader class of boron compound complexing reagents, and the phrase phenylboronic acid is used herein to include the broader class of boron compounds which complex with the boron compound complexing reagents. In the present invention, in the place of prior art Avidin-Biotin and Digoxigenin α-Digoxigenin systems, boron compound complexing reagents are utilized in conjunction with the boron compound, such as phenylboronic acid reagents (many of which are known in the prior art) to facilitate chemical conjugation without the use of intermediary biological macromolecules. Bioconjugate preparation often involves the conjugation of several components including, but not limited to, proteins, peptides, polysaccharides, hormones, nucleic acids, liposomes and cells, with each other or with any other molecular species that add useful properties, including, but not limited to, drugs, radionuclides, toxins, haptens, inhibitors, fluorophores, ligands, solid-phase supports, and boron compound complexing reagents conjugates. These various components utilized in bioconjugate preparation will collectively and individually be termed biologically active species or bioactive species.

Reagents suitable for the modification of a bioactive species for the purpose of incorporating a phenylboronic acid complexing moiety for subsequent conjugation to a different (or the same) bioactive species having pendant phenylboronic acid moieties are of the general formula of General Formula CI: 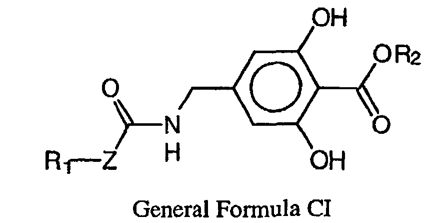 Group R₁ is a reactive electrophilic or nucleophilic moiety suitable for reaction of the putative phenylboronic acid complexing reagent with a bioactive species. Group Z is a spacer selected from a saturated or unsaturated chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group R₂ is selected from an alkyl (e.g., methyl, ethyl, etc.) and a methylene bearing an electronegative substituent.

Reaction of a reagent of General Formula CI with a bioactive species affords a conjugate having pendant putative phenylboronic acid complexing moieties (one or more) of General Formula CII, 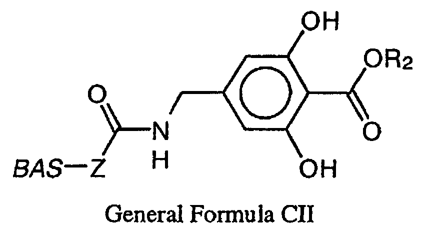 The symbol labeled *BAS* represents a biologically active species (or bioactive species) that may or may not contain a portion of a reactive moiety (which may itself have a spacer) used to attach the bioactive species to the reagent. It will be appreciated that, in many embodiments, several identical reagents of the general formula of General Formula CI will react with a single *BAS* molecule. For example, if the *BAS* is a protein, many phenylboronic acid complexing reagents will react with the protein, each reacting at one of the several sites on the protein which are reactive with the R₁ group. Group Z in General Formula CII is a spacer selected from a saturated or unsaturated chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group R₂ is selected from an alkyl (e.g., methyl, ethyl, etc.) and a methylene bearing an electronegative substituent.

The reagent of General Formula CII may be further reacted to afford a class of conjugate of boronic compound complexing reagents, e.g., reagents with one or more pendant phenylboronic acid complexing moieties of General Formula CIV:

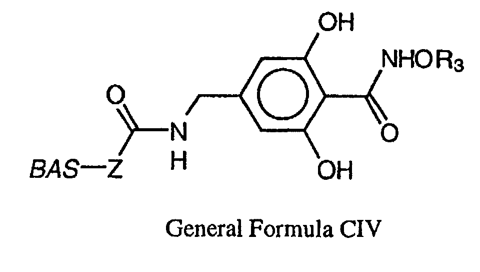

With respect to the conjugate of General Formula CIV, group Z is a spacer selected from a saturated or unsaturated chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group R₃ is selected from one of an H, an alkyl, and a methylene or ethylene with an electronegative substitutent. Finally, the symbol *BAS* represents the bioactive species that may or may not contain a portion of a reactive moiety used to attach the bioactive species.

Phenylboronic acid reagents, many of which are known in the prior art, as well as those described in greater detail in U.S. Patent No. 5,594,111, titled Phenylboronic Acid Complexes for Bioconjugate Preparation, may be appended to a biologically active species to afford a conjugate having pendant phenylboronic acid moieties (one or more) of General Formula CV: 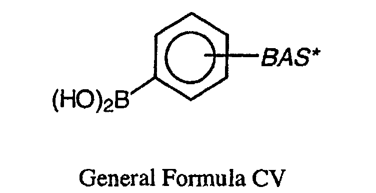 wherein the symbol labeled *BAS** represents a second bioactive species, that may include a linker portion and that may differ from the bioactive species labeled *BAS*. The *BAS** may also include a portion of a reactive moiety used to attach the bioactive species to the phenylboronic acid reagent.

A conjugate of General Formula CIV, with at least one biologically active species and having pendent phenylboronic acid complexing moieties (one or more), may be complexed with a conjugate of General Formula CV, prepared from a second bioactive species *BAS** and having pendant phenylboronic acid moieties (one or more), to afford a bioconjugate of General Formula CVI, 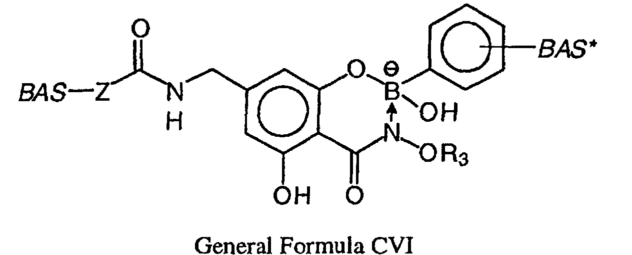 wherein the symbols labeled *BAS* and *BAS**, and groups Z and R₃ are as were previously defined. In this manner, biological macromolecules may be conjugated to one another or with other functionalities which impart useful properties.

Bioconjugates of General Formula CVI may be prepared in buffered aqueous solution or organic solvents. The bioconjugate is formed within a few minutes over a range of temperatures of from about 4° C to 70° C. The stability of the bioconjugate in aqueous solution at a given pH and temperature is significantly influenced by substituent group R₃. Bioconjugates of Genera] Formula CVI, are stable in aqueous solutions of approximate pH greater than 3.5 and less than 10.5.

The bioconjugation reaction (phenylboronic acid complexation) is insensitive to significant variations in ionic strength, the presence of organic solvents, the presence of detergents, and the presence of chaotropic agents (protein denaturants), which are incompatible with prior art indirect labeling systems wherein the structure of a biological macromolecule must be maintained to preserve requisite binding properties. In most instances, the constraints governing the formation of bioconjugates, by the system herein described, are limited to those imposed by the conditions required to maintain viability of the bioactive species.

In summary, boron compound complexing reagents, intermediate reagents of those reagents and methods of synthesizing the reagents are described. These reagents, including those shown as General Formulas Cl and Cll may be used, after further reactions described herein, to complex with boronic compounds, such as phenylboronic acid or derivatives thereof.

Reagents suitable for the modification of a bioactive species for the purpose of incorporating a phenylboronic acid complexing moiety for subsequent conjugation to a different (or the same) bioactive species having pendant phenylboronic acid moieties are of the general formula of General Formula CIII: 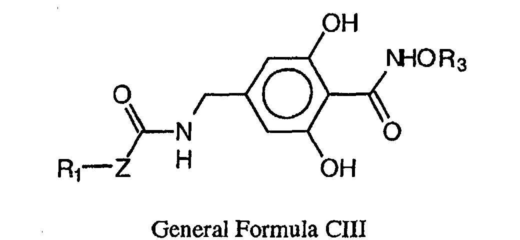 Group R₁ is a reactive electrophilic or nucleophilic moiety suitable for reaction of the phenylboronic acid complexing reagent with a bioactive species. Group Z is a spacer selected from a saturated or unsaturated chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group R₃ is selected from one of an H, an alkyl, and a methylene or ethylene moiety with an electronegative substitutent.

Group R₃ is preferably selected from one of H, CH₃, CH₂CN, CH₂COOH, CH₂CONH₂, CH₂CH₂OH and CH₂OCH₃. When group R₃ is H, group R₁ is preferably selected from one of acrylamide, amino, dithiopyridyl, hydrazide, imidate ester, maleimide, and thiol moieties. Group Z is preferably an unbranched alkyl chain of the general formula (CH₂)ₙ, wherein n = 1 to 6.

Reaction of a reagent of General Formula CIII with a bioactive species affords a conjugate having pendant phenylboronic acid complexing moieties (one or more) of General Formula CIV, 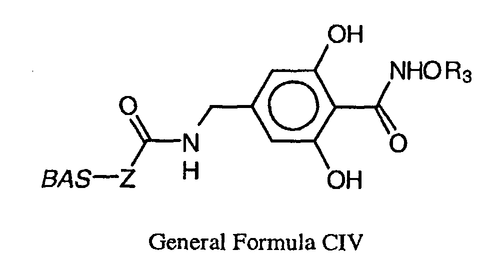 The symbol labeled *BAS* represents a biologically active species (or bioactive species) that may or may not contain a portion of a reactive moiety (which may itself have a spacer) used to attach the bioactive species to the reagent. It will be appreciated that, in many embodiments, several identical reagents of the general formula of General Formula CIII will react with a single *BAS* molecule. For example, if the *BAS* is a protein, many phenylboronic acid complexing reagents will react with the protein, each reacting at one of the several sites on the protein which are reactive with the R₁ group. Group Z in General Formula CIV is a spacer selected from a saturated or unsaturated chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group R₃ is selected from one of an H, an alkyl, and a methylene or ethylene moiety with an electronegative substitutent.

Phenylboronic acid reagents, many of which are known in the prior art, as well as those described in greater detail in U.S Paten No. 5,594,111, titled Phenylboronic Acid Complexes For Bioconjugate Preparation, may be appended to a biologically active species to afford a conjugate having pendant phenylboronic acid moieties (one or more) of General Formula CV: 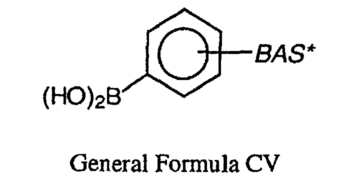 wherein the symbol labeled *BAS** represents a second bioactive species, that may include a linker portion and that may differ from the bioactive species labeled *BAS*. The *BAS** may also include a portion of a reactive moiety used to attach the bioactive species to the phenylboronic acid reagent.

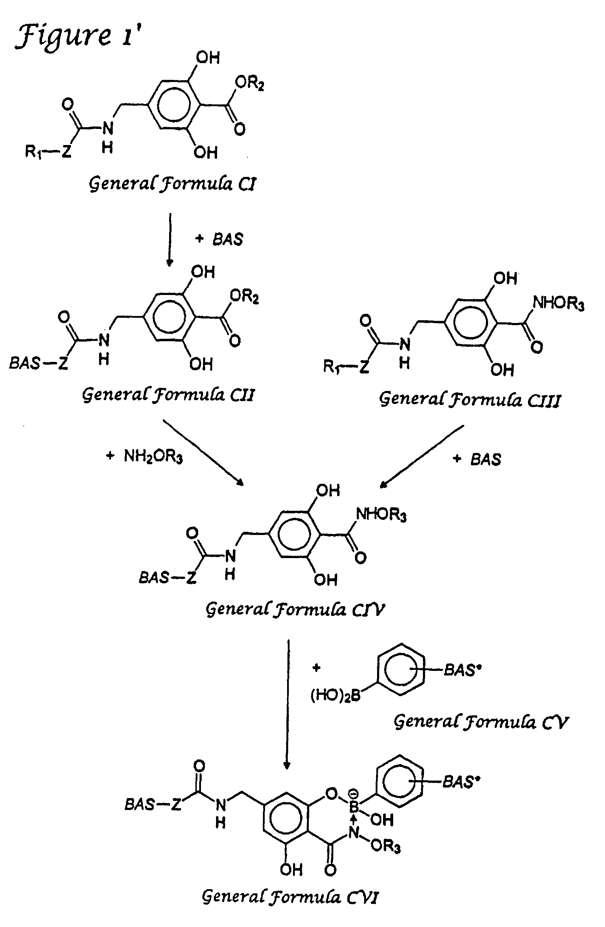

A conjugate of General Formula CIV, with at least one biologically active species and having pendent phenylboronic acid complexing moities (one or more), may be complexed with a conjugate of General Formula CV, prepared from a second bioactive species *BAS** and having pendant phenylboronic acid moities (one or more), to afford a bioconjugate of General Formula CVI, 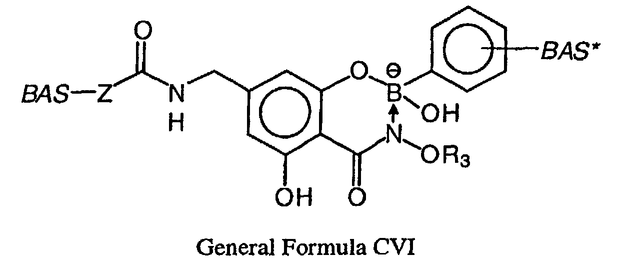 wherein the symbols labeled *BAS* and *BAS**, and groups Z and R₃ are as were previously defined. In this manner, as summarized in ***Figure 1*,** biological macromolecules may be conjugated to one another or with other functionalities which impart useful properties.

Bioconjugates of General Formula CVI may be prepared in buffered aqueous solution or organic solvents. The bioconjugate is formed within a few minutes over a range of temperatures of from about 4° C to 70° C. The stability of the bioconjugate in aqueous solution at a given pH and temperature is significantly influenced by substituent group R₃. Bioconjugates of General Formula CVI, are stable in aqueous solutions of approximate pH greater than 2.5 and less than 12.5. The bioconjugation reaction (phenylboronic acid complexation) is insensitive to significant variations in ionic strength, the presence of organic solvents, the presence of detergents, and the presence of chaotropic agents (protein denaturants), which are incompatible with prior art indirect labeling systems wherein the structure of a biological macromolecule must be maintained to preserve requisite binding properties. In most instances, the constraints governing the formation of bioconjugates, by the system herein described, are limited to those imposed by the conditions required to maintain viability of the bioactive species.

In summary, boron compound complexing reagents, intermediate reagents of these reagents, boron compound complexes, and methods of synthesizing these reagents amd complexes are described. These reagents and complexes include those shown in General Formula CIII, CIV and CVI. In one embodiment, the reagent of General Formula CIII may be used to produce, after condensation with a bioactive species (BAS), the reagent of General Formula CIV. The reagent of General Formula CIV may be used to form a complex with a boron compound, such as a complex shown in General Formula CVI.

### BRIEF DESCRIPTION OF THE FIGURES

***Figure 1*** illustrates the utilization of putative phenylbornic acid complexing reagents of General Formula CI and phenylboronic acid complexing reagents of General Formula CIII, to prepare conjugates of General Formula CIV which may, in turn, be utilized to prepare bioconjugates of General Formula CVI.
***Figure 2*** summarizes the preparation of alkyl 4-aminomethyl-2,6-dihydroxybenzoates, synthetic intermediates leading to reagents of General Formula CI, wherein R₂ is an alkyl group, e.g., methyl, ethyl, propyl, etc. Alkyl 4-aminomethyl-2,6-dihydroxybenzoates are also useful synthetic intermediates leading to reagents of General Formula CIII.
***Figure 3*** summarizes the preparation of alkyl 4-aminomethyl-2,6-dihydroxybenzoates, synthetic intermediates leading to reagents of General Formula CI, wherein R₂ is a methylene group bearing an electronegative moiety, e.g., carboxymethyl, cyanomethyl, methoxymethyl, etc.
***Figure 4*** summarizes the preparation of 4-aminomethyl-2,6-dihydroxybenzohydroxamic acids, synthetic intermediates leading to reagents of General Formula CIII, wherein R₃ is one of either an alkyl group or a methylene or ethylene group bearing an electronegative moiety.
***Figure 5*** summarizes the synthesis of reagents of General Formulas CI and CIII, wherein R₂ is one of either an alkyl group or a methylene group bearing an electronegative moiety, and wherein R₁ is selected from either imidazolide, hydrazide and N-hydroxysuccinimidyl ester moieties.
***Figure 6*** summarizes the synthesis of reagents of General Formulas CI and CIII, wherein R₂ is one of either an alkyl group or a methylene group bearing an electronegative moiety, and wherein R₁ is selected from either bromo, chloro, iodo, maleimide, dithiopyridyl and imidate ester moieties.
***Figure 7*** summarizes the synthesis of reagents of General Formulas CI and CIII, wherein R₁ is an *N*-hydroxysuccinimidy ester, and wherein Z an unbranched saturated or unsaturated chain of from about 6 to 18 carbon equivalents in length with at least one of either an intermediate amide or disulfide moiety.

### DETAILED DESCRIPTION OF THE INVENTION

### PHENYLBORONIC ACID COMPLEXING REAGENTS AND CONJUGATES DERIVED FROM 4-AMINOMETHYL-2,6-DIHYDROXYBENZOIC ACID

A three-step process which utilizes reagents of General Formula CI for the preparation of bioconjugates is summarized in ***Figure 1*.** Initially, a reagent of General Formula CI is selected that is comprised of an appropriate reactive electrophilic or nucleophic group R₁ suitable for reaction with the desired biologically active species.

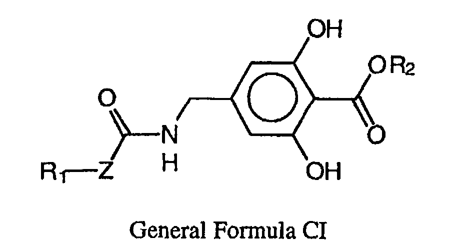

Group R₁ is a reactive electrophilic or nucleophilic moiety suitable for reaction of the putative phenylboronic acid complexing reagent with a bioactive species. Group R₁ is preferably selected from, but not limited to, acrylamide, bromo, dithiopyridyl, bromoacetamide, hydrazide, *N*-hydroxysuccinimidyl ester, N-hydroxysulfosuccinimidyl ester, imidate ester, imadazolide, iodo, iodoacetamide, maleimide, amino and thiol moieties.

Group Z is a spacer selected from a saturated or unsaturated, preferably unbranched, chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group Z is preferably selected from an unbranched alkyl chain of general formula (CH₂)ₙ, wherein n = 1 to 6.

Group R₂ is selected from an alkyl (e.g., methyl, ethyl, etc.) and a methylene bearing an electronegative substituent. An electronegative substituent is a substituent with a negative dipole moment, e.g., CN, COOH, etc. Group R₂ is preferably selected from one of CH₃, CH₂CH₃, CH₂CN, CH₂COOH, CH₂CONH₂ and CH₂OCH₃.

The next step in a three-step process in the preparation of bioconjugates is to condense the appropriate reagent with the bioactive species to yield a conjugate of General Formula CII: 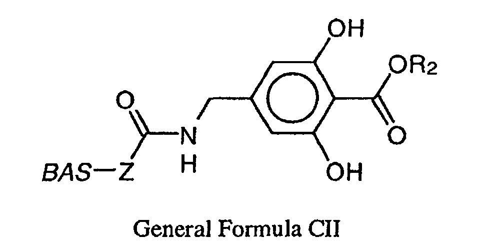 In General Formula CII, Z and R₂ are as defined above, and *BAS* represents a biologically active species which may or may not contain a portion of a reactive moiety (and any spacer) used to attach the biologically active species to the reagent.

Next, the conjugate is reacted with a hydroxylamine derivative of the general formula NH₂OR₃, wherein R₃ is selected from either an H, an alkyl (e.g., methyl, ethyl, etc.), or a methylene or ethylene with an electronegative moiety. Suitable hydroxylamine derivatives include, but not limited to, NH₂OH, NH₂OCH₃, NH₂OCH₂CN, NH₂OCH₂COOH, NH₂OCH₂CONH₂ and NH₂OCH₂CH₂OH. The resulting conjugate has the general formula of General Formula CIV: 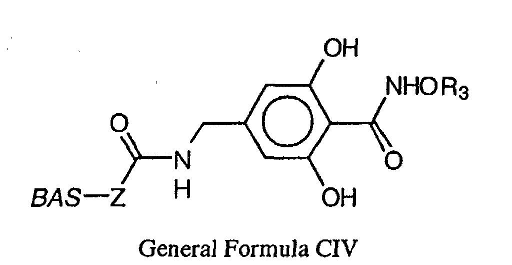 In General Formula CIV, Groups Z and *BAS* are as defined above, and R₃ is selected from one of an H, an alkyl, and a methylene moiety with an electronegative substituent.

The conjugate of General Formula CIV is then complexed with a phenylboronic acid having the general formula of General Formula CV: 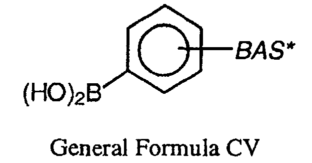 wherein the symbol labeled *BAS** represents a second biologically active species, that may include a linker portion and differ from the biologically active species labeled *BAS* of the complexing reagent. The *BAS** may also include a portion of a reactive moiety used to attach the bioactive species to the phenylboronic acid reagent. The complexation yields the stereoisomeric complex (about tetrahedral boron) of General Formula CVI:

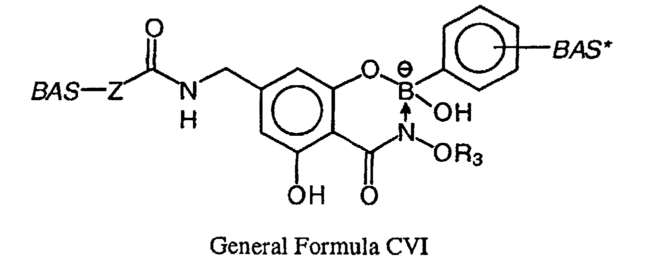

It will be appreciated by one of ordinary skill in the art, upon reference to the disclosure of International Application No. PCT/US97/13314 (WO98/05627), that the various syntheses concerning reagents and conjugates derived from 4 or 5 aminomethyl-salicylic acid and shown in Figures 1 through 7 of that document may be utilized where appropriate for the corresponding synthesis of the reagents shown in Figures 1-7 and/or described in this section. For example, the techniques for adding Z groups and R1 groups from the previous section may be considered for use in preparing the reagents shown in Figures 1 through 7 and/or described in this section.

### SYNTHESIS OF PUTATIVE PHENYLBORONIC ACID COMPLEXING REAGENTS OF GENERAL FORMULA CI

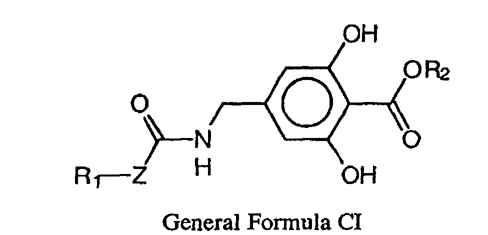

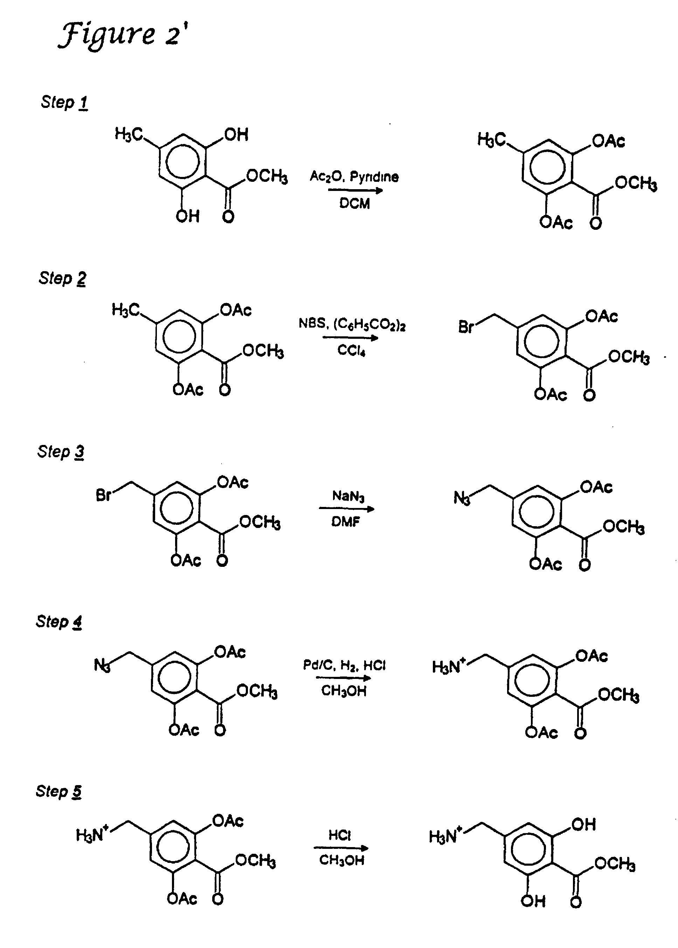

Reagents of General Formula CI are derived from alkyl 2,6-dihydroxy-4-methylbenzoates. ***Figure 2*** summarizes the preparation of alkyl 4-aminomethyl-2,6-dihydroxy-benzoates, synthetic intermediates leading to reagents of General Formula CI, wherein R₂ is an alkyl group, e.g., methyl, ethyl, etc. Figure 2 shows an example where R₂ is a methyl group. Initially, in step 1, an alkyl 2,6-dihydroxy-4-methylbenzoate is acetylated to reduce the reactivity of the ring system toward bromination, affording the corresponding alkyl 2,6-diacetoxy-4-methylbenzoate. In step 2, the ester is brominated with *N*-bromosuccinimide and benzoyl peroxide catalyst to afford the corresponding benzyl bromide. In step 3, the benzyl bromide is alkylated with sodium azide to afford the corresponding benzyl azide. In step 4, the benzyl azide is subjected to palladium catalyzed hydrogenation in the presence of HCI to afford the corresponding benzyl amine hydrochloride. Finally, in step 5, the acetoxy protecting groups are removed by acid catalyzed methanolysis.

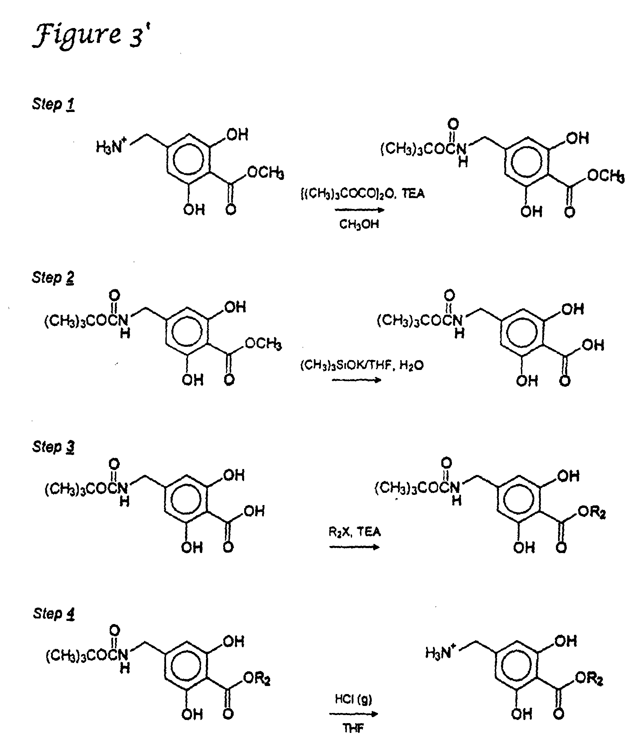

***Figure 3*** summarizes the preparation of synthetic intermediates leading to reagents of General Formula CI, wherein group R₂ is a methylene bearing an electronegative moiety, for example, carboxymethyl, acetamidomethyl and cyanomethyl 4-aminomethyl-2,6-dihydroxy-benzoate. It is to be appreciated that other contemplated substituents for group R₂ may be prepared by a similar synthesis. Initially, in step 1, the alkyl 4-aminomethyl-2, 6-dihydroxy-benzoate, prepared as summarized in ***Figure 2*,** is reacted with di-*tert*-butyl dicarbonate and triethylamine in methanol to afford the corresponding protected alkyl *N*-(*tert*-butoxy-carbonyl)aminomethylbenzoate. In step 2, the alkyl ester is cleaved by reaction with potassium trimethylsilanolate and worked up in aqueous acid to afford the corresponding benzoic acid. In step 3, the benzoic acid is alkylated by reaction with either an α-haloacid, α-haloacetamide or α-haloacetonitrile and triethylamine to afford the corresponding carboxymethyl, acetamido-methyl or cyanomethyl ester, respectively. Finally, in step 4, the *N*-(*tert*-butoxycarbonyl) protecting group is removed by reaction with anhydrous HCl in tetrahydrofuran to afford an alkyl 4-aminomethyl-2, 6-dihydroxybenzoate hydrochloride.

Another reagent of the present invention, which is the acrylic acid amide of alkyl 4-amino methyl-2, 6-dihydroxy-benzoate can be prepared in a single step, using the end product of Figure 3 by condensing acrylic acid anhydride or acryloyl chloride with alkyl 4-aminomethyl-2, 6-dihydroxy-benzoate.

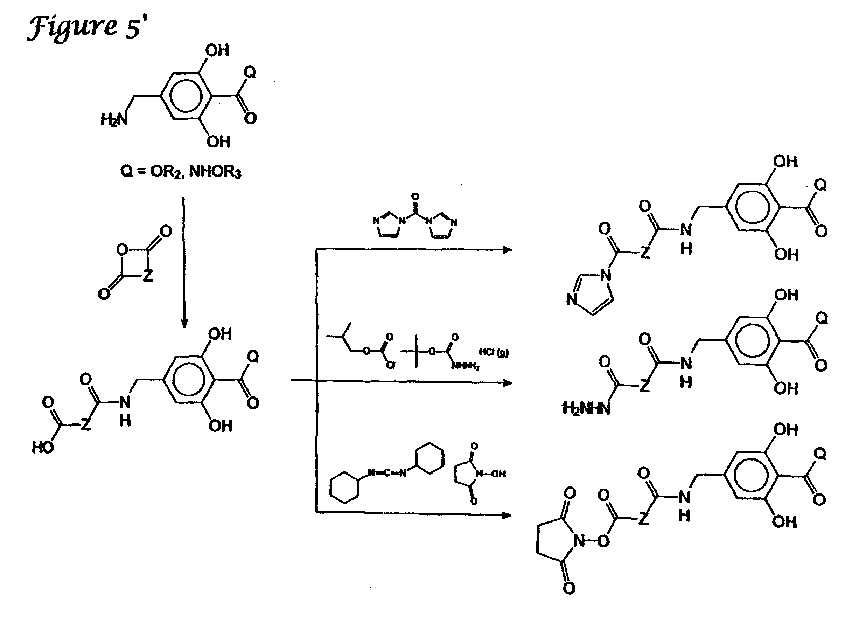

***Figure 5*** summarizes the synthesis of reagents of General Formula CI, wherein group R₂ is one of either an alkyl or a methylene bearing an electronegative moiety, and wherein group R₁ is selected from either imidazolide, hydrazide and *N*-hydroxysuccinimidyl ester moeties. These reagents are each prepared by a two-step process in which an aliphatic acid anhydride is utilized in the first step. Initially, an alkyl 4-aminomethyl-2,6-dihydroxybenzoate, prepared as summarized in either ***Figure 2*** or ***Figure 3*,** is condensed of an aliphatic acid anhydride preferably selected from, but not limited to, either succinic anhydride, glutaric anhydride, maleic anhydride and glycolic acid anhydride, in an aprotic organic solvent, which results in the introduction of a spacer (group Z) having a free terminal carboxylic acid moiety. In the case where the aliphatic acid anhydride is maleic anhydride in this condensation reaction, the resulting Z group is unsaturated as it contains an alkene group. Subsequently, the carboxylic acid moiety is further functionalized by reaction with either *N*,*N*-carbonyldiimidazole, isobutylchloroformate and *tert*-butyl carbazate, or *N,N*-dicyclohexylcarbodiimide and *N*-hydroxy-succinimide to afford the corresponding imidazolide, protected hydrazide and *N*-hydroxy-succinimidyl ester, respectively. In the instance of the protected hydrazide, the *N*-(*tert*-butoxycarbonyl) protecting group is removed by contacting the reagent with anhydrous hydrochloric acid.

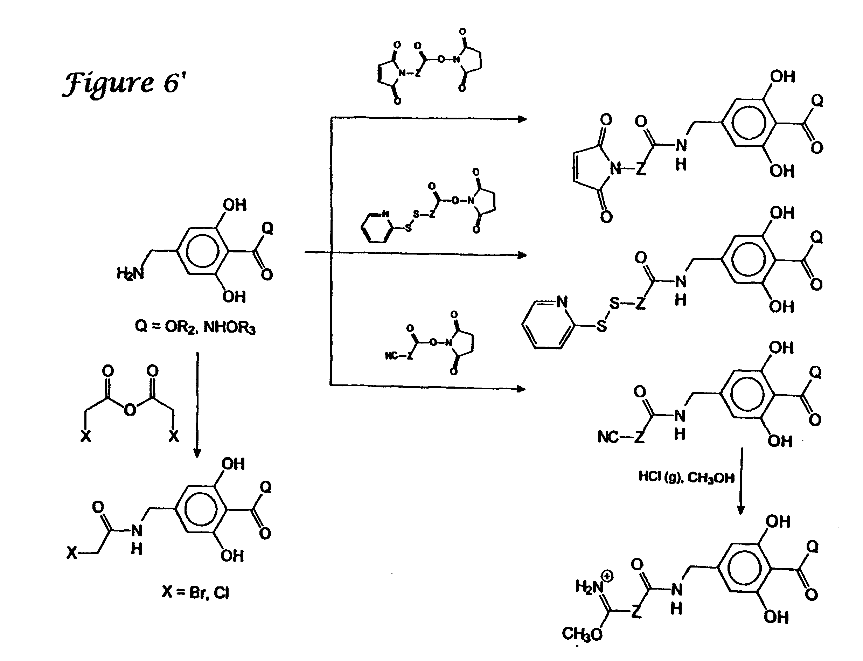

***Figure 6*** summarizes the synthesis of reagents of General Formula CI, wherein group R₂ is one of either an alkyl or a methylene bearing an electronegative moiety, and wherein group R₁ is selected from either acrylamide, bromoacetamide, chloroacetamide, maleimide, dithiopyridyl and imidate ester moieties. Reagents of General Formula CI, wherein group R₁ is selected from either bromo and chloro moieties, are prepared by condensing an alkyl 4- or 5-aminomethyl-salicylate, prepared as summarized in either ***Figure 2*** or ***Figure 3*,** with either bromoacetic acid anhydride or chloroacetic acid anhydride, respectively. The homologous iodo reagent is prepared by halogen exchange of the chloro reagent with sodium iodide. Reagents of General Formula CI, wherein R₁ is selected from either bromo, chloro, iodo, bromoacetamide, chloroacetamide and iodoacetamide moieties, may not be conveniently prepared when R₃ is H, due to the potential for intermolecular alkylation of the unprotected hydroxamate. Reagents of General Formula CI, wherein group R₁ is selected from either maleimide and dithiopyridyl moieties, are prepared by condensing an alkyl 4-aminomethyl-2,6-dihydroxybenzoate, prepared as summarized in either ***Figure 2*** or ***Figure 3*,** with an *N*-hydroxysuccinimidyl ester of an aliphatic carboxylic ester which bears either a terminal maleimide or dithiopyridyl moiety. Reagents of General Formula CI, wherein R₁ is an imidate ester moiety, are prepared by a two-step process in which an alkyl 4-aminomethyl-2,6-dihydroxybenzoate, prepared as summarized in either ***Figure 2*** or ***Figure 3,*** is first condensed with an *N*-hydroxysuccinimidyl ester of an aliphatic carboxylic ester which bears a terminal nitrile moiety. Subsequently, the nitrile moiety is converted to the methyl imidate ester by reaction with anhydrous hydrogen chloride acid in methanol at 0° C.

Reagents of General Formula CI, wherein group R₁ is selected from either *N*-hydroxy-succinimidyl ester and dithiopyridyl moieties, may be utilized as synthetic intermediates to prepare reagents of General Formula CI, wherein group Z is an unbranched saturated or unsaturated chain with at least one of intermediate amide or disulfide moieties.

Reagents of General Formula CI, wherein group R₁ is an *N*-hydroxysuccinimidyl ester moiety, may be condensed with compounds having primary aliphatic amine moieties of the general formula R₁-Z₂-NH₂, wherein Z₂ is an unbranched saturated or unsaturated chain preferably of from about 1 to 5 carbon equivalents in length, to afford reagents of General Formula CI, wherein group Z is an unbranched saturated or unsaturated chain with at least one of intermediate amide moieties.

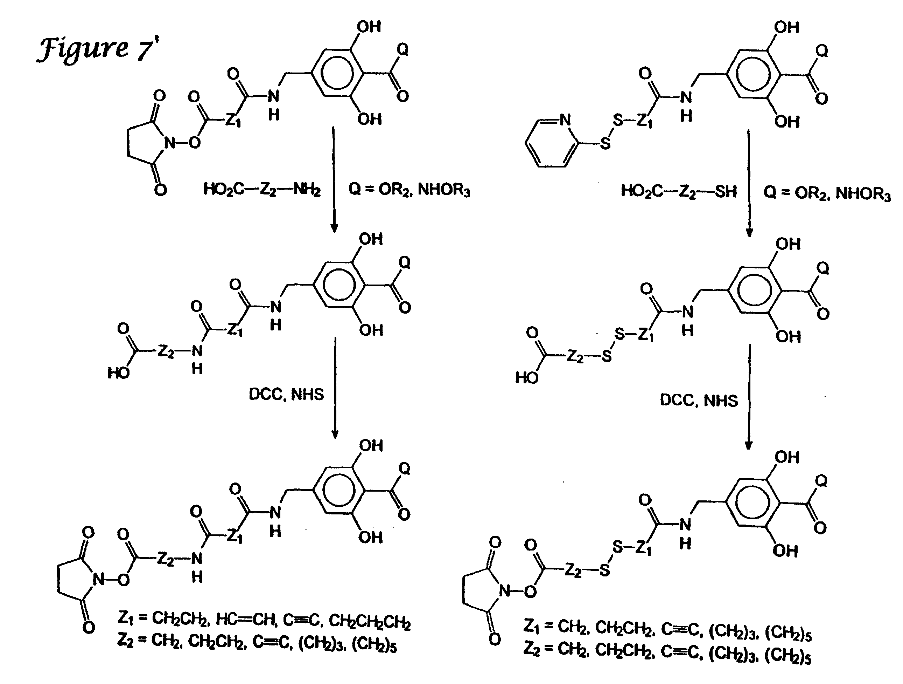

Alternatively, *N*-hydroxysuccinimidyl ester reagents of General Formula CI, preferably derived from a dicarboxylic acid selected from either succinic acid, maleic acid, fumaric acid, acetylenedicarboxylic acid and glutaric acid, may be condensed with compounds having primary aliphatic amine moieties of the general formula HO₂C-Z₂-NH2, wherein Z₂ is an unbranched saturated or unsaturated chain preferably of from about 1 to 5 carbon equivalents in length, preferably selected from, but not limited to, either glycine, β-alanine, aminopropiolic acid, 4-aminobutyric acid and 6-aminocaproic acid, to afford compounds having free terminal carboxylic acid moieties which may be further functionalized in accordance with ***Figure 5*** to afford reagents of General Formula CI, wherein Z is an unbranched saturated or unsaturated chain with at least one of intermediate amide moieties. This process is summarized in ***Figure 7*** for the synthesis of a reagent of General Formula CI, wherein R₁ is an *N*-hydroxysuccinimidyl ester, and wherein Z is an unbranched saturated or unsaturated chain with at least one of an intermediate amide moiety.

Reagents of General Formula CI, wherein group R₁ is a dithiopyridyl moiety, may be condensed with compounds having terminal thiol moieties of the general formula R₁-Z₂-SH, wherein Z₂ is an unbranched saturated or unsaturated chain preferably of from about 1 to 5 carbon equivalents in length, to afford reagents of General Formula CI, wherein group Z is an unbranched saturated or unsaturated chain with at least one of intermediate disulfide moieties.

Alternatively, dithiopyrinyl reagents of General Formula CI, preferably derived from a mercaptocarboxylic acid selected from either mercaptoacetic acid, β-mercaptopropionic acid, mercaptopropiolic acid, 4-mercaptobutyric acid and 6-mercaptocaproic acid, may be condensed with compounds having a thiol moiety of the general formula HO₂C-Z₂-SH, wherein Z₂ is an unbranched saturated or unsaturated chain preferably of from about 1 to 5 carbon equivalents in length, preferably selected from, either mercaptoacetic acid, β-mercaptopropionic acid, mercaptopropiolic acid, 4-mercaptobutyric acid and 6-mercaptocaproic acid, to afford compounds having free terminal carboxylic acid moieties which may be further functionalized in accordance with ***Figure 5*** to afford reagents of General Formula I, wherein group Z is an unbranched saturated or unsaturated chain with at least one of intermediate disulfide moieties. This process is summarized in ***Figure 7*** for the synthesis of a reagent of General Formula CI, wherein R₁ is an *N*-hydroxysuccinimidyl ester, and wherein Z is an unbranched saturated or unsaturated chain with at least one of an intermediate disulfide moiety.

Reagents of General Formula CI, wherein group Z is a polyethylene glycol chain of from about 3 to 12 carbon equivalents in length, are prepared by condensing an alkyl 4-aminomethyl-2,6-dihydroxybenzoate, prepared as summarized in either ***Figure 2*** or ***Figure 3,*** with a polyethylene glycol reagent having both an *N*-hydroxysuccinimidyl ester moiety and either a reactive electrophilic or nucleophilic moiety (or a protected precursor thereof), many of which are commercially available, to afford reagents of General Formula CI, wherein group Z is a polyethylene glycol chain of from about 3 to 12 carbon equivalents in length.

### SYNTHESIS OF PHENYLBORONIC ACID COMPLEXING REAGENTS OF GENERAL FORMULA CIII

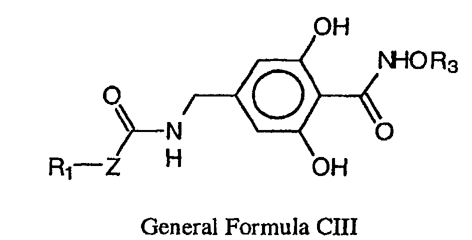

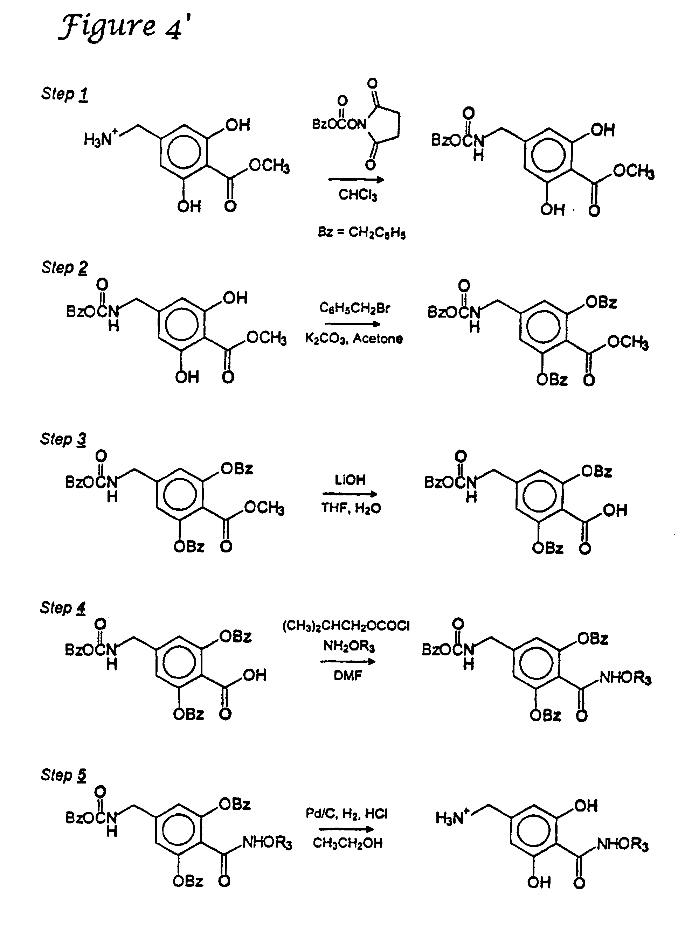

***Figure 4*** summarizes the preparation of 4-aminomethyl-2,6-dihydroxybenzohydroxamic acids, synthetic intermediates leading to reagents of General Formula CIII, wherein group R₃ is one of an alkyl or methylene bearing an electronegative moiety. Initially, in step 1, an alkyl 4-aminomethyl-2,6-dihydroxybenzoate, prepared as summarized in ***Figure 2*,** is condensed with *N*-(benzyloxycarbonyl)oxy succinimide to afford the alkyl *N*-benzyloxycarbonyl protected alkyl 4-aminomethyl-2,6-dihydroxybenzoate. In step 2, the phenolic hydroxyl moieties are condensed with benzyl bromide to afford the further protected benzyl ether intermediate. In step 3, the alkyl ester is selectively cleaved by reaction with LiOH to afford the corresponding carboxylic acid. In step 4, the carboxylic acid is activated by reaction with isobutylchloroformate to form a mixed anhydride which is subsequently reacted with a hydroxylamine derivative preferably selected from, but not limited to, either NH₂OH, NH₂OCH₃, NH₂OCH₂CN, NH₂OCH₂COOH, NH₂OCH₂CONH₂, NH₂OCH₂CH₂OH and NH₂OCH₂OCH₃ to afford the corresponding protected hydroxamic acid. Finally, in step 5, both the amine and phenolic hydroxyl moieties are simultaneously deprotected by paladium catalyzed hydrogenation in the presence of HCI to afford the corresponding 4-aminomethyl-2,6-dihydroxybenzohydroxamic acid hydrochloride. These synthetic intermediates, which are structurally related to the corresponding alkyl 4-amino-methyl-2,6-dihydroxybenzoates, may be further functionalized in accordance with either ***Figure 5*** or ***Figure 6*** to afford reagents of General Formula CIII.

### PREPARATION OF PHENYLBORONIC ACID COMPLEXING CONJUGATES OF GENERAL FORMULA CIV

At this point, the putative phenylboronic acid complexing reagents of General Formula CI may be reacted with a suitable biologically active species to yield the conjugate of the general formula of General Formula CII:

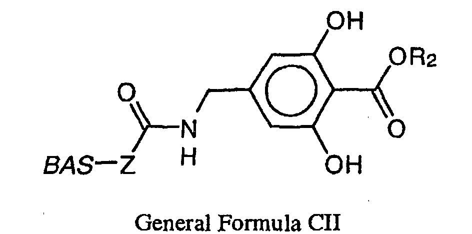

The conjugate of General Formula CII is next condensed with a hydroxylamine derivative to yield the corresponding phenylboronic acid complexing conjugate of the general formula of General Formula CIV:

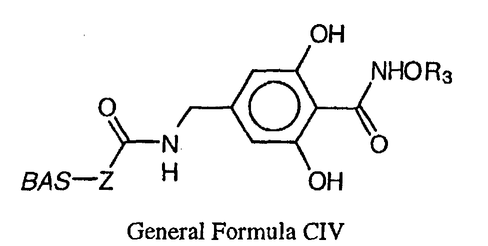

Suitable hydroxylamine derivatives are preferably selected from, but not limited to, NH₂OH, NH₂OCH₃, NH₂OCH₂CN, NH₂OCH₂COOH, NH₂OCH₂CONH₂, NH₂OCH₂CH₂OH, NH₂OCH₂OCH₃. When group R₂ in General Formula CII is an alkyl group, NH₂OH is preferably utilized to effect the interconversion of General Formula CII to General Formula CIV.

Alternatively, conjugates of General Formula CIV may also be prepared by the route which is also described in ***Figure 1*** which utilizes a reagent of General Formula CIII:

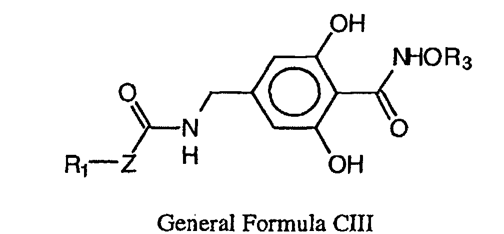

Phenylboronic acid complexing reagents of General Formula CIII may be reacted with a suitable biologically active species to yield the conjugate of the general formula of General Formula CIV:

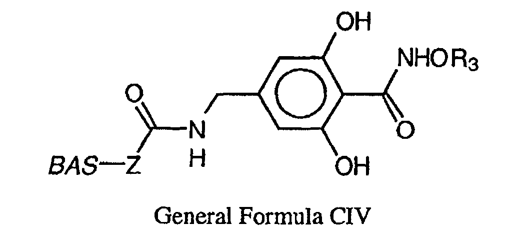

A two-step process which utilizes reagents of General Formula CIII for the preparation of bioconjugates is summarized in ***Figure 1*.** Initially, a reagent of General Formula CIII is selected that is comprised of an appropriate reactive electrophilic or nucleophic group R₁ suitable for reaction with the desired biologically active species.

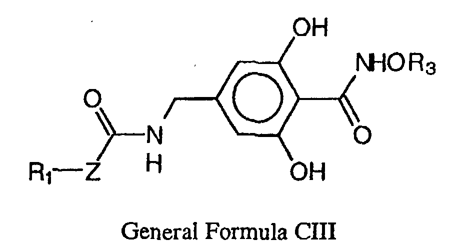

Group R₁ is a reactive electrophilic or nucleophilic moiety suitable for reaction of the phenylboronic acid complexing reagent with a bioactive species. Group R₁ is preferably selected from, but not limited to, acrylamide, bromo, dithiopyridyl, bromoacetamide, hydrazide, *N*-hydroxysuccinimidyl ester, *N*-hydroxysulfosuccinimidyl ester, imidate ester, imidazolide, iodo, iodoacetamide, maleimide, amino and thiol moieties.

Group Z is a spacer selected from a saturated or unsaturated, preferably unbranched, chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group Z is preferably selected from an unbranched alkyl chain of general formula (CH₂)ₙ, wherein n = 1 to 6.

Group R₃ is selected from one of an H, an alkyl, and a methylene or ethylene moiety with an electronegative substitutent.

Group R₃ is preferably selected from one of H, CH₃, CH₂CN, CH₂COOH, CH₂CONH₂, CH₂CH₂OH and CH₂OCH₃. When group R₃ is H, group R₁ is preferably selected from one of acrylamide, amino, dithiopyridyl, hydrazide, imidate ester, maleimide, and thiol moieties.

The reagent of General Formula CIII is condensed with the bioactive species to yield a conjugate of General Formula CIV: 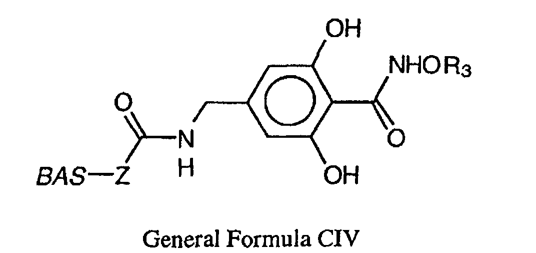 wherein groups Z and *BAS* are as defined above, and R₃ is selected from one of an H, an alkyl, and a methylene or ethylene moiety with an electronegative substitutent.

The conjugate of General Formula CIV is then complexed with a phenylboronic acid conjugate having the general formula of General Formula CV: 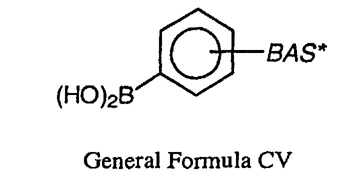 wherein the symbol labeled *BAS** represents a second biologically active species, that may include a linker portion and differ from the biologically active species labeled *BAS* of the complexing reagent. The *BAS** may also include a portion of a reactive moiety used to attach the bioactive species to the phenylboronic acid reagent. The complexation yields the stereoisomeric complex (tetrahedral boron) of General Formula CVI:

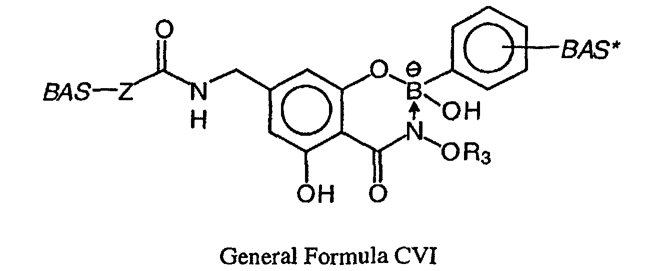

An alternative three-step process which utilizes reagents of General Formula CI for the preparation of bioconjugates is also summarized in ***Figure 1*.** Initially, a reagent of General Formula CI is selected that is comprised of an appropriate reactive electrophilic or nucleophic group R₁ suitable for reaction with the desired biologically active species.

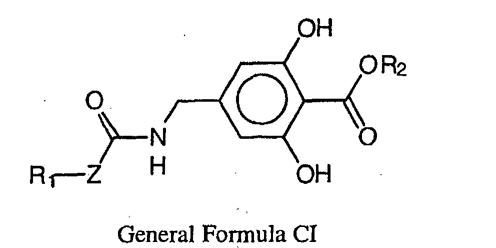

Group R₁ is a reactive electrophilic or nucleophilic moiety suitable for reaction of the putative phenylboronic acid complexing reagent with a bioactive species. Group R₁ is perferably selected from, but not limited to, acrylamide, bromo, dithiopyridyl, bromoacetamide, hydrazide, *N*-hydroxysuccinimidyl ester, *N*-hydroxysulfosuccinimidyl ester, imidate ester, imidazolide, iodo, iodoacetamide, maleimide, amino and thiol moieties.

Group Z is a spacer selected from a saturated or unsaturated, preferrably unbranched, chain of from about 0 to 6 carbon equivalents in length, an unbranched saturated or unsaturated chain of from about 6 to 18 carbon equivalents in length with at least one of intermediate amide or disulfide moieties, and a polyethylene glycol chain of from about 3 to 12 carbon equivalents in length. Group Z is preferably selected from an unbranched alkyl chain of general formula (CH₂)ₙ, wherein n = 1 to 6.

Group R₂ is selected from an alkyl (e.g., methyl, ethyl, etc.) and a methylene or ethylene bearing an electronegative substituent. An electronegative substitutent is a substituent with a negative dipole moment, e.g., CN, COOH, etc. Group R₂ is preferably selected from one of CH₃, CH₂CH₃, CH₂CN, CH₂COOH, CH₂CONH₂ and CH₂OCH₃.

The next step in a three-step process in the preparation of bioconjugates is to condense the appropriate reagent with the bioactive species to yield a conjugate of General Formula CII: 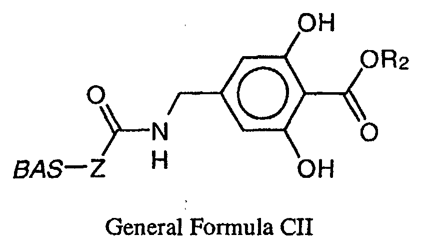 In General Formula CII, Z, BAS and R₂ are as defined above.

Next, the conjugate is reacted with a hydroxylamine derivative of the general formula NH₂OR₃, wherein R₃ is selected from either an H, an alkyl (e.g., methyl, ethyl, etc.), or a methylene or ethylene with an electronegative moiety. Suitable hydroxylamine derivatives include, but not limited to, NH₂OH, NH₂OCH₃, NH₂OCH₂CN, NH₂OCH₂COOH, NH₂OCH₂CONH₂ and NH₂OCH₂CH₂OH. The resulting conjugate has the general formula of General Formula CIV: 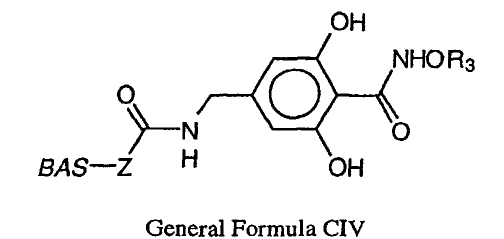 In General Formula CIV, Groups Z and *BAS* are as defined above, and R₃ is selected from one of an H, an alkyl, and a methylene or ethylene moiety with an electronegative substitutent.

The conjugate of General Formula CIV is then complexed with a phenylboronic acid having the general formula of General Formula CV: 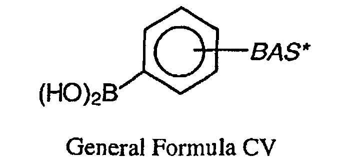 wherein the symbol labeled *BAS** represents a second biologically active species, that may include a linker portion and differ from the biologically active species labeled *BAS* of the complexing reagent. The *BAS** may also include a portion of a reactive moiety used to attach the bioactive species to the phenylboronic acid reagent. The complexation yields the stereoisomeric complex (about tetrahedral boron) of General Formula CVI:

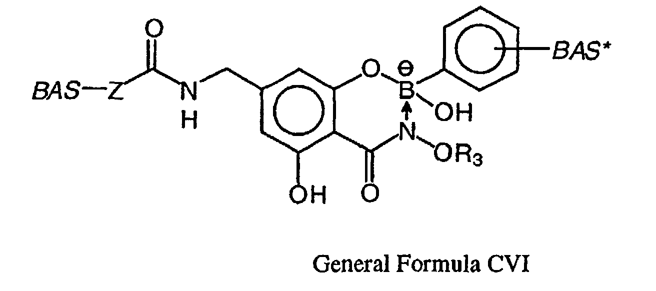

It will be appreciated by one of the ordinary skill in the art, upon reference to this disclosure, that the various syntheses described above in the previous section (concerning reagents and conjugates derived from 4 or 5 aminomethyl-salicylic acid and shown in Figures 1 through 7) may be utilized where appropriate for the corresponding synthesis of the reagents shown in Figures 1-7 and/or described in this section. For example, the techniques for adding Z groups and R1 group from the previous section may be considered for use in preparing the reagents shown in Figures 1-7 and/or described in this section.

### SYNTHESIS OF PHENYLBORONIC ACID COMPLEXING REAGENTS OF GENERAL FORMULA CIII

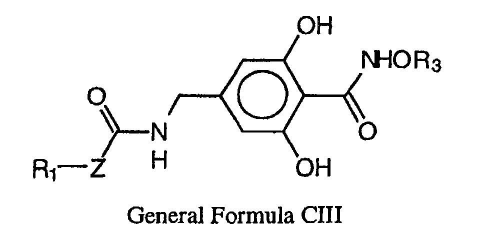

Reagents of General Formula CIII are derived from 2,6-dihydroxy-4-methylbenzoic acid. In each instance, the reagent is ultimately prepared from a synthetic intermediate which is an alkyl 4-aminomethyl-2,6-dihydroxybenzoate.

***Figure 2*** summarizes the preparation of alkyl 4-aminomethyl-2,6-dihydroxybenzoates, synthetic intermediates leading to reagents of General Formula CI, wherein R₂ is an alkyl group, e.g., methyl, ethyl, etc. Initially, in step 1, an alkyl 2,6-dihydroxy-4-methylbenzoate is acetylated to reduce the reactivity of the ring system toward bromination, affording the corresponding alkyl 2,6-diacetoxy-4-methylbenzoate. In step 2, the ester is brominated with *N*-bromosuccinimide and benzoyl peroxide catalyst to afford the corresponding benzyl bromide. In step 3, the benzyl bromide is alkylated with sodium azide to afford the corresponding benzyl azide. In step 4, the benzyl azide is subjected to palladium catalyzed hydrogenation in the presence of HCl to afford the corresponding benzyl amine hydrochloride. Finally, in step 5, the acetoxy protecting groups are removed by acid catalyzed methanolysis.

***Figure 4*** summarizes the preparation of 4-aminomethyl-2,6-dihydroxybenzohydroxamic acids, synthetic intermediates leading to reagents of General Formula CIII, wherein group R₃ is one of an alkyl or methylene or ethylene bearing an electronegative substituent. Initially, in step 1, an alkyl 4-aminomethyl-2,6-dihydroxybenzoate, prepared as summarized in ***Figure 2*,** is condensed with *N*-(benzyloxycarbonyl)oxy succinimide to afford the alkyl *N*-benzyloxycarbonyl protected 4-aminomethyl-2,6-dihydroxybenzoate. In step 2, the phenolic hydroxyl moieties are condensed with benzyl bromide to afford the further protected benzyl diether intermediate. In step 3, the alkyl ester is selectively cleaved by reaction with LiOH to afford the corresponding benzoic acid. In step 4, the benzoic acid is activated by reaction with isobutylchloroformate to form a mixed anhydride which is subsequently reacted with a hydroxylamine derivative preferrably selected from, but not limited to, either NH₂OH, NH₂OCH₃, NH₂OCH₂CN, NH₂OCH₂COOH, NH₂OCH₂CONH₂, NH₂OCH₂CH₂OH and NH₂OCH₂OCH₃ to afford the corresponding protected hydroxamic acid. Finally, in step 5, both the amine and phenolic hydroxyl moieties are simultaneously deprotected by palladium catalyzed hydrogenation in the presence of HCl to afford the corresponding 4-aminomethyl-2,6-dihydroxybenzohydroxamic acid hydrochloride.

Another reagent of the present invention, which is the acrylic acid amide of 4-aminomethyl 2, 6 dihydroxybenzo hydroxamic acid, can be prepared in a single step, using the end procuct of Figure *4*, by condensing acrylic acid anhydride or acryloyl chloride with 4 aminomethyl 2, 6 dihydroxybenzo hydroxamic acid, provided that R₃ is not H.

***Figure 5*** summarizes the synthesis of reagents of General Formula CIII, wherein group R₃ is preferably selected from one of H, CH₃, and a methylene or ethylene moiety with an electronegative substitutent, and wherein group R₁ is selected from either imidazolide, hydrazide and *N*-hydroxysuccinimidyl ester moieties. These reagents are each prepared by a two-step process in which an aliphatic acid anhydride is utilized in the first step. Initially, a 4-amino-methyl-2,6-dihydroxybenzohydroxamic acid, prepared as summarized in ***Figure 4*,** is condensed of an aliphatic acid anhydride preferably selected from, but not limited to, either succinic anhydride, glutaric anhydride, maleic anhydride and glycolic acid anhydride, in an aprotic organic solvent, which results in the introduction of a spacer (group Z) having a free terminal carboxylic acid moiety. In the case where the aliphatic acid anhydride is maliec anhydride in this condensation reaction, the resulting Z group is unsaturated as it contains an alkene group. Subsequently, the carboxylic acid moiety is further functionalized by reaction with either *N*,*N*-carbonyldiimidazole, isobutylchloroformate and *tert*-butyl carbazate, or *N,N*-dicyclohexyl-carbodiimide and *N*-hydroxysuccinimide to afford the corresponding imidazolide, protected hydrazide and *N*-hydroxysuccinimidyl ester, respectively. In the instance of the protected hydrazide, the *N*-(*tert*-butoxycarbonyl) protecting group is removed by contacting the reagent with anhydrous hydrochloric acid.

***Figure 6*** summarizes the synthesis of reagents of General Formula CIII, wherein group R₃ is preferably selected from one of H, CH₃, and a methylene or ethylene moiety with an electronegative substitutent, and wherein group R₁ is selected from either bromo, chloro, maleimide, dithiopyridyl and imidate ester moieties. Reagents of General Formula CIII, wherein group R₁ is selected from either bromo and chloro moieties, are prepared by condensing a 4-aminomethyl-2,6-dihydroxybenzohydroxamic acid, prepared as summarized in ***Figure 4,*** with either bromoacetic acid anhydride or chloroacetic acid anhydride, respectively. The homologous iodo reagent is prepared by halogen exchange of the chloro reagent with sodium iodide. Reagents of General Formula CIII, wherein R₁ is selected from either bromo, chloro, iodo, bromoacetamide, chloroacetamide and iodoacetamide moieties, may not be conveniently prepared when R₃ is H, due to the potential for intermolecular alkylation of the unprotected hydroxamate. Reagents of General Formula CIII, wherein group R₁ is selected from either maleimide and dithiopyridyl moieties, are prepared by condensing a 4-aminomethyl-2,6-dihydroxybenzohydroxamic acid, prepared as summarized in ***Figure 4***, with an *N*-hydroxysuccinimidyl ester of an aliphatic carboxylic ester which bears either a terminal maleimide or dithiopyridyl moiety. Reagents of General Formula CIII, wherein R₁ is an imidate ester moiety, are prepared by a two-step process in which a 4-aminomethyl-2,6-dihydroxybenzohydroxamic acid, prepared as summarized in ***Figure 4*,** is first condensed with an *N*-hydroxysuccinimidyl ester of an aliphatic carboxylic ester which bears a terminal nitrile moiety. Subsequently, the nitrile moiety is converted to the methyl imidate ester by reaction with anhydrous hydrogen chloride in methanol at 0° C.

Reagents of General Formula CIII, wherein group R₁ is selected from either N-hydroxy-succinimidyl ester and dithiopyridyl moieties may be utilized as synthetic intermediates to prepare reagents of General Formula CIII, wherein group Z is an unbranched saturated or unsaturated chain with at least one of intermediate amide or disulfide moieties.

Reagents of General Formula CIII, wherein group R₁ is an *N*-hydroxysuccinimidyl ester moieties, may be condensed with compounds having primary aliphatic amine moieties of the general formula R₁-Z₂-NH₂, wherein Z₂ is an unbranched saturated or unsaturated chain preferably of from about I to 5 carbon equivalents in length, to afford reagents of General Formula CIII, wherein group Z is an unbranched saturated or unsaturated chain with at least one of intermediate amide moieties.

Alternatively, *N*-hydroxysuccinimidyl ester reagents of General Formula CIII, preferably derived from a dicarboxylic acid selected from either succinic acid, maleic acid, fumaric acid, acetylenedicarboxylic acid and glutaric acid, may be condensed with compounds having primary aliphatic amine moieties of the general formula HO2C-Z₂-NH₂, wherein Z₂ is an unbranched saturated or unsaturated chain preferably of from about 1 to 5 carbon equivalents in length, preferably selected from, either glycine, β-alanine, amniopropiolic acid, 4-amino-butyric acid and 6-aminocaproic acid, to afford compounds having free terminal carboxylic acid moieties which may be further functionalized in accordance with ***Figure 5*** to afford reagents of General Formula CIII, wherein Z is an unbranched saturated or unsaturated chain with at least one of intermediate amide moieties. This process is summarized for in ***Figure 7*** for the synthesis of a reagent of General Formula CIII, wherein R₁ is an *N*-hydroxysuccinimidyl ester, and wherein Z is an unbranched saturated or unsaturated chain with at least one of an intermediate amide moiety.

Reagents of General Formula CIII, wherein group R₁ is a dithiopyridyl moiety, may be condensed with compounds having terminal thiol moieties of the general formula R₁-Z₂-SH, wherein Z₂ is an unbranched saturated or unsaturated chain preferably of from about 1 to 5 carbon equivalents in length, to afford reagents of General Formula CIII, wherein group Z is an unbranched saturated or unsaturated chain with at least one of intermediate disulfide moieties.

Alternatively, dithiopyridyl reagents of General Formula CIII, preferably derived from a mercaptocarboxylic acid selected from either mercaptoacetic acid, β-mercaptopropionic acid, mercaptopropiolic acid, 4-mercaptobutyric acid and 6-mercaptocaproic acid, may be condensed with compounds having a terminal thiol moiety of the general formula HO₂C-Z₂-SH, wherein Z₂ is an unbranched saturated or unsaturated chain preferably of from about 1 to 5 carbon equivalents in length, preferably selected from either mercaptoacetic acid, β-mercaptopropionic acid, mercaptopropiolic acid, 4-mercaptobutyric acid and 6-mercaptocaproic acid, to afford compounds having free terminal carboxylic acid moieties which may be further functionalized in accordance with ***Figure 5*** to afford reagents of General Formula CIII, wherein group Z is an unbranched saturated or unsaturated chain with at least one of intermediate disulfide moieties. This process is summarized in ***Figure 7*** for the synthesis of a reagent of General Formula CIII, wherein R₁ is an *N*-hydroxysuccinimidyl ester, and wherein Z is an unbranched saturated or unsaturated chain with at least one of a intermediate disulfide moiety.

Reagents of General Formula CIII, wherein group Z is a polyethylene glycol chain of from about 3 to 12 carbon equivalents in length, are prepared by condensing a 4-aminomethyl-2,6-dihydroxybenzohydroxamic acid, prepared as summarized in ***Figure 4*,** with a polyethylene glycol reagent having both an *N*-hydroxysuccinimidyl ester moiety and either a reactive electrophilic or nucleophilic moiety (or a protected precursor thereof), many of which are commercially available, to afford reagents of General Formual CIII, wherein group Z is a polyethylene glycol chain of from about 3 to 12 carbon equivalents in length.

### SYNTHESIS OF PUTATIVE PHENYLBORONIC ACID COMPLEXING REAGENTS OF GENERAL FORMULA CI

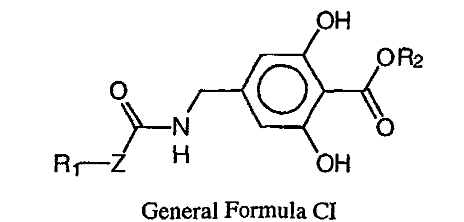

Reagents of General Formula CI are derived from alkyl 2,6-dihydroxy-4-methylbenzoates. ***Figure 2*** summarizes the preparation of alkyl 4-aminomethyl-2,6-dihydroxy-benzoates, synthetic intermediates leading to reagents of General Formula CI, wherein R₂ is an alkyl group, e.g., methyl, ethyl, etc. Figure 2 shows an example where R₂ is a methyl group. Initially, in step 1, an alkyl 2,6-dihydroxy-4-methylbenzoate is acetylated to reduce the reactivity of the ring system toward bromination, affording the corresponding alkyl 2,6-diacetoxy-4-methylbenzoate. In step 2, the ester is brominated with N-bromosuccinimide and benzoyl peroxide catalyst to afford the corresponding benzyl bromide. In step 3, the benzyl bromide is alkylated with sodium azide to afford the corresponding benzyl azide. In step 4, the benzyl azide is subjected to palladium catalyzed hydrogenation in the presence of HCl to afford the corresponding benzyl amine hydrochloride. Finally, in step 5, the acetoxy protecting groups are removed by acid catalyzed methanolysis.

***Figure 3*** summarizes the preparation of synthetic intermediates leading to reagents of General Formula CI, wherein group R₂ is a methylene bearing an electronegative substituent, for example, carboxymethyl, acetamidomethyl and cyanomethyl 4-aminomethyl-2,6-dihydroxy-benzoate. It is to be appreciated that other contemplated substituents for group R₂ may be prepared by a similar synthesis. Initially, in step 1, the alkyl 4-aminomethyl-2,6-dihydroxy-benzoate, prepared as summarized in ***Figure 2,*** is reacted with di-*tert*-butyl dicarbonate and triethylamine in methanol to afford the corresponding protected alkyl *N*-(*tert*-butoxycarbonyl) aminomethylbenzoate. In step 2, the alkyl ester is cleaved by reaction with potassium trimethylsilanolate and worked up in aqueous acid to afford the corresponding benzoic acid. In step 3, the benzoic acid is alkylated by reaction with either an α-haloacid, α-haloacetamide or α-haloacetonitrile and triethylamine to afford the corresponding carboxymethyl, acetamidomethyl or cyanomethyl ester, respectively. Finally, in step 4, the *N*-(*tert*-butoxycarbonyl) protecting group is removed by reaction with anhydrous HCl in tetrahydrofuran to afford an alkyl 4-aminomethyl-2,6-dihydroxybenzoate hydrochloride.

***Figure 5*** summarizes the synthesis of reagents of General Formula CI, wherein group R₂ is one of either an alkyl or a methylene or ethylene bearing an electronegative substituent, and wherein group R₁ is selected from either imidazolide, hydrazide and *N*-hydroxysuccinimidyl ester moeties. These reagents are each prepared by a two-step process in which an aliphatic acid anhydride is utilized in the first step. Initially, an alkyl 4-aminomethyl-2,6-dihydroxybenzoate, prepared as summarized in either ***Figure 2*** or ***Figure 3*,** is condensed of an aliphatic acid anhydride preferably selected from, but not limited to, either succinic anhydride, glutaric anhydride, maleic anhydride and glycolic acid anhydride, in an aprotic organic solvent, which results in the introduction of a spacer (group Z) having a free terminal carboxylic acid moiety. Subsequently, the carboxylic acid moiety is further functionalized by reaction with either *N,N*-carbonyldiimidazole, isobutylchloroformate and *tert*-butyl carbazate, or *N*,*N*-dicyclohexyl-carbodiimide and *N*-hydroxysuccinimide to afford the corresponding imidazolide, protected hydrazide and *N*-hydroxysuccinimidyl ester, respectively. In the instance of the protected hydrazide, the *N*-(*tert*-butoxycarbonyl) protecting group is removed by contacting the reagent with anhydrous hydrochloric acid.

***Figure 6*** summarizes the synthesis of reagents of General Formula CI, wherein group R₂ is one of either an alkyl or a methylene or ethylene bearing an electronegative substituent, and wherein group R₁ is selected from either bromo, chloro, maleimide, dithiopyridyl and imidate ester moieties. Reagents of General Formula CI, wherein group R₁ is selected from either bromo and chloro moieties, are prepared by condensing an alkyl 4-aminomethyl-2,6-dihydroxy-benzoate, prepared as summarized in either ***Figure 2*** or ***Figure 3*,** with either bromoacetic acid anhydride or chloroacetic acid anhydride, respectively. The homologous iodo reagent is prepared by halogen exchange of the chloro reagent with sodium iodide. Reagents of General Formula CI, wherein R₁ is selected from either bromo, chloro, iodo, bromoacetamide, chloroacetamide and iodoacetamide moieties, may not be conveniently prepared when R₃ is H, due to the potential for intermolecular alkylation of the unprotected hydroxamate. Reagents of General Formula CI, wherein group R₁ is selected from either maleimide and dithiopyridyl moieties, are prepared by condensing an alkyl 4-aminomethyl-2,6-dihydroxybenzoate, prepared as summarized in either ***Figure 2*** or ***Figure 3*,** with an *N*-hydroxysuccinimidyl ester of an aliphatic carboxylic ester which bears either a terminal maleimide or dithiopyridyl moiety. Reagents of General Formula CI, wherein R₁ is an imidate ester moiety, are prepared by a two-step process in which an alkyl 4-aminomethyl-2,6-dihydroxybenzoate, prepared as summarized in either ***Figure 2*** or ***Figure 3*,** is first condensed with an *N*-hydroxysuccinimidyl ester of an aliphatic carboxylic ester which bears a terminal nitrile moiety. Subsequently, the nitrile moiety is converted to the methyl imidate ester by reaction with anhydrous hydrochloric acid in methanol at 0° C.

Reagents of General Formula CI, wherein group R₁ is selected from either dithiopyridyl, imidazolide and *N*-hydroxysuccinimidyl ester moieties may be utilized as synthetic intermediates to prepare reagents of General Formula CI, wherein group Z is an unbranched saturated or unsaturated chain with at least one of intermediate amide or disulfide moieties.

Reagents of General Formula CI, wherein group R₁ is selected from either *N*-hydroxy-succinimidyl ester and dithiopyridyl moieties may be utilized as synthetic intermediates to prepare reagents of General Formula CI, wherein group Z is an unbranched saturated or unsaturated chain with at least one of intermediate amide or disulfide moieties.

Reagents of General Formula CI, wherein group R₁ is an *N*-hydroxysuccinimidyl ester moieties, may be condensed with compounds having primary aliphatic amine moieties of the general formula R₁-Z₂-NH₂, wherein Z₂ is an unbranched saturated or unsaturated chain preferably of from about 1 to 5 carbon equivalents in length, to afford reagents of General Formula CI, wherein group Z is an unbranched saturated or unsaturated chain with at least one of intermediate amide moieties.

Alternatively, *N*-hydroxysuccinimidyl ester reagents of General Formula CI, preferably derived from a dicarboxylic acid selected from either succinic acid, maleic acid, fumaric acid, acetylenedicarboxylic acid and glutaric acid, may be condensed with compounds having primary aliphatic amine moieties of the general formula HO2C-Z₂-NH₂, wherein Z₂ is an unbranched saturated or unsaturated chain preferably of from about 1 to 5 carbon equivalents in length, preferably selected from, either glycine, β-alanine, amniopropiolic acid, 4-amino-butyric acid and 6-aminocaproic acid, to afford compounds having free terminal carboxylic acid moieties which may be further functionalized in accordance with ***Figure 5*** to afford reagents of General Formula CI, wherein Z is an unbranched saturated or unsaturated chain with at least one of intermediate amide moieties. This process is summarized for in ***Figure 7*** for the synthesis of a reagent of General Formula CI, wherein R₁ is an *N*-hydroxysuccinimidyl ester, and wherein Z is an unbranched saturated or unsaturated chain with at least one of an intermediate amide moiety.

Reagents of General Formula CI, wherein group R₁ is a dithiopyridyl moiety, may be condensed with compounds having terminal thiol moieties of the general formula R₁-Z₂-SH, wherein Z₂ is an unbranched saturated or unsaturated chain preferably of from about 1 to 5 carbon equivalents in length, to afford reagents of General Formula CI, wherein group Z is an unbranched saturated or unsaturated chain with at least one of intermediate disulfide moieties.

Alternatively, dithiopyridyl reagents of General Formula CI, preferably derived from a mercaptocarboxylic acid selected from either mercaptoacetic acid, β-mercaptopropionic acid, mercaptopropiolic acid, 4-mercaptobutyric acid and 6-mercaptocaproic acid, may be condensed with compounds having a terminal thiol moiety of the general formula HO₂C-Z₂-SH, wherein Z₂ is an unbranched saturated or unsaturated chain preferably of from about 1 to 5 carbon equivalents in length, preferably selected from either mercaptoacetic acid, β-mercaptopropionic acid, mercaptopropiolic acid, 4-mercaptobutyric acid and 6-mercaptocaproic acid, to afford compounds having free terminal carboxylic acid moieties which may be further functionalized in accordance with ***Figure 5*** to afford reagents of General Formula CI, wherein group Z is an unbranched saturated or unsaturated chain with at least one of intermediate disulfide moieties. This process is summarized in ***Figure 7*** for the synthesis of a reagent of General Formula CI, wherein R₁ is an *N*-hydroxysuccinimidyl ester, and wherein Z is an unbranched saturated or unsaturated chain with at least one of a intermediate disulfide moiety.

Reagents of General Formula CI, wherein group Z is a polyethylene glycol chain of from about 3 to 12 carbon equivalents in length, are prepared by condensing an alkyl 4-aminomethyl-2,6-dihydroxybenzoate, prepared as summarized in either ***Figure 2*** or ***Figure 3,*** with a polyethylene glycol reagent having both an *N*-hydroxysuccinimidyl ester moiety and either a reactive electrophilic or nucleophilic moiety (or a protected precursor thereof), many of which are commercially available, to afford reagents of General Formula CI, wherein group Z is a polyethylene glycol chain of from about 3 to 12 carbon equivalents in length.

### PREPARATION OF PHENYLBORONIC ACID COMPLEXING CONJUGATES OF GENERAL FORMULA CIV

At this point, phenylboronic acid complexing reagents of General Formula CIII may be reacted with a suitable biologically active species to yield the conjugate of the general formula of General Formula CIV:

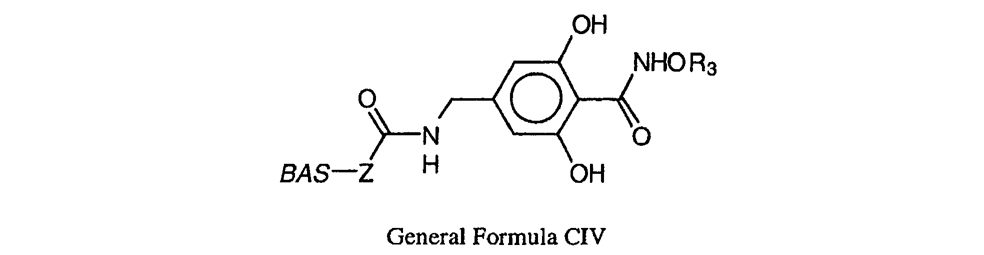

Alternatively, the putative phenylboronic acid complexing reagents of General Formula CI may be reacted with a suitable biologically active species to yield the conjugate of the general formula of General Formula CII:

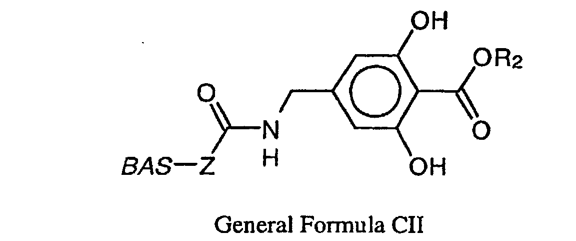

The conjugate of General Formula CII is next condensed with a hydroxylamine derivative to yield the corresponding phenylboronic acid complexing conjugate of the general formula of General Formula CIV:

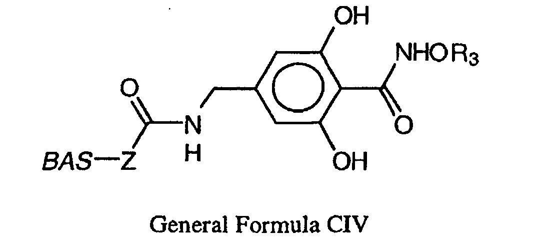

Suitable hydroxylamine derivatives are preferrably selected from, but not limited to, NH₂OH, NH₂OCH₃, NH₂OCH₂CN, NH₂OCH₂COOH, NH₂OCH₂CONH₂, NH₂OCH₂CH₂OH, NH₂OCH₂OCH₃. When group R₂ in General Formula CII is an alkyl group, NH₂OH is preferably utilized to effect the interconversion of General Formula CII to General Formula CIV.

### PREPARATION OF BIOCONJUGATES OF GENERAL FORMULA CVI

Bioconjugates of General Formula CVI may be prepared in buffered aqueous solutions or organic solvents. Preferred buffers include acetate, citrate, phosphate, carbonate and diglycine. Borate buffers should be avoided due to their ability to complex with the phenylboronic acid complexing moiety. Tris, β-hydroxyamine and β-hydroxyacid buffers should be avoided due to their ability to complex with the phenylboronic acid. The bioconjugate is formed within a few minutes over a range of temperatures of from about 4°C to 70°C. The stability of the bioconjugate in aqueous solution at a given pH and temperature is influenced, to some extent, by substituent group R₃. Bioconjugates of General Formula CVI are stable in aqueous solutions of approximate pH greater than 2.5 and less than 12.5. The bioconjugation reaction (phenylboronic acid complexation) is insensitive to significant variations in ionic strength over the range 0.01 to 1 M, the presence of organic solvents including acetonitrile, methanol, ethanol, isopropanol, butanol, *N,N*-dimethylformamide and dimethylsulfoxide, the presence of detergents, and the presence of chaotropic agents (protein denaturants) including urea, guanidine hydrochloride, guanidine thiocyanate and formamide, which are incompatible with prior art indirect labeling systems wherein the structure of a biological macromolecule must be maintained to preserve requisite binding properties. Once formed, the bioconjugates are stable upon removal of water, and can be lyophilized for storage.

The stability of the bioconjugate at a given pH is determined to some extent by substituent group R₃. Phenylboronic acid complexes of General Formula CVI, wherein group R₃ is H, are stable in buffered aqueous solutions over the approximate pH range 2.5 to 12.5. Phenylboronic acid complexes of General Formula CVI, wherein group R₃ is CH₃, are stable in buffered aqueous solutions over the approximate pH range 3.5 to 12.5. Phenylboronic acid complexes of General Formula CVI, wherein group R₃ includes an electronegative moiety, are stable in buffered aqueous solutions over the approximate pH range less than 2.5 to 12.5.

The stability of the phenylboronic acid complex toward acid catalyzed hydrolysis is related to the pKₐ of the hydroxamic acid participating in the complex. The lower the pKₐ of the hydroxamic acid moiety the more stable the complex. Consequently, phenylboronic acid complexes of General Formula CVI wherein group R₃ is an electronegative moiety exhibit greater stability toward acid catalyzed hydrolysis than do those in which R₃ is either H or CH₃.

It should be appreciated that the pH stability associated with bioconjugates of General Formula CVI, derived from 4-aminomethyl-2,6-dihydroxybenzohydroxamic acid, is significantly better than that of bioconjugaes of General Formula VI, derived from either 4- or 5-aminomethyl-salicylhydroxamic acid, with respect to both acid and base catalyzed hydrolysis.

The following examples present a detailed description of the synthesis of reagents of General Formula CI and General Formula CIII.

### Example CI

### Preparation of Methyl 4-Aminomethyl-2,6-dihydroxybenzoate Hydrochloride

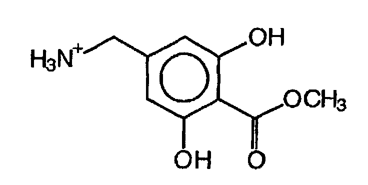

### Methyl 4-Methyl-2, 6-diacetoxybenzoate.

Methyl 4-methyl-2,6-dihydroxybenzoate (14.0 grams, 76.8 mmoles) was dissolved in anhydrous dichloromethane (250 mL) and acetic anhydride (21 mL, 223 mmoles) and anhydrous pyridine (18 mL, 223 mmoles) were added carefully. The solution was refluxed under dry nitrogen for 30 hours, then cooled to room temperature. The solution was washed twice with 1 M aqueous hydrochloric acid (200 mL portions) and then with saturated aqueous sodium chloride (200 mL). The dichloromethane solution was dried over anhydrous magnesium sulfate, filtered, and evaporated to an off-white solid. This crude product was flash chromatographed on silica gel (250 grams) using hexanes:ethyl acetate (70:30 [v/v]) as the eluting solvent. Fractions containing the major product (R_{f} = 0.3) were pooled and evaporated to dryness to afford 19.0 grams (98% yield) of a white solid of methyl 4-methyl-2,6-diacetoxybenzoate (m.p. 70-71° C, open capillary, uncorrected).

¹H NMR (300 MHz, CHCl₃ *d*) δ 2.27 (singlet, 6H, COC**H**₃), 2.37 (singlet, 3H, ArC**H**₃), 3.83 (singlet, 3H, OC**H**₃), 6.84 (singlet, 2H, Ar**H**). ¹³C NMR (75 MHz, CHCl₃ *d*) δ 20.9, 21.5, 52.4, 116.5, 122.1, 143.8, 150.2, 163.9, 169.4.

### Methyl 4-bromomethyl-2,6-diacetoxybenzoate.

Methyl 4-methyl-2,6-diacetoxybenzoate (14.9 grams, 56.0 mmoles) was dissolved in carbon tetrachloride (200 mL), and *N*-bromosuccinimide (11.1 grams, 62.2 mmoles) and benzoyl peroxide (0.2 grams, 0.8 mmoles) were added. The mixture was refluxed under nitrogen. After 3.5 hours, an additional portion (0.2 grams) of *N*-bromosuccinimide was added. Reflux was continued for an additional hour. The reaction mixture was cooled to room temperature and then in ice for 1 hour, and the solid removed by filtration. The filtrate was evaporated to dryness. Hexanes (250 mL) was added to the residue, and the mixture was boiled until dissolution was obtained. The hexanes solution was concentrated to about 75 mL when a solid just began to precipitate. The mixture was heated to dissolve the solid, and the solution was allowed to cool slowly to room temperature. White crystals formed slowly, and the mixture was chilled in ice for 2 hours to complete crystallization. The solid was filtered, washed with cold hexanes (100 mL), and dried *in vacuo* to afford 11.9 grams (62% yield) of methyl 4-bromomethyl-2,6-diacetoxy-benzoate (m.p. 93-95° C, open capillary, uncorrected). The product was contaminated with a small amount of methyl 2,6-diacetoxy-4-dibromomethylbenzoate, which did not interfere with subsequent reactions.

¹H NMR (300 MHz, CHCl₃₋ *d*) δ 2.29 (singlet, 6H, COC**H**₃), 3.85 (singlet, 3H, OC**H**₃), 4.41 (singlet, 2H, C**H**₂), 7.08 (singlet, 2H, Ar**H**). ¹³C NMR (75 MHz, CHCl₃₋*d*) δ 20.9, 30.9, 52.7, 119.6, 121.8, 142.4, 150.2, 163.5, 169.1.

### Methyl 4-aminomethyl-2,6-dihydroxybenzoate hydrochloride.

Methyl 4-bromomethyl-2,6-diacetoxybenzoate (45.8 grams, 133 mmoles) was dissolved in N,N-dimethylformamide (150 mL), and sodium azide (8.8 g, 135 mmoles) was added. The reaction mixture was stirred for 6 hours at room temperature. Dichloromethane (350 mL) was added to the reaction mixture, and this solution was washed with water (250 mL), 1 M aqueous hydrochloric acid (250 mL), and saturated aqueous sodium chloride (150 mL). The organic solution was dried over anhydrous magnesium sulfate, filtered, and evaporated to a clear, pale yellow oil. The oil was dissolved in methanol (750 mL) and transferred to a 2L Parr hydrogenation flask. Palladium on carbon catalyst (10% [w/w], 1.5 g) in water (10 mL) was added, followed by concentrated hydrochloric acid (15 mL). The flask was affixed to a Parr hydrogenator, and the mixture was shaken at room temperature under 30 psi of hydrogen for 24 hours. The reaction mixture was filtered through a 0.45 mm nylon filter. The retained solid was then washed with methanol (150 mL), and concentrated hydrochloric acid (7 mL) was added to the filtrate. The filtrate was heated to reflux for 2 hours, cooled to room temperature, and evaporated to dryness to give an off-white solid. This solid was dissolved in hot denatured ethanol (250 mL) and the solution was allowed to cool to room temperature. White crystals formed quickly. The mixture was then chilled for 16-18 hours at 4°C to complete crystallization. The solid was filtered, washed with a little cold ethanol (50 mL) and then diethyl ether (150 mL), and dried *in vacuo* over potassium hydroxide pellets to afford 16.0 grams (52% yield based on monobromo starting material) of methyl 4-aminomethyl-2,6-dihydroxybenzoate hydrochloride (m.p. >260°C, open capillary, uncorrected).

¹H NMR (300 MHz, DMSO-*d*_{*6*}) δ 3.77 (singlet, 3H, OC**H**₃), 3.83 (singlet, 2H, C**H**₂), 6.44 (singlet, 2H, Ar**H**), 8.35 (broad singlet, 3H, N**H**₃), 10.20 (singlet, 2H, O**H**). ¹³C NMR (75 MHz, DMSO-*d*_{*6*}) δ 41.9, 52.1, 107.2, 107.3, 138.5, 157.6, 168.3.

### Example CII

### Preparation of a Reagent of General Formula CI Methyl (6-Aminohexanoyl)aminomethyl-2,6-dihydroxybenzoate Hydrochloride

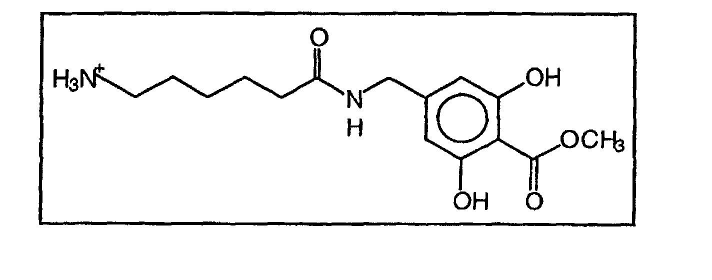

### Methyl (N-tert-butoxycarbonyl-6-aminohexanoyl)aminomethyl-2,6-dihydroxybenzoate.

Methyl 4-aminomethyl-2,6-dihydroxybenzoate hydrochloride (1,50 grams, 6.4 mmoles) was suspended in anhydrous *N*,*N*-dimethylformamide (25 mL), and *N*,*N*-diisopropylethylamine (2.2 mL, 12.8 mmoles) was added, followed by *N-tert*-butoxycarbonyl-6-aminohexanoic acid succinimidyl ester (2.10 grams, 6.4 mmoles). The mixture was stirred under dry nitrogen for 4 hours, during which time all solids dissolved. The solvent was then evaporated to leave a light brown syrup, which was partitioned between ethyl acetate (70 mL) and 1 M aqueous hydrochloric acid (50 mL). The layers were separated, and the ethyl acetate solution was washed with saturated aqueous sodium bicarbonate (50 mL) and saturated aqueous sodium chloride (50 mL). The ethyl acetate solution was dried over anhydrous sodium sulfate, filtered, and evaporated to an amorphous off-white solid. The solid was crystallized from ethyl acetate/hexanes, filtered, and dried *in vacuo* to afford 2.10 grams (80% yield) of methyl (*N*-*tert*-butoxycarbonyl-6-aminohexanoyl)aminomethyl-2,6-dihydroxybenzoate (m.p. 73-76°C, open capillary, uncorrected).

¹H NMR (300 MHz, DMSO-*d*_{*6*}) δ 1.26 (multiplet, 2H, NHCH₂CH₂C**H**₂CH₂CH₂CO), 1.36 (multiplet, 2H, C**H**₂CH₂CO), 1.36 (singlet, 9 H, C(C**H**₃)₃), 1.50 (multiplet, 2 H, NHCH₂C**H**₂), 2.10 (triplet, *J* = 7 Hz, 2 H, CH₂C**H**₂CO), 2.87 (quartet, *J* = 6 Hz, 2 H, NHC**H**₂CH₂), 3.78 (singlet, 3 H, OC**H**₃), 4.08 (doublet, *J =* 6 Hz, 2 H, C**H**₂Ar), 6.23 (singlet, 2H, Ar**H**), 6.75 (triplet, *J* = 6 Hz, 1 H, CON**H**CH₂CH₂), 8.26 (triplet, J = 6 Hz, I H, CON**H**CH₂Ar), 10.04 (singlet, 2H, O**H**). ¹³C NMR (75 MHz, CHCl₃-*d*) δ 25.0, 26.0, 28.3, 29.3, 35.3, 41.7, 52.0, 77.4, 104.3, 105.6, 145.8, 155.8, 158.2, 169.0, 172.4.

### Methyl (6-aminohexanoyl)aminomethyl-2,6-dihydroxybenzoate hydrochloride.

Methyl (*N*-*tert*-butoxycarbonyl-6-aminohexanoyl)aminomethyl-2,6-dihydroxybenzoate (2.00 grams, 4.87 mmoles) was dissolved in ethyl acetate (50 mL), and dry hydrogen chloride was bubbled slowly through the solution. The reaction mixture warmed as the gas dissolved. After 5 minutes, the gas was shut off, and the solution was stirred at room temperature. A white precipitate formed in the solution. After 30 minutes, the reaction mixture was chilled in ice for 2 hours, then the solid was filtered, washed with ethyl acetate (50 mL) and then diethyl ether (50 mL), and dried *in vacuo* over potassium hydroxide pellets to afford 1.68 grams (99% yield) of methyl (6-aminohexanoyl)aminomethyl-2,6-dihydroxybenzoate hydrochloride (m.p. shrinks at 145°C, decomposes with effervescence at 152-154°C, open capillary, uncorrected).

¹H NMR (300 MHz, DMSO-*d*_{*6*}) δ 1.29 (multiplet, 2H, H₃CH₂CH₂C**H**₂CH₂CH₂CO), 1.55 (multiples, 4H, NH₃CH₂C**H**₂CH₂C**H**₂CH₂CO), 2.13 (triplet, *J* = 8 Hz, 2 H, CH₂C**H**₂CO), 2.70 (multiplet, 2 H, NH₃C**H**₂CH₂), 3.75 (singlet, 3 H, OC**H**₃), 4.07 (doublet, *J* = 6 Hz, 2 H, C**H**₂Ar), 6.26 (singlet, 2 H, Ar**H**), 8.05 (broad singlet, 3 H, NH₃), 8.39 (triplet, J = 6 Hz, 1 H, CON**H**CH₂Ar), 9.84 (broad singlet, 2 H, O**H**). ¹³C NMR(75 MHz, CHCl₃-*d*) δ 24.8, 25.6, 26.7, 35.1, 41.8, 52.0, 104.7, 105.5, 145.5, 158.1, 169.0, 172.3.

### Example CIII

### Preparation of a Reagent of General Formula CI Methyl N-Bromoacetyl-4-aminomethyl-2,6-dihydroxybenzoate

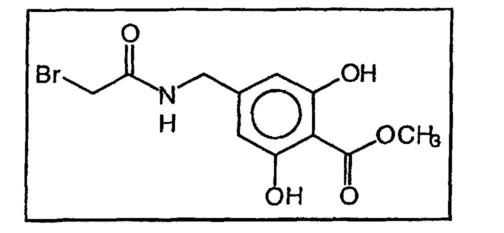

### Methyl N-Bromoacetyl-4-aminomethyl-2,6-dihydroxybenzoate.

Bromoacetic acid (0.30 grams, 2.16 mmol) was dissolved in dry *N,N*-dimethylformamide (15 mL) and *N*-hydroxysuccinimide (0.25 grams, 2.21 mmol) and 1,3-dicyclohexylcarbodiimide (0.44 grams, 2.14 mmol) were added. The solution was stirred at room temperature for 1.25 hours. Methyl 4-aminomethyl-2,6-dihydroxybenzoate hydrochloride (0.50 grams, 2.14 mmol) was added, followed by *N,N*-diisopropylethylamine (0.38 mL, 2.15 mmol). The reaction mixture was stirred at room temperature for 2.5 hours, and was then diluted with ethyl acetate (100 mL). The mixture was filtered, and the filtrate washed twice with 1 N aqueous hydrochloric acid (100 mL then 50 mL) and with saturated aqueous sodium chloride (50 mL). The organic solution was dried over anhydrous magnesium sulfate, filtered, and evaporated to dryness. The crude product was purified by chromatography on silica gel, eluting with hexanes:ethyl acetate (1:1 [v/v]). Fractions containing pure product were pooled and evaporated to dryness to afford 0.43 grams of a white solid (63% yield) of methyl N-bromoacetyl-4-aminomethyl-2,6-dihydroxybenzoate (m.p. 157 - 158°C).

¹H NMR (300 MHz, DMSO-*d*_{*6*}) δ 3.77 (singlet, 3H, OC**H**₃), 4.11 (singlet, 2H, BrC**H**₂), 4.13 (doublet, *J*= 6Hz, 2H, C**H**₂NH), 6.25 (singlet, 2H, Ar**H**), 8.70 (triplet, *J* = 6 Hz, 1H, CH₂N**H**), 10.05 (singlet, 2H, O**H**). ¹³C NMR (75 MHz, DMSO-*d*_{*6*}) δ 42.3, 42.6, 52.1, 104.7, 105.7, 144.7, 158.2, 166.3, 168.9.

### Example CIV

### Preparation of a Reagent of General Formula CI Methyl 4-Glutarylaminomethyl-2,6-dihydroxybenzoate Succinimidyl Ester

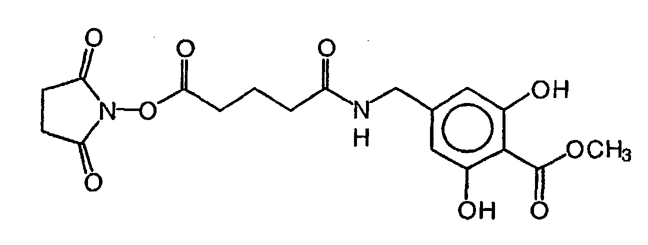

### Methyl 4-glutarylaminomethyl-2,6-dihydroxybenzoate.

Glutaric anhydride (2.3grams, 20 mmoles) was dissolved in dry tetrahydrofuran (100 mL), and the suspension was stirred under nitrogen. *N*,*N*-diisopropylethylamine (7.0 mL, 40 mmoles) was added, followed by methyl 4-aminomethyl-2,6-dihydroxybenzoate hydrochloride (4.7 grams, 20 mmoles). After stirring for 18 hours, the mixture was evaporated to dryness, and the resulting solid was dissolved in dichloromethane (100 mL) and allowed to sit at room temperature for 2 hours. The dichloromethane solution was evaporated to dryness to give a thick syrup, which was treated with cold 1 M aqueous hydrochloric acid (100 mL) for 30 minutes in ice. An off-white solid precipitated, which was filtered, washed with cold water, and dried *in vacuo* to afford 5.5 grams (88% yield) of methyl 4-glutarylaminomethyl-2,6-dihydroxybenzoate (m.p. 163-164°C).

¹H (300 MHz, DMSO-*d*_{*6*}) δ 1.74 (quintet, *J* = 8 Hz, 2H, CH₂C**H**₂CH₂), 2.16 (triplet, *J* = 8 Hz , 2H, C**H**₂CONH), 2.20 (triplet, *J* = 8 Hz , 2H, HO₂CC**H**₂), 3.78 (singlet, 3H, OC**H**₃), 4.10 (doublet, *J* = 6 Hz, 2H, ArC**H**₂NH), 6.23 (singlet, 2H, Ar**H**), 8.31 (triplet, *J* = 6 Hz, 1H, N**H**), 10.05 (broad singlet, 3H, O**H** and CO₂**H**). ¹³C NMR (75 MHz, DMSO-*d*_{*6*}) δ 20.7, 33.1, 34.4, 41.8, 52.1, 104.4, 105.6, 145.7, 158.2, 169.0, 172.0, 174.5.

### Methyl 4-glutarylaminomethyl-2,6-dihydroxybenzoate succinimidyl ester.

Methyl 4-glutarylaminomethyl-2,6-dihydroxybenzoate (2.0 grams, 6.4 mmoles) was dissolved in dry tetrahydrofuran (80 mL), and *N*-hydroxysuccinimide (0.8 grams, 7.0 mmoles) and 1,3-dicyclohexylcarbodiimide (1.3 grams, 6.4 mmoles) were added. The mixture was stirred at room temperature under dry nitrogen, and the solids rapidly dissolved. After about 20 minutes, a white precipitate formed. The reaction mixture was stirred 16-18 hours, then chilled several hours at -20°C. The mixture was filtered cold, and the solid washed with a little tetrahydrofuran (25 mL). The combined filtrates were evaporated to dryness, and the residue was crushed under ice-cold water (25 mL). The solid was filtered, washed quickly with cold water (25 mL) and then diethyl ether (100 mL), and dried *in vacuo*. The solid was recrystallized from ethyl acetate/hexanes to afford 2.5 grams (95% yield) of methyl 4-glutarylaminomethyl-2,6-dihydroxy-benzoate succinimidyl ester (m.p. 161-163°C).

¹H NMR (300 MHz, DMSO-*d*_{*6*}) δ 1.83 (quintet, *J* = 8 Hz, 2H, CH₂C**H**₂CH₂), 2.26 (triplet, *J* = 8 Hz, 2H, C**H**₂CONH), 2.73 (triplet, *J* = 8 Hz, 2H, NO₂CC**H**₂), 2.80 (singlet, 4H, COC**H**₂C**H**₂CO), 3.77 (singlet, 3H, OC**H**₃), 4.10 (doublet, *J* = 6 Hz , 2H, ArC**H**₂NH), 6.24 (singlet, 2H, Ar**H**), 8.35 (triplet, *J* = 6 Hz, 1H, N**H**), 10.04 (broad singlet, 2H, O**H**). ¹³C NMR (75 MHz, DMSO-*d*_{*6*}) δ 20.4, 25.4, 29.7, 33.6, 41.8, 52.0, 104.5, 105.6, 145.5, 158.1, 168.9, 170.5, 171.4.

### Example CV

### Preparation of a Reagent of General Formula CI Methyl 4-Glutarylaminomethyl-2,6-dihydroxybenzoic Acid Hydrazide

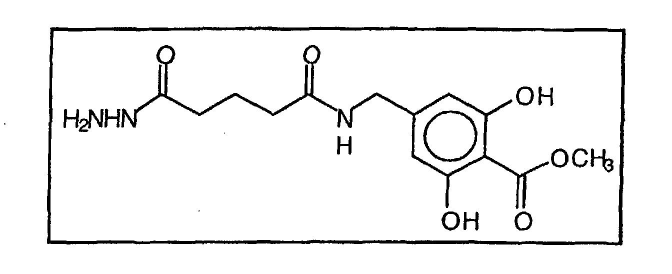

### Methyl 4-glutarylaminomethyl-2,6-dihydroxybenzoate N-tert-butyloxycarbonylhydrazide.

Methyl 4-glutarylaminomethyl-2,6-dihydroxybenzoate succinimidyl ester (1.6 grams, 3.9 mmoles) was suspended in dry dichloromethane (50 mL), and *tert*-butylcarbazate (0.50 grams, 3.9 mmoles) was added. The reaction was stirred at room temperature for 24 hours. The pale solution was then washed twice with saturated aqueous sodium bicarbonate (50 mL portions), 0.1 M aqueous hydrochloric acid (50 mL), and saturated aqueous sodium chloride (50 mL). It was then dried over anhydrous sodium sulfate, filtered, and evaporated to dryness. The residue was flash chromatographed on a 2.5 X 150 cm column of silica gel, eluting with ethyl acetate. Fractions containing pure product were pooled and evaporated to a clear syrup. This syrup was crystallized from ethyl acetate:hexanes at -20°C. The solid was filtered, washed with cold hexanes, and dried *in vacuo* to afford 1.0 grams (62% yield) of methyl 4-glutarylaminomethyl-2,6-dihydroxybenzoate *N*-*tert*-butyloxycarbonylhydrazide (m.p. 95-100°C, softens at 88°C, open capillary, uncorrected).

¹H (300 MHz, DMSO-*d*_{*6*}) δ 1.37 (singlet, 9H, (CH₃)₃C), 1.74 (quintet, *J* = 7 Hz, 2H, CH₂C**H**₂CH₂), 2.07 (triplet, *J* = 7 Hz, 2H, C**H**₂CONHCH₂), 2.15 (triplet, *J* = 7 Hz, 2H, HNHNOCC**H**₂), 3.77 (singlet, 3H, OC**H**₃), 4.09 (doublet, *J* = 6 Hz, 2H, ArC**H**₂NH), 6.23 (singlet, 2H, Ar**H**), 8.28 (triplet, *J* = 6 Hz, 1H, N**H**), 8.65 (singlet, 1H, NHN**H**COCH₂), 9.47 (singlet, OCON**H**NH), 9.96 (singlet, 2H, O**H**). ¹³C NMR (75 MHz, DMSO-*d*_{*6*}) δ 21.2, 28.0, 32.7, 34.7, 41.7, 52.1, 79.1, 104.4, 105.6, 145.7, 155.5, 158.2, 169.0, 171.7, 172.0.

### Methyl 4-Glutarylaminomethyl-2, 6-dihydroxybenzoic Acid Hydrazide.

Methyl 4-glutarylaminomethyl-2,6-dihydroxybenzoate *N*-*tert*-butyloxycarbonyl hydrazide (0.50 grams, 1.2 mmoles) is dissolved in dry tetrahydrofuran (25 mL), and dry hydrogen chloride was bubbled slowly through the solution. The reaction mixture warmed as the gas dissolved. After 5 minutes, the gas was shut off, and the solution was stirred at room temperature. A white precipitate formed in the solution. After 30 minutes, the reaction mixture was chilled in ice for 2 hours, then the solid was filtered, washed with diethyl ether (50 mL), and dried *in vacuo* over potassium hydroxide pellets to afford 0.42 grams (98% yield) of methyl 4-glutarylaminomethyl-2,6-dihydroxybenzoic acid hydrazide (m.p. 158-159°C, decomposed with effervescence, open capillary, uncorrected).

¹H (300 MHz, DMSO-*d*_{*6*}) δ 1.76 (quintet, *J* = 7 Hz, 2H, CH₂C**H**₂CH₂), 2.16 (triplet, *J* = 7 Hz, 2H, C**H**₂CONHCH₂), 2.21 (triplet, *J* = 7 Hz , 2H, H₃NHNOCC**H**₂), 3.75 (singlet, 3H, OC**H**₃), 4.07 (doublet, *J* = 6 Hz, 2H, ArC**H**₂NH), 5.00-7.00 (very broad singlet, 2H, O**H**), 6.25 (singlet, 2H, Ar**H**), 8.39 (triplet, *J* = 6 Hz, 1H, N**H**), 10.49 (broad singlet, 3H, NHN**H**₃), 11.09 (singlet, 1H, N**H**NH₃). ¹³C NMR(75 MHz, CHCl₃-*d*) δ 20.9, 32.2, 34.4, 41.8, 52.0, 104.8, 105.6, 145.5, 158.1, 169.0, 171.6, 171.9.

### Example CVI

### Preparation of a Reagent of General Formula CIII Synthesis of 4-Glutarylaminomethyl-2,6-dihydroxybenzohydroxamic Acid Hydrazide

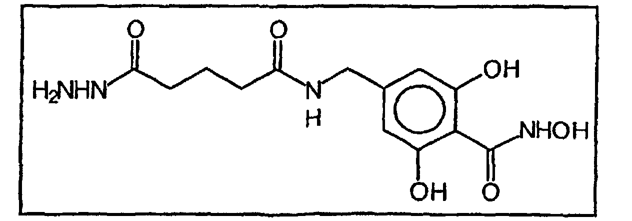

### 4-Glutarylaminomethyl-2,6-dihydroxybenzohydroramic Acid N-tert-Butyloxycarbonylhydrazide.

Methyl 4-glutarylaminomethyl-2,6-dihydroxybenzoate *N-tert*-butyloxycarbonylhydrazide (0.30 grams, 0.7 mmoles) was added to a cooled (ice/water bath) solution of hydroxylamine sulfate (0.12 grams, 0.7 mmoles), sodium hydroxide (0.15 grams, 3.6 mmoles) and sodium sulfite (0.05 grams, 0.4 mmoles) in water (6 mL). The suspension was stirred for 24 hours in the dark, allowing it to warm to room temperature. The solution was dilute with I M hydrochloric acid (4 mL), and extracted twice with ethyl acetate (15 mL portions). The combined ethyl acetate extracts were washed with water (10 mL) and saturated aqueous sodium chloride (10 mL), dried over anhydrous magnesium sulfate, filtered, and evaporated to dryness. The residue was dissolved in a minimum volume of ethyl acetate, and diethyl ether was added to precipitate an orange gummy material. The gum was triturated under dry diethyl ether to produce a solid, which was filtered, washed with ether, and dried *in vacuo* to afford 0.21 grams (69% yield) of 4-glutarylaminomethyl-2,6-dihydroxybenzohydroxamic acid *N*-tert-butyloxycarbonylhydrazide (m.p. decomposed with effervescence at 60-65°C, open capillary, uncorrected).

¹H (300 MHz, DMSO-*d*_{*6*}) δ 1.37 (singlet, 9H, (CH₃)₃C), 1.73 (quintet, *J* = 7 Hz, 2H, CH₂C**H**₂CH₂), 2.07 (triplet, *J* = 7 Hz, 2H, C**H**₂CONHCH₂), 2.15 (triplet, *J* = 7 Hz, 2H, HNHNOCC**H**₂), 4.08 (doublet, *J* = 6 Hz, 2H, ArC**H**₂NH), 6.22 (singlet, 2H, Ar**H**), 8.28 (triplet, *J* = 6 Hz, 1H, N**H**), 8.65 (singlet, 1H, NHN**H**COCH₂), 9.47 (singlet, OCON**H**NH), 10.77 (broad singlet, 1H, N**H**OH), 12.30 (broad singlet, 3H, ArO**H** and NHO**H**). ¹³C NMR (75 MHz, DMSO-*d*_{*6*}) δ 21.3, 28.1, 32.7, 34.7, 41.7, 79.1, 100.1, 105.6, 145.8, 155.6, 159.8, 167.5, 171.7, 172.0.

### 4-Glutarylaminomethyl-2,6-dihydroxybenzohydroxamic acid hydrazide trifluoroacetate.

4-Glutarylaminomethyl-2,6-dihydroxybenzohydroxamic acid *tert*-butyl carbazate (0.09 grams, 0.2 mmoles) was suspended in dry dichloromethane (5 mL), and trifluoroacetic acid (0.5 mL) was added. The solid dissolved on addition of the acid, and the reaction was stirred at room temperature for three hours. After one hour, an orange precipitate had formed. The precipitate was collected by filtration and dried *in vacuo* over potassium hydroxide pellets to afford 0.10 grams (100% yield) of 4-glutarylaminomethyl-2,6-dihydroxybenzohydroxamic acid hydrazide trifluoroacetate (m.p. 104-108°C, open capillary, uncorrected).

¹H (300 MHz, DMSO-*d*_{*6*}) δ 1.80 (quintet, *J* = 7 Hz, 2H, CH₂C**H**₂CH₂), 2.17 (triplet, *J* = 7 Hz, 2H, C**H**₂CONHCH₂), 2.23 (triplet, *J* = 7 Hz, 2H, HNHNOCC**H**₂), 4.09 (doublet, *J* = 6 Hz, 2H, ArC**H**₂NH), 6.22 (singlet, 2H, Ar**H**), 8.32 (triplet, *J* = 6 Hz, 1H, N**H**), 8.95 - 10.60 (very broad singlet, 5H, N**H**N**H**₃ and N**H**OH), 11.80-12.95 (very broad singlet, 3H, ArO**H** and NHO**H**). ¹³C NMR (75 MHz, DMSO-*d*_{*6*}) δ 20.9, 32.2, 34.4, 41.8, 100.1, 105.7, 145.8, 158.5, 159.0, 159.9, 167.5, 171.8, 171.9.

### Example CVII

### Preparation of Conjugates of General Formula CIV

### Synthesis of 5'-PBA-labeled Oligodeoxyribonucleotide Conjugates.

Oligodeoxyribonucleotide 7172 (sequence 5'-GATTACGCCAGTTGTACGGAC-3,) was synthesized on a 1 _mole scale using standard automated phosphoramidite chemistry (Beckman Instruments Oligo 1000 and associated reagents). A protected amine-containing phosphor-amidite (Aminolink 2, Applied Biosystems or UniLink Amino Modifier, Clontech) was employed on the same instrument to introduce one to four, reactive primary amines onto the 5'-end of the oligodeoxyribonucleotide using standard chemistry. The completed oligodeoxyribo-nucleotide was then cleaved from the support and the nucleobases deprotected using an UltraFast Deprotection kit (Beckman Instruments) and the protocol supplied by the manufacturer.

The amino-oligonucleotides were purified by ethanol precipitation, dissolved in 0.8 mL of 0.1 M NaHCO₃, and condensed with phenylboronic acid reagent (PBA-Z-NHS) having a reactive *N*-hydroxysuccinimidyl ester moiety (5 mgs per mmole of primary amino groups on the amino-oligonucleotide in 0.2 mL of anhydrous *N,N*-dimethylformamide) for 2-18 hours at room temperature.

The crude PBA-modified oligonucleotide was isolated from the reaction mixture by gel filtration on a KwikSep Dextran column (Pierce Chemical) in 0.1 M aqueous triethylammonium acetate, pH 6.5. The PBA-modified oligonucleotide was then concentrated in a vacuum centrifuge to 1 mL, and purified by reverse phase HPLC on a 4.6 mm x 250 mm C18 column, with a triethylammonium acetate-acetonitrile gradient. The desired product peak was collected and evaporated to a small pellet in a vacuum centrifuge, dissolved in 0.5 mL of water, and stored frozen.

### Preparation of Salicylhydroxamic Acid Magnetic Beads.

Ten milliliters of unmodified M280 or M450 magnetic beads (Dynal) were gradually dehydrated into acetonitrile, and converted to aldehyde modified beads using oxalyl chloride activated *N,N*-dimethylsulfoxide and triethylamine in dichloromethane at -78° C. The resulting aldehyde bearing beads were gradually rehydrated and suspended in 5 mL of 0.1 M sodium acetate pH 5.5. The aldehyde groups were coupled with 4-glutarylaminomethylsalicyl-hydroxamic acid hydrazide (SHA-Z-NHNH₂) by adding 15-25 mgs dissolved in 200 uL *N*,*N*-dimethylsulfoxide, and rotating coupling reaction over night at room temperature. The beads were then washed extensively with water and stored in 5 mL of 10% ethanol.

### Preparation of Salicylhydroxamic Acid (SHA) Sepharose 4B.

SHA-Sepharose 4B was prepared by mixing 130 mg of (6-aminohexanoyl)-4-amino-methylsalicylhydroxamic acid (SHA-Z-NH₂), dissolved in 30 mL 0.2 M NaHCO₃, with. 6.5 g HCl washed CNBr activated Sepharose 4B (Pharmacia) overnight at room temperature. After the coupling reaction, 2 mL of 0.5 M Tris, pH 8.5 were added and the gel slurry mixed at room temperature for 1 hour, and washed with water, 0.5 M NaCl, and water again. The resulting SHA-Sepharose 4B was suspended in 30 mL of 20% ethanol, and stored at 4° C.

### Preparation of 2,6-Dihydroxybenzohydroxamic Acid (DHBHA) Sepharose 4B.

DHBHA-Sepharose 4B was prepared by mixing 114 mg methyl (6-aminohexanoyl)-4-aminomethyl-2,6-dihydroxybenzoate [DHBA(OMe)-X-NH₂], dissolved in 30 mL 0.2 M NaHCO₃, with 5 g HCl washed CNBr activated Sepharose 4B (Pharmacia), overnight at room temperature. After the coupling reaction, the gel was washed with water and suspended in 50 mL 0.1 M NH₂OH, pH 9, and rotated at room temperature for two hours. Finally, the gel was washed with water and suspended in 30 mL of 20% ethanol, and stored at 4°C.

### Preparation of a Phenylboronic Acid-α-Biotin Antibody Conjugate.

One milliliter of α-Biotin monoclonal IgG₁ antibody (6.5 mg/mL in 0.1 M NaHCO₃) was conjugated with 440 nmoles of PBA-Z-NHS (7.4 ul of 60 mM PBA-Z-NHS dissolved in *N*,*N*-dimethylsulfoxide) for 1 hour at room temperature. Unconjugated PBA-Z-NHS and its hydrolysis products were removed by dialysis. The ultra-violet absorbance spectrum of the resulting conjugate (PBA-α-Biotin) exhibited an increase in A₂₆₀ relative to A₂₈₀ consistent with phenylboronic acid modification.

### Preparation of a Phenylboronic Acid-Alkaline Phosphatase Conjugate.

One milliliter of alkaline phosphatase (Sigma, 6 mg/mL) was dialyzed against one liter of 0.1 M NaHCO₃, and conjugated with 700 nmoles of PBA-Z-NHS (10 uL of 70 mM stock in *N*,*N*-dimethylformamide) for two hours on ice. Unconjugated PBA-Z-NHS and its hydrolysis products were removed by dialysis in 0.1 M NaHCO₃. The ultra-violet absorbance spectrum of the resulting conjugate (PBA-AP) exhibited an increase in A₂₆₀ relative to A₂₈₀ consistent with phenylboronic acid modification. The conjugate was stored at 4° C.

### Preparation of a 2,6-Dihydroxybenzohydroxamic Acid-Alkaline Phosphatase Conjugate.

One milliliter of alkaline phosphatase (Sigma, 6 mg/mL) was dialyzed against one liter of 0.1 M NaHCO₃, and conjugated with 714 nmoles of methyl 4-glutarylaminomethyl-2,6-di-hydroxybenzoate succinimidyl ester [DHBA(OMe)-Z-NHS] (10.5 uL of 68 mM in *N*,*N*-dimethylformamide) for two hours on ice. The methyl ester of the conjugate was converted to a hydroxamic acid by adding one milliliter of 2 M NH₂OH, pH 10, and incubating the reaction at 4° C for six days. The NH₂OH reaction mixture was then dialyzed against 0.1 M NaHCO₃ and stored at 4° C.

### Preparation of a Salicylhydroxamic Acid-Goat α-Mouse Antibody Conjugate.

Two milliliters of goat α-mouse antibody (Rockland, 8.8 mg/mL in 0.1 M NaHCO₃) were conjugated with 2.35 umoles of methyl 4-glutarylaminomethylsalicylate succinimidyl ester [SA(OMe)-Z-NHS] for 1 hour at room temperature. The methyl ester of the conjugate was converted to a hydroxamic acid by adding two milliliters of 2 M NH₂OH, pH 10, adjusting the pH to 10 with 1 N NaOH, and incubating the reaction at room temperature for three days. NH₂OH and unconjugated SA(OMe)-X-NHS and its hydrolysis products were removed by gel filtration on a G-25 Sephadex column (Pharmacia) in 0.1 M NaHCO₃, and the conjugate (SHA-goat α-mouse) was stored at 4°C.

### Preparation of a 2,6-Dihydroxybenzohydroxamic Acid-Goat α-Mouse Antibody.

Two milliliters of goat α-mouse antibody (Rockland, 8.8 mg/mL in 0.1 M NaHCO₃) were conjugated with 2.35 umoles of with DHBA(OMe)-Z-NHS for 1 hour at room temperature. The methyl ester of the conjugate was converted to a hydroxamic acid by adding two milliliters of 2 M NH₂OH, pH 10, adjusting the pH to 10 with 1 N NaOH, and incubating the reaction at room temperature for three days. NH₂OH and unconjugated DHBA(OMe)-X-NHS and its hydrolysis products were removed by gel filtration on a G-25 Sephadex column (Pharmacia) in 0.1 M NaHCO₃, and the conjugate (DHBHA-goat α-mouse) stored at 4°C.

### Polymerase Chain Reaction (PCR) Protocol.

A region of Lambda DNA (801bp) was amplified by the polymerase chain reaction. The PCR reaction contained 200 uM dATP, dCTP, dGTP, and dTTP in addition to Biotin- and PBA- modified oligonucelotide primers at 1uM in 1X PCR Buffer II (Perkin Elmer), Lambda DNA (1 ng/uL), and 1U of *Thermus aquaticus* DNA polymerase. The reaction mixture was denatured at 92° C for one minute and amplified by 35 cycles of PCR at 95° C for 10 seconds, 62° C for 20 seconds, and 72° C for 30 seconds, with a final extension at 72° C for 5 minutes. The reaction produced 50-100 ng of amplified product (801bp), which exhibited retarded mobility relative to unmodified PCR product during electrophoresis on 1% agarose gels in 50 mM Tris, 100 mM borate, 2 mM EDTA, pH 8.3.

### Example CVIII

### Preparation of Bioconjugates of General Formula CVI Sandwich Hybridization Detection of Nucleic Acid Probes

A 42-mer oligonucleotide was hybridized with two 21-mer oligonucleotides bearing 5'-PBA and Biotin labels in 1.5 M NaCI, 150 mM sodium citrate, pH 7, at 45° C for ten minutes. Twenty-five microliters of the hybridization mixture was mixed with twenty five microliters of M280 streptavidin-magnetic particles (Dynal) in a polypropylene microtiter plate well. After 30 minutes, the magnetic particles were captured in the bottom of the well with a magnetic plate, and washed five times with 150 mM NaCl, 20 mM Tris-HCl, 0.02% Tween20, pH 8.

One hundred microliters of DHBHA-AP (1 ug/mL in 1 mg/mL BSA, 140 mM NaCl, 10 mM Tris-HCl, pH 8) were added to the magnetic particles and mixed well. After 30 minutes, the magnetic particles were captured in the bottom of the well with a magnetic plate, and washed six times with 150 mM NaCI, 20 mM Tris-HCI, 0.02% Tween20, pH 8. Alkaline phosphatse substrate (1 mg/mL *p*-nitrophenylphosphate in 1 M diethanolamine buffer, 1 mM MgCl₂, 0.1 mM ZnCl₂, pH 10.4) was added, and incubated at 37° C for 90 minutes. The Absorbance at 405 nm (A₄₀₅) was measured with an ELISA plate reader (Molecular Devices).

A strong A₄₀₅ was produced when all components of the hybridization sandwich were present, and the signal was proportional to the amount of 42-mer present. Experimental controls lacking either the 42-mer, the Biotin-oligonucleotides and PBA-oligonucleotides did not produce a significant A₄₀₅.

### Example CIX

### Preparation of Bioconjugates of General Formula CVI

### Detection of PBA-Labeled PCR Product on DHBHA-Coated Microtiter Plates.

The wells of a polystyrene microtiter plate (Becton Dickinson) were coated with DHBHA by filling the wells with 200 uL of DHBHA-goat α-mouse conjugate (30 ug/mL in 0.1M NaHCO₃ pH 9.0) and incubating overnight at 4° C. The coating solution was removed and the plate backcoated with 5 mg/mL BSA (300 ul per well in 0.2 M NaHCO₃, pH 9.0) for 1 hour at room temperature. The BSA solution was removed by washing the plate five times with ELISA Wash Buffer (150 mM NaCI, 20 mM Tris-HCl, .02% Tween 20, pH 8.0).

One hundred microliters of unpurified PBA and biotin labeled PCR product were added to 900 ul of 1.5N NaCl, 150 mM sodium citrate, pH 7.0 (10X SSC) and serially-diluted in 10X SSC. One hundred microliters of the diluted PCR products were added to the wells and incubated for one hour at room temperature. The plate was then washed five times with ELISA Wash Buffer, and 100 ul of Streptavidin-Alkaline Phosphatase (Boehringer Mannheim, 0.2 U/mL in 1 mg/ml BSA, 140 mM NaCl, 10 mM Tris-HCl, pH 8.0) were added to each well and incubated for thirty minutes at room temperature.

The plate was washed 5 times with ELISA Wash Buffer, and 200 ul of p-nitrophenyl- phosphate (1 mg/mL in diethanolamine, 1 mM MgCl₂, 0.1 mM ZnCl₂, pH 10.4) were added to the plate and incubated at 37° C for 30-60 minutes. Less than 1 uL of PCR product was detected. PCR product lacking either PBA or biotin labels was not detected.

### Example CX

### Preparation of Bioconjugates of General Formula CVI Detection of PBA-Labeled PCR Product

### Detection of PBA-Labeled PCR Product on SHA-Magnetic Beads.

PBA-labeled PCR product (0.02 µL - 5 µL) was diluted into 25-100 µL of 1.5 M NaCl, 150 mM sodium citrate, pH 7 (10X SSC), and added to a polypropylene microtiter plate well containing SHA-magnetic particles (10-50 µl). The particles and PCR product were mixed occasionally for 30-60 minutes at room temperature. The magnetic particles were captured in the bottom of the wells with a magnetic plate and washed five times in 150 mM NaCl, 20 mM Tris-HCl, 0.02% Tween 20, pH 8 (ELISA Wash Buffer). One hundred microliters of streptavidin alkaline-phosphatase (Boehringher Mannheim, 0.2 U/mL in 1 mg/mL BSA, NaCl, Tris-HCl, pH 8) were added and mixed with the magnetic particles for 30 minutes at room temperature. The magnetic particles were captured in the bottom of the wells with a magnetic plate and washed 5 times with ELISA Wash. Alkaline phosphatase substrate was added (1 mg/ml *p*-nitrophenyl phosphate in 1M diethanolamine buffer, 1 mM MgCl₂, 0.1 mM ZnCl₂, pH 10.4), and the color developed at 37° C for 10-60 minutes. The lower limit of detection was 50 pg of PCR product.

### Example CXI

### Preparation of Bioconjugates of General Formula CVI Detection of a PBA-Labeled Oligonucleotide Hybrid

### Detection of a PBA-Labeled Oligonucleotide Hybridized to a Biotin-Labeled Oligonucleotide.

A 42-mer oligonucleotide was hybridized with two 21-mer oligonucleotides bearing 5'-PBA and Biotin labels in 1.5 M NaCl, 150 mM sodium citrate, pH 7, at 45 C for ten minutes. Twenty-five microliters of the hybridization mixture was mixed with 1-50 µL of SHA-magnetic particles (Dynal, M450) in a polypropylene microtiter plate well. After 30 minutes, the magnetic particles were captured in the bottom of the well with a magnetic plate, and washed five times with 150 mM NaCI, 20 mM Tris-HCl, 0.02% Tween 20, pH 8.

One hundred microliters of SHA-AP (1 ug/mL in 1 mg/mL BSA, 140 mM NaCI, 10 mM Tris-HCl, pH 8) were added to the magnetic particles and mixed well. After 30 minutes, the magnetic particles were captured in the bottom of the well with a magnetic plate, and washed six times with 150 mM NaCl, 20 mM Tris-HCl, 0.02% Tween 20, pH 8. The particles were mixed with alkaline phosphatse substrate (1 mg/mL *p*-nitrophenyl phosphate in 1 M diethanolamine buffer, 1 mM MgCl₂, 0.1 mM ZnCl₂, pH 10.4) and incubated at 37° C for 90 minutes. The A₄₀₅ was measured with a ELISA plate reader (Molecular Devices). As little as 45 pg of oligonucleotide 42-mer was detected. Experimental controls lacking either the 42-mer, or the PBA or Biotin labeled oligonucleotides did not produce a significant A₄₀₅.

### Example CXII

### Preparation of Bioconjugates of General Formula CVI

### Immobilization of a PBA-α-Biotin Conjugate on SHA-Sepharose 4B.

One mg of PBA-α-Biotin, diluted to 1 mL with Tris buffered saline, was applied to small column of SHA-Sepharose 4B (1.0 x 2.0 cm), and washed extensively with Tris buffered saline. The size of the A₂₈₀ peak of the material not binding to the column indicated that almost all of the PBA-conjugate was immobilized on the column.

Biotin binding activity of the column was assayed by applying to the column 5 mL of 1 ug/mL biotinylated alkaline phosphatase in Tris buffered saline containing 5 mg/mL bovine serum albumin (BSA). A sample of the peak of the material flowing through the column was collected for comparison of the enzymatic activity with a sample of the alkaline phosphatase dilution applied to column. After applying the sample, the column was washed with 20 mL of buffer. After washing, a very small sample of column material (25 µL liquid containing about 1 µL gel) was collected to measure the enzymatic activity bound to the gel as a result of capture by the immobilized α-biotin antibody.

The alkaline phosphatase activity was measured by incubating 25 uL of the enzyme samples in 250 uL of 1 mg/mL p-nitrophenylphosphate in 1 M diethanolamine buffer, 1 mM MgCl₂, and 0.1 mM ZnCl₂, pH 10.4, for 20 minutes and then adding 650 uL of 0.1 M NaHCO₃, 10 mM EDTA. Relative to a buffer blank, the A₄₀₅ of the sample applied to the column was 1.57, while the A₄₀₅ of the peak of the material not retained by the column was only 0.042, indicating that virtually all the enzyme conjugate was captured by the column. The small amount of gel assayed produced an A₄₀₅ of 1.30, demonstrating that the enzyme was in fact captured by the column.

### Example CXIII

### Preparation of Bioconjugates of General Formula CVI

### Immobilization of a PBA-Alkaline Phosphatase Conjugate on SHA-Magnetic Beads.

PBA-conjugated alkaline phosphatase was diluted to 5 ug/mL in Tris buffered saline containing 5 mg/mL bovine serum albumin. Two hundred microliters of diluted PBA-conjugated enzyme were mixed with 5, 10, or 20 uL of SHA-magnetic beads (Dynal, M280). The enzyme was also mixed with 40 uL of unmodified beads as a control. The beads were mixed gently for 10 minutes on ice, after which the beads were captured with a rare earth magnet and washed 4 times with Tris buffered saline. The beads were then suspended in 250 uL of 1 mg/mL *p*-nitrophenylphosphate in 1 M diethanolamine buffer, 1 mM MgCl₂, and 0.1 mM ZnCl₂, pH 10.4, and mixed occasionally at 37° C for 10 minutes. The reactions were terminated with 750 uL of Tris buffered saline, 5 mM EDTA. The A₄₀₅ relative to a buffer blank was measured to determine the alkaline phosphatase activity bound to the magnetic beads. The control beads produced an A₄₀₅ of only 0.15, while the SHA-magnetic beads produced an A₄₀₅ of 0.62, 0.97, and 1.33 for 5, 10, and 20 uL of beads, respectively, indicating the immobilization of significant amounts of PBA-AP conjugate on the surface of the beads.

## Claims

1. A reagent having the general formula of General Formula CI: 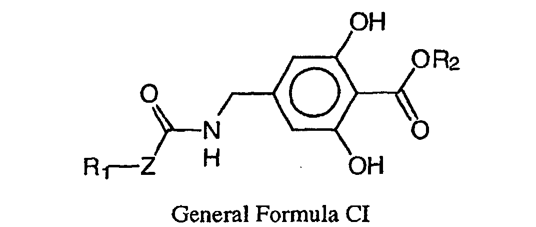 wherein group Z comprises a spacer selected from a saturated or unsaturated up to 6 carbon equivalents in length, an unbranched saturated or unsaturated chain of from about 6 to 18 carbon equivalents in length with at least one of intermediate amide and disulfide moieties, and a polyethylene glycol chain of from about 3 to 12 carbon equivalents in length;
wherein group R₁ is a electrophilic or nucleophilic moiety suitable for reaction of the reagent with a bioactive species selected from the group consisting of acrylamide, amino, bromo, dithiopyridyl, bromoacetamide, hydrazide, N-hydroxysuccinimide ester, N-hydroxysulfoxuccinimide ester, imidate ester, imidazolide, iodo, iodoacetamide, maleimide, and thiol moieties; and
wherein group R₂ is one of an alkyl and a methylene group bearing a moiety with a negative dipole moment.

2. The reagent of claim 1, wherein group R₂ is preferably selected from one of CH₃, CH₂CH₃, CH₂CN, CH₂COOH, CH₂CONH₂, and CH₂OCH₃.

3. The reagent of claim 1, wherein group Z is preferably an unbranched alkyl chain of the general formula (CH₂)ₙ, wherein n = 1 to 6.

4. A reagent of claim 1 having the formula: 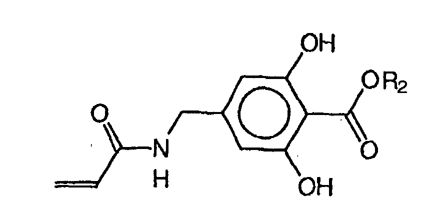 wherein group R₂ is one of an alkyl and a methylene group bearing a moiety with a negative dipole moment.

5. A conjugate of the reagent of claim 1, wherein R₁ is a biologically active species (*BAS*), said conjugate having the general formula of General Formula CII:

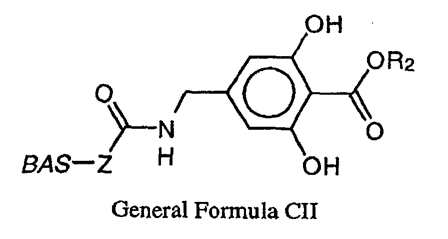

6. The conjugate of claim 5, wherein group R₂ is preferably selected from one of CH₃, CH₂CH₃, CH₂CN, CH₂COOH, CH₂CONH₂, and CH₂OCH₃.

7. The conjugate of claim 5, wherein group Z is preferably an unbranched alkyl chain of general formula (CH₂)ₙ, wherein n = 1 to 6.

8. The conjugate of claim 5, having the general formula: 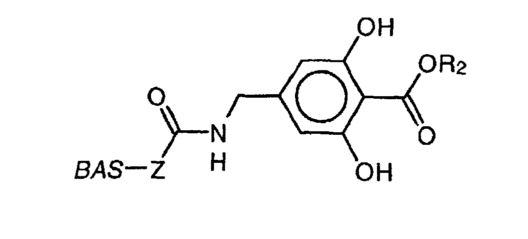 wherein group R₂ is CH₃.

9. A compound comprising: 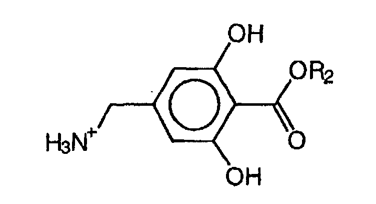 wherein R₂ is one of an alkyl and a methylene group bearing a moiety with a negative dipole moment.

10. The compound of claim 9, wherein group R₂ is preferably selected from one of CH₃, CH₂CH₃, CH₂CN, CH₂COOH, CH₂CONH₂, and CH₂OCH₃.

11. A method for preparing a reagent according to claim 9, the method comprising:
condensing methyl 2,6-dihydroxy-4-methylbenzoate with a suitable protecting groups Q to modify the reactivity of the ring system: 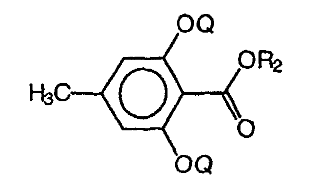 wherein R₂ is an alkyl,
halogenating the alkyl benzoate ester to yield a benzyl halide wherein group Y is a halogen: 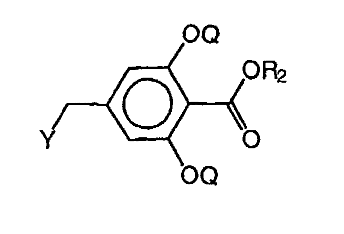
alkylating the benzyl halide with an azide salt to yield a benzyl azide: 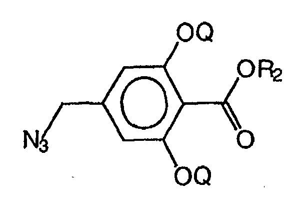
and hydrogenating the benzyl azide to yield the corresponding benzyl amine and remove the protective groups Q:

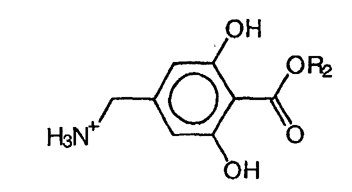

12. The method of claim 11, wherein the protective group Q is an acetyl group.

13. The method of claim 11, further comprising:
condensing the 2,6-dihydroxybenzylamine with one of an activated carboxylic acid to yield the reagent of general formula CI according to claim 1:

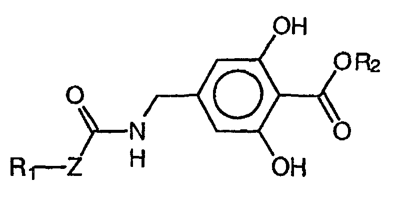

14. The method of claim 13, further comprising the step of condensing the reagent with a bioactive species to yield the reagent of general formula CII: 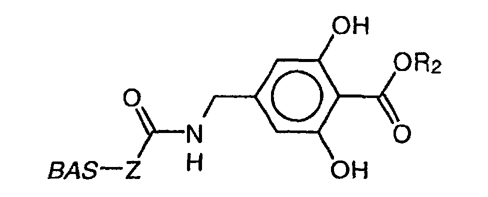 wherein *BAS* is a biologically active species.

15. A method for preparing the reagent of General Formula CI: 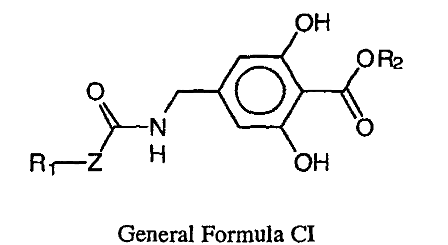 wherein group Z comprises a spacer selected from a saturated or unsaturated up to 6 carbon equivalents in length, an unbranched saturated or unsaturated chain of from about 6 to 18 carbon equivalents in length with one of intermediate amide and disulfide moieties, and a polyethylene glycol chain of from about 3 to 12 carbon equivalents in length;
wherein group R₁ is a electrophilic or nucleophilic moiety suitable for reaction of the reagent with a bioactive species selected from the group consisting of acrylamide, amino, bromo dithiopyridyl, bromoacetamide, hydrazide, N-hydroxysuccinimide ester, N-hydroxysulfosuccinimide ester, imidate ester, imidazolide, iodo, iodoacetamide, maleimide, and thiol moieties; and
wherein group R₂ is one of an alkyl and a methylene group bearing a moiety with a negative dipole moment,the method comprising:
condensing a 2,6-dihydroxy-4-methylbenzoate with a suitable protective group Q to modify the reactivity of the ring system: 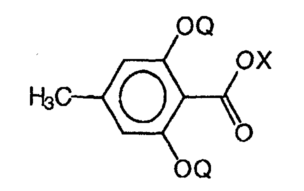 wherein group X is an alkyl group of from about 1 to 3 carbons,
halogenating the 2,6-dihydroxy-4-methylbenzoate to yield a benzyl halide wherein group Y is a halogen: 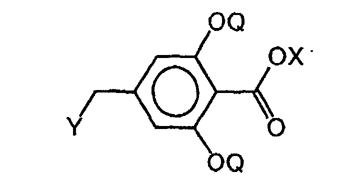
alkylating the benzyl halide with an azide salt to yield a benzyl azide: 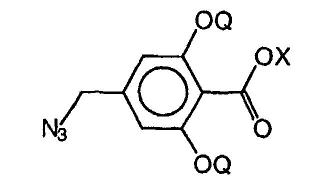
hydrogenating the benzyl azide and removing the protecting groups Q to yield a benzyl amine: 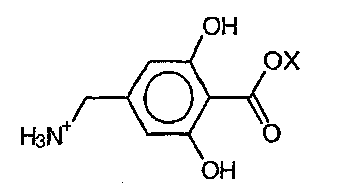
condensing the benzyl amine with a suitable protective group Q' to protect the amine moiety from participation in subsequent reactions, to yield an alkyl aminomethylbenzoate: 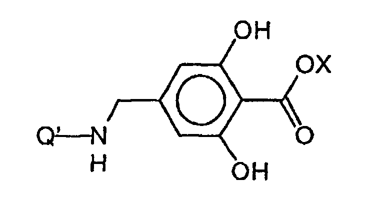
hydrolyzing the alkyl ester and to yield a benzoic acid: 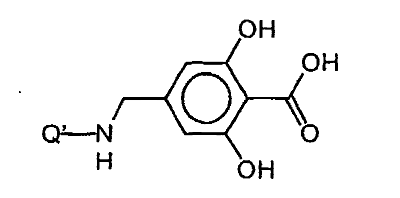
alkylating the benzoic acid to yield an ester: 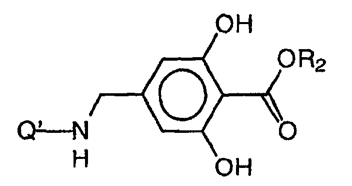 wherein group R₂ is selected from one of an alkyl and a methylene group bearing a moiety with a negative dipole moment,
removing the protective group Q' to yield a benzyl amine: 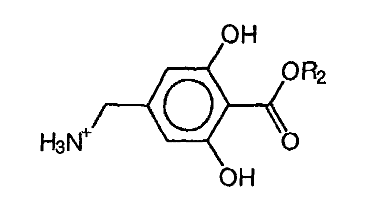
condensing the benzylamine with one of an activated carboxylic acid to yield the reagent.

16. The method of claim 15, wherein group R₂ is selected from one of CH₃, CH₂CH₃, CH₂CN, CH₂COOH, CH₂CONH₂, and CH₂OCH₃.

17. The method of claim 15, wherein the protective group Q is an acetyl group.

18. The method of claim 15, wherein the protective group Q' is a N-(tert-butoxycarbonyl) group.

19. The method of claim 15 comprising the step of condensing the reagent of General Formula CI as in claim 1 with a bioactive species to yield the reagent of General Formula CII: 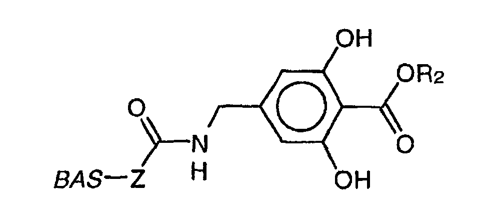 wherein *BAS* is a biologically active species.

20. A reagent having the general formula of General Formula CIII: 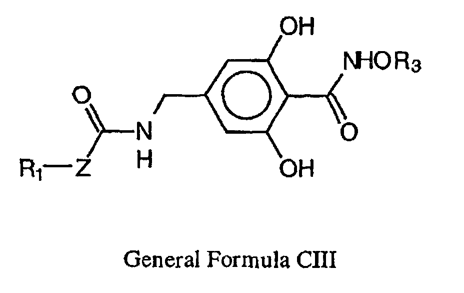 wherein group Z comprises a spacer selected from a saturated or unsaturated up to 6 carbon equivalents in length, an unbranched saturated or unsaturated chain of from about 6 to 18 carbon equivalents in length with at least one of intermediate amide and disulfide moieties, and a polyethylene glycol chain of from about 3 to 12 carbon equivalents in length;
wherein group R₃ is selected from one of an H, an alkyl, and a methylene or ethylene moiety bearing a substituent with a negative dipole moment; and
wherein group R₁ is a electrophilic or nucleophilic moiety suitable for reaction of the reagent with a bioactive species selected from the group consisting of acrylamide, amino, bromo, dithiopyridyl, bromoacetamide, hydrazide, N-hydroxysuccinimide ester, N-hydroxysulfosuccinimide ester, imidate ester, imidazolide, iodo, iodoacetamide, maleimide, and thiol moieties.

21. The reagent of claim 20, wherein group R₃ is preferably selected from one of H, CH₃, CH₂CH₃, CH₂CN, CH₂COOH, CH₂CONH₂, CH₂CH₂OH, and CH₂OCH₃.

22. The reagent of claim 20, wherein group Z is preferably an unbranched alkyl chain of the general formula (CH₂)ₙ, wherein n = 1 to 6.

23. The reagent of claim 20, wherein R1 is an ethylene moiety, having the general formula:

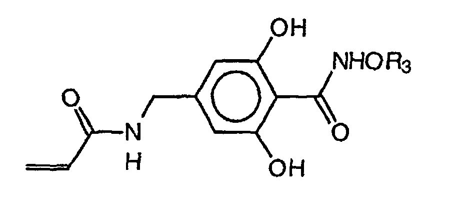

24. A conjugate of a reagent of claim 20, said conjugate having the general formula of General Formula CIV: 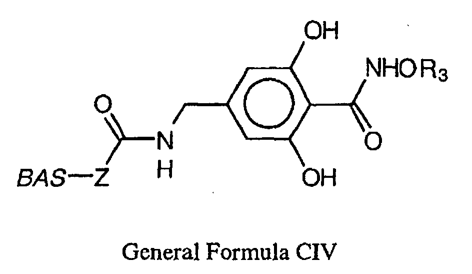 wherein *BAS* is a biologically active species.

25. The conjugate of claim 24, wherein group R₃ is preferably selected from one of H, CH₃, CH₂CH₃, CH₂CN, CH₂COOH, CH₂CONH₂, CH₂CH₂OH, and CH₂OCH₃.

26. The conjugate of claim 24, wherein group Z is preferably an unbranched alkyl chain of general formula (CH₂)ₙ, wherein n = 1 to 6.

27. The conjugate of claim 24, having the general formula: 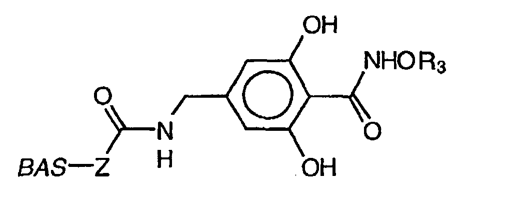 wherein group R3 is H.

28. A bioconjugate of the conjugate of claim 24 having the general formula of General Formula CVI: 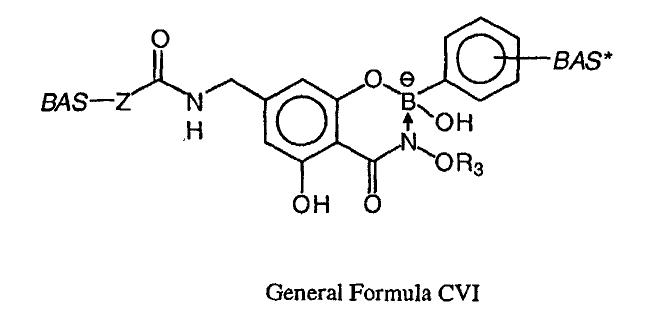 wherein *BAS** is a biologically active species.

29. The bioconjugate of claim 28, wherein group R₃ is preferably selected from one of H, CH₃, CH₂CH₃, CH₂CN, CH₂COOH, CH₂CONH₂, CH₂CH₂OH, and CH₂OCH₃.

30. The bioconjugate of claim 28, wherein group Z is preferably an unbranched alkyl chain of general formula (CH₂)ₙ, wherein n = 1 to 6.

31. The bioconjugate of claim 28, wherein *BAS* and *BAS** are different bioactive species.

32. A method of preparing the reagent of General Formula CIV according to claim 24 comprising the step of condensing the reagent of General Formula CIII according to claim 20 with a bioactive species to yield the reagent of General Formula CIV:

33. The method of claim 32, wherein group R₃ is preferably selected from one of H, CH₃, CH₂CH₃, CH₂CN, CH₂COOH, CH₂CONH₂, CH₂CH₂OH, and CH₂OCH₃.

34. The method of claim 32, wherein group Z is preferably an unbranched alkyl chain of general formula (CH₂)ₙ, wherein n = 1 to 6.

35. A method of preparing the bioconjugate of General Formula CVI according to claim 28.
the method comprising the step of complexing the conjugate of claim 6 with a second conjugate comprising a second biologically active species and a phenylboronic acid.

36. The method of claim 35, wherein group R₃ is preferably selected from one of H, CH₃, CH₂CH₃, CH₂CN, CH₂COOH, CH₂CONH₂, CH₂CH₂OH, and CH₂OCH₃.

37. The method of claim 35, wherein group Z is preferably an unbranched alkyl chain of general formula (CH₂)ₙ, wherein n = 1-6.

38. The method of claim 35, wherein *BAS* and *BAS** are different bioactive species.

39. A method for preparing a conjugate according to claim 24 having the general formula of General Formula CIV:
the method comprising:
condensing a reagent of general formula 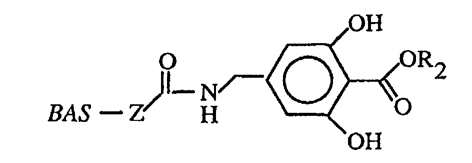 with a hydroxlamine derivative of the general formula NH₂OR₃, wherein group R₂ is selected from one of an alkyl, and a methylene group bearing a moiety with a negative dipole moment.

40. The method of claim 39, wherein group R₃ is preferably selected from one of H, CH₃, CH₂CH₃, CH₂CN, CH₂COOH, CH₂CONH₂, CH₂CH₂OH, and CH₂OCH₃.

41. The method of claim 39, wherein group Z is preferably an unbranched alkyl chain of general formula (CH₂)ₙ, wherein n = 1 to 6.

42. The compound comprising: 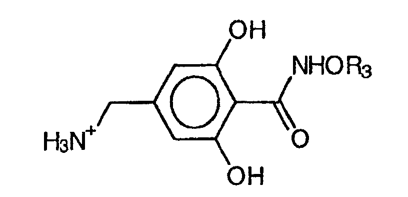 wherein group R₃ is selected from one of H, an alkyl, and an alkyl or methylene group bearing a moiety with a negative dipole moment.

43. A method of making the compound of claim 42,
the method comprising:
condensing a methyl 2,6-dihydroxy-4-methylbenzoate of general formula: 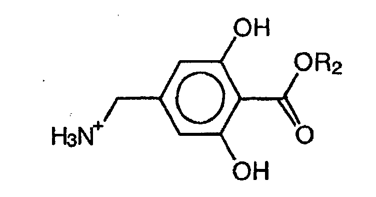 wherein R₂ is an alkyl, with a suitable group Q to protect the amine moiety: 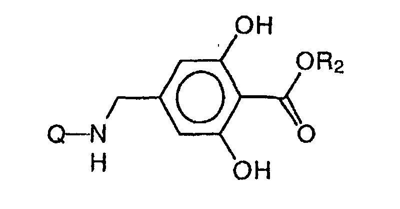
condensing the benzoate ester with suitable groups Q' to reduce the reactivity of the ring system: 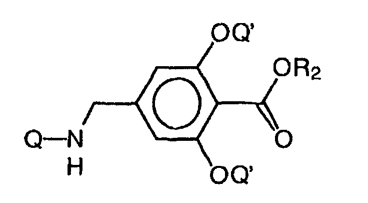
hydrolyzing the benzoate ester to yield the salicylic acid: 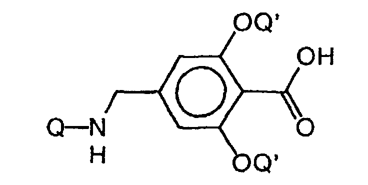
condensing the acid with a hydroxylamine derivative of the general formula NH₂OR_{3,} wherein R₃ is selected from one of an H, an alkyl, and a methylene or ethylene group bearing a moiety with a negative dipole moment: 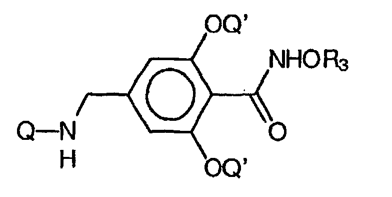
and removing the protecting groups Q and Q'.

## Patentansprüche

1. Reagenz der allgemeinen Formel CI 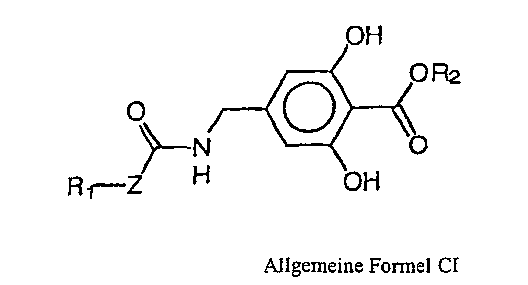 worin die Gruppe Z einen Spacer umfaßt, der aus einer gesättigten oder ungesättigten bis zu 6 Kohlenstoffäquivalenten langen, einer unverzweigten gesättigten oder ungesättigten Kette mit einer Länge von etwa 6 bis 18 Kohlenstoffäquivalenten mit mindestens einem Zwischenproduktamid und Disulfidresten und einer Polyethylenglycolkette mit einer Länge von etwa 3 bis 12 Kohlenstoffatomäquivalenten ausgewählt ist,
worin die Gruppe R₁ ein elektrophiler oder nukleophiler Rest ist, der zur Umsetzung des Reagenzes mit einem bioaktiven Rest geeignet ist. ausgewählt aus der Gruppe, die besteht aus Resten, wie Acrylamid, Amino, Brom, Dithiopyridyl, Bromacetamid, Hydrazid, N-Hydrovysuccinimidester, N-Hydroxysulfosuccinimidester. Imidatester, Imidazolid. Iod. Iodacetamid, Maleimid und Thiol, und
worin die Gruppe R₂ entweder für eine Alkyl- oder eine Methylengruppe steht, die einen Rest mit einem negativen Dipolmoment trägt.

2. Reagenz nach Anspruch 1, worin die Gruppe R₂ vorzugsweise ausgewählt ist aus CH₃, CH₂CH₃, CH₂CN, CH₂COOH, CH₂CONH₂ und CH₂OCH₃.

3. Reagenz nach Anspruch 1, worin die Gruppe Z vorzugsweise eine unverzweigte Alkylkette der allgemeinen Formel (CH₂)ₙ ist. worin n fiir 1 bis 6 steht.

4. Reagenz nach Anspruch 1 der Formel 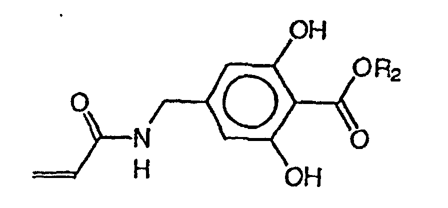 worin die Gruppe R₂ entweder für eine Alkyl- oder eine Methylengruppe steht, die einen Rest mit einem negativen Dipolmoment trägt.

5. Konjugat des Reagenzes nach Anspruch 1. worin R₁ ein biologisch aktiver Rest (BAS) ist, wobei das Konjugat die allgemeine Formel CII aufweist

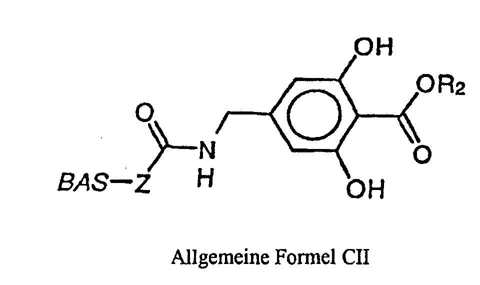

6. Konjugat nach Anspruch 5, worin die Gruppe R₂ vorzugsweise ausgewählt ist aus CH₃, CH₂CH₃, CH₂CN, CH₂COOH, CH₂CONH₂ und CH₂OCH₃.

7. Konjugat nach Anspruch 5, worin die Gruppe Z vorzugsweise eine unverzweigte Alkylkette der allgemeinen Formel (CH₂)ₙ ist, worin n für 1 bis 6 steht.

8. Konjugat nach Anspruch 5 der allgemeinen Formel 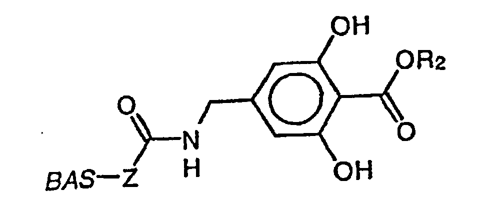 worin die Gruppe R₂ für CH₃ steht.

9. Verbindung, die umfaßt 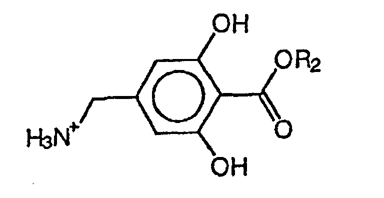 worin R₂ entweder für eine Alkyl- oder eine Methylengruppe steht, die einen Rest mit einem negativen Dipolmoment trägt.

10. Verbindung nach Anspruch 9. worin die Gruppe R₂ vorzugsweise ausgewählt ist aus CH₃, CH₂CH₃, CH₂CN, CH₂COOH, CH₂CONH₂ und CH₂OCH₃.

11. Verfahren zur Herstellung eines Reagenzes nach Anspruch 9, wobei das Verfahren **gekennzeichnet ist durch** Kondensation von Methyl-2,6-dihydroxy-4-methylbenzoat mit einer geeigneten Schutzgruppe Q unter Modifizierung der Reaktivität des Ringsystems 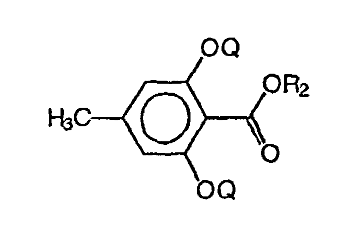 worin R₂ für ein Alkyl steht,
Halogenierung des Alkylbenzoatesters unter Bildung eines Benzylhalogenids, worin die Gruppe Y für ein Halogen steht 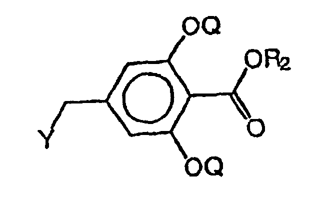 Alkyliening des Benzylhalogenids mit einem Azidsalz unter Bildung eines Benzylazids 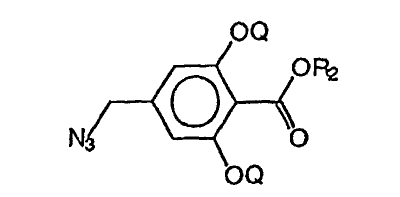 und
Hydrierung des Benzylazids unter Bildung des entsprechenden Benzylamins und Entfernung der Schutzgruppen Q

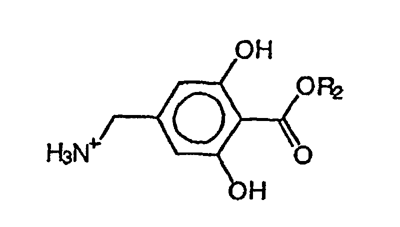

12. Verfahren nach Anspruch 11. worin die Schutzgruppe Q eine Acetylgruppe ist.

13. Verfahren nach Anspruch 11, ferner **gekennzeichnet durch**
Kondensation des 2.6-Dihydroxybenzylamins mit einer aktivierten Carbonsäure unter Bildung des Reagenzes der allgemeinen Formel CI nach Anspruch 1

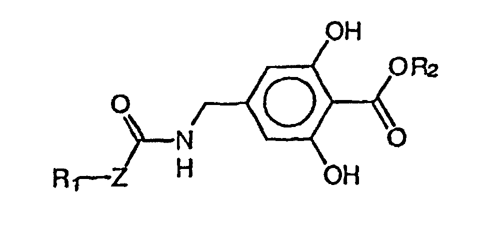

14. Verfahren nach Anspruch 13, ferner **gekennzeichnet durch** den Kondensationsschritt des Reagenzes mit einem bioaktiven Rest unter Bildung des Reagenzes der allgemeinen Formel CII 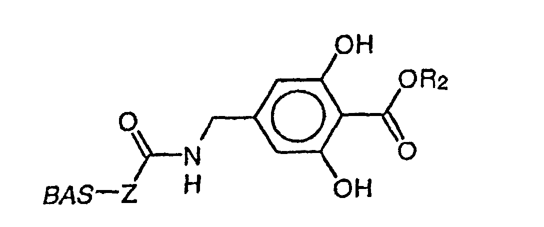 worin BAS für einen biologisch aktiven Rest steht.

15. Verfahren zur Herstellung des Reagenzes der allgemeinen Formel CI 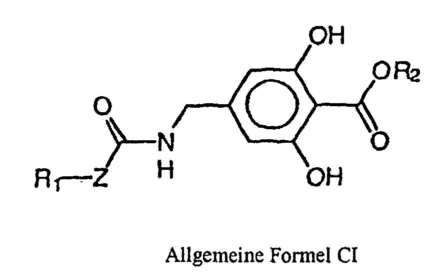 worin die Gruppe Z einen Spacer umfaßt, der aus einer gesättigten oder ungesättigten bis zu 6 Kohlenstoffäquivalenten langen, einer unverzweigten gesättigten oder ungesättigten Kette mit einer Länge von etwa 6 bis 18 Kohlenstoffäquivalenten mit einem Zwischenproduktamid und Disulfidresten und einer Polyethylenglycolkette mit einer Länge von etwa 3 bis 12 Kohlenstoffatomäquivalenten ausgewählt ist,
worin die Gruppe R₁ ein elektrophiler oder nukleophiler Rest ist, der zur Umsetzung des Reagenzes mit einem bioaktiven Rest geeignet ist, ausgewählt aus der Gruppe. die besteht aus Resten, wie Acrylamid. Amino, Brom, Dithiopyridyl, Bromacetamid. Hydrazid. N-Hydroxysuccinimidester. N-Hydroxysulfosuccinimidester. Imidatester. Imidazolid, Iod, Iodacetamid, Maleimid und Thiol, und
worin die Gruppe R₂ entweder für eine Alkyl- oder eine Methylengruppe steht, die einen Rest mit einem negativen Dipolmoment trägt, wobei das Verfahren **gekennzeichnet ist durch**
Kondensation von 2,6-Dihydroxy-4-methylbenzoat mit einer geeigneten Schutzgruppe Q unter Modifizierung der Reaktivität des Ringsystems 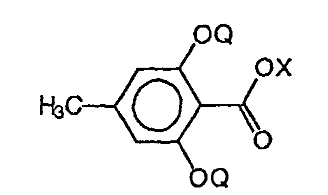 worin die Gruppe X für eine Alkylgruppe mit 1 bis 3 Kohlenstoffen steht.
Halogenierung des 2,6-Dihydroxy-4-methylbenzoats unter Bildung eines Benzylhalogenids. worin die Gruppe Y fiir ein Halogen steht 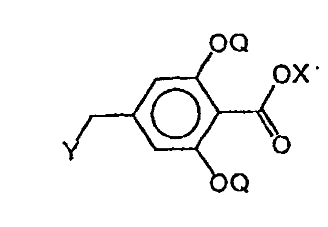 Alkylierung des Benzylhalogenids mit einem Azidsalz unter Bildung eines Benzylazids 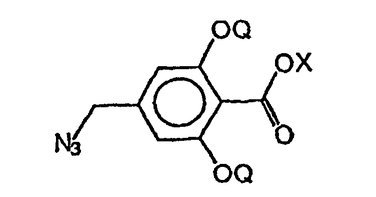 Hydrierung des Benzylazids und Entfernung der Schutzgruppen Q unter Bildung eines Benzylamins 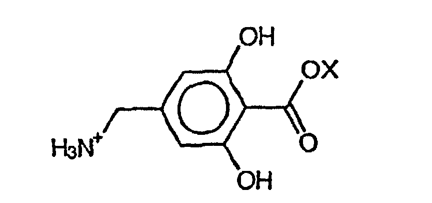 Kondensation des Benzylamins mit einer geeigneten Schutzgruppe Q' zum Schutz des Aminrests vor der Teilnahme in anschließenden Umsetzungen unter Bildung eines Alkylaminomethylbenzoats 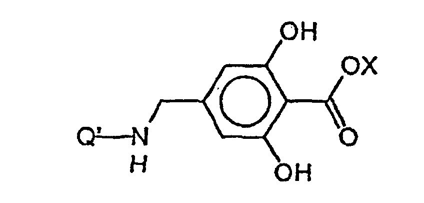 Hydrolyse des Alkylesters unter Bildung einer Benzoesäure 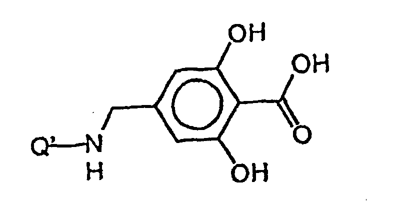 Alkylierung der Benzoesäure unter Bildung eines Esters 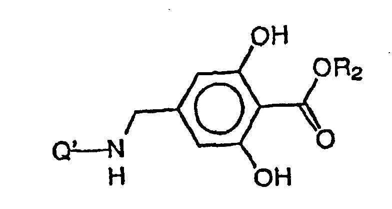 worin die Gruppe R₂ ausgewählt ist aus einem Alkyl- und einer Methylengruppe, die einen Rest mit einem negativen Dipolmoment trägt.
Entfernung der Schutzgruppe Q' unter Bildung des Benzylamins 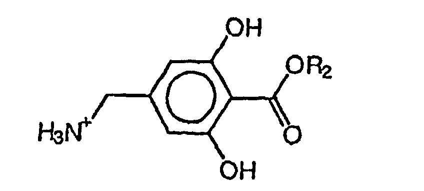 Kondensation des Benzylamins mit einer aktivierten Carbonsäure unter Bildung des Reagenzes.

16. Verfahren nach Anspruch 15. worin die Gruppe R₂ ausgewählt ist aus CH₃, CH₂CH₃, CH₂CN, CH₂COOH, CH₂CONH₂ und CH₂OCH₃.

17. Verfahren nach Anspruch 15, worin die Schutzgruppe Q eine Acetylgruppe ist.

18. Verfaluen nach Anspruch 15, worin die Schutzgruppe Q' eine N-(tert-Butoxycarbonyl)gruppe ist.

19. Verfahren nach Anspruch 15, **gekennzeichnet durch** die Kondensation des Reagenzes der allgemeinen Formel CI nach Anspruch 1 mit einem bioaktiven Rest unter Bildung des Reagenzes der allgemeinen Formel CII 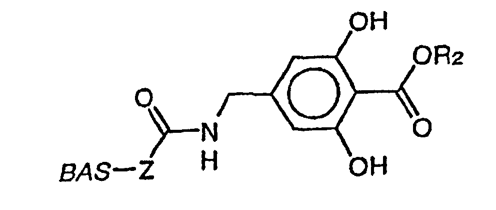 worin BAS ein biologisch aktiver Rest ist.

20. Reagenz der allgemeinen Formel CIII 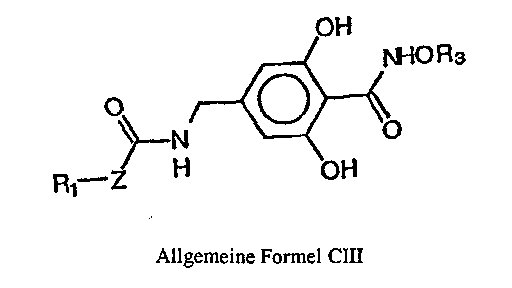 worin die Gruppe Z einen Spacer umfaßt, der aus einer gesättigten oder ungesättigten bis zu 6 Kohlenstoffäquivalenten langen, einer unverzweigten gesättigten oder ungesättigten Kette mit einer Länge von etwa 6 bis 18 Kohlenstoffäquivalenten mit mindestens einem Zwischenproduktamid und Disulfidresten und einer Polyethylenglycolkette mit einer Länge von etwa 3 bis 12 Kohlenstoffatomäquivalenten ausgewählt ist,
worin die Gruppe R₃ ausgewählt ist aus H, einem Alkyl und einem Methylen- oder Ethylenrest, der einen Substituenten mit einem negativen Dipolmoment trägt, und
worin die Gruppe R₁ ein elektrophiler oder nukleophiler Rest ist, der zur Umsetzung des Reagenzes mit einem bioaktiven Rest geeignet ist, ausgewählt aus der Gruppe, die besteht aus Resten, wie Acrylamid, Amino, Brom. Dithiopyridyl. Bromacetamid, Hydrazid, N-Hydroxysuccinimidester, N-Hydroxysulfosuccinimidester, Imidatester. Imidazolid, Iod. Iodacetamid, Maleimid und Thiol.

21. Reagenz nach Anspruch 20. worin die Gruppe R₃ ausgewählt ist aus H, CH₃, CH₂CH₃, CH₂CN, CH₂COOH, CH₂CONH₂, CH₂CH₂OH und CH₂OCH₃.

22. Reagenz nach Anspruch 20, worin die Gruppe Z vorzugsweise für eine unverzweigte Alkylkette der allgemeinen Formel (CH₂)ₙ steht, worin n fiir 1 bis 6 steht.

23. Reagenz nach Anspruch 20, worin R₁ für einen Ethylenrest mit der folgenden allgemeinen Formel steht

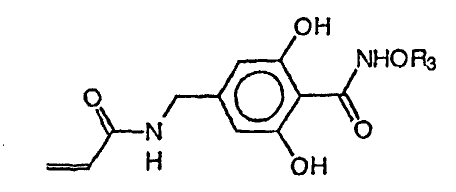

24. Konjugat eines Reagenzes nach Anspruch 20, worin das Konjugat die allgemeine Formel CIV aufweist 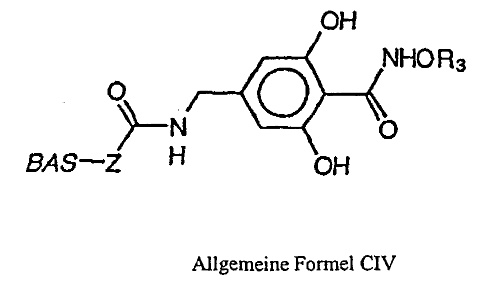 worin BAS für einen biologisch aktiven Rest steht.

25. Konjugat nach Anspruch 24, worin die Gruppe R₃ vorzugsweise ausgewählt ist aus H, CH₃, CH₂CH₃, CH₂CN, CH₂COOH, CH₂CONH₂ , CH₂CH₂OH und CH₂OCH₃.

26. Konjugat nach Anspruch 24, worin die Gruppe Z vorzugsweise für eine unverzweigte Alkylkette der allgemeinen Formel (CH₂)ₙ steht, worin n für 1 bis 6 steht.

27. Konjugat nach Anspruch 24 der allgemeinen Formel 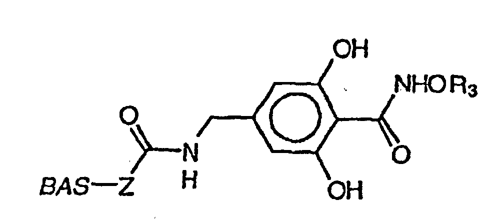 worin die Gruppe R₃ für H steht.

28. Biokonjugat des Konjugats nach Anspruch 24 der allgemeinen Formel CVI 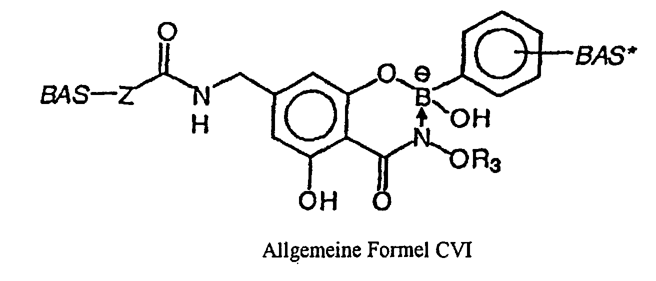 worin BAS* für einen biologisch aktiven Rest steht.

29. Biokonjugat nach Anspruch 28, worin die Gruppe R₃ vorzugsweise ausgewählt ist aus H, CH₃, CH₂CH₃, CH₂CN, CH₂COOH, CH₂CONH₂, CH₂CH₂OH und CH₂OCH₃.

30. Biokonjugat nach Anspruch 28, worin die Gruppe Z vorzugsweise für eine unverzweigte Alkylkette der allgemeinen Formel (CH₂)ₙ steht, worin n für 1 bis 6 steht.

31. Biokonjugat nach Anspruch 28. worin BAS und BAS* unterschiedliche bioaktive Reste sind.

32. Verfahren zur Herstellung des Reagenzes der allgemeinen Formel CIV nach Anspruch 24, **gekennzeichnet durch** die Kondensation des Reagenzes der allgemeinen Formel CIII nach Anspruch 20 mit einer bioaktiven Gruppe unter Bildung des Reagenzes der allgemeinen Formel CIV.

33. Verfahren nach Anspruch 32, worin die Gruppe R₃ vorzugsweise ausgewählt ist aus H, CH₃, CH₂CH₃, CH₂CN, CH₂COOH, CH₂CONH₂, CH₂CH₂OH und CH₂OCH₃.

34. Verfahren nach Anspruch 32, worin die Gruppe Z vorzugsweise für eine unverzweigte Alkylkette der allgemeinen Formel (CH₂)ₙ steht, worin n für 1 bis 6 steht.

35. Verfahren zur Herstellung des Biokonjugats der allgemeinen Formel CVI nach Anspruch 28, wobei das Verfahren die Komplexierung des Konjugats nach Anspruch 6 mit einem zweiten Konjugat umfaßt, das eine zweite biologisch aktive Gruppe und eine Phenylborsäure umfaßt.

36. Verfahren nach Anspruch 35, worin die Gruppe R₃ vorzugsweise ausgewählt ist aus H, CH₃, CH₂CH₃, CH₂CN, CH₂COOH, CH₂CONH₂, CH₂CH₂OH und CH₂OCH₃.

37. Verfahren nach Anspruch 35, worin die Gruppe Z vorzugsweise für eine unverzweigte Alkylkette der allgemeinen Formel (CH₂)ₙ steht, worin n für 1 bis 6 steht.

38. Verfahren nach Anspruch 35, worin BAS und BAS* unterschiedliche bioaktive Reste sind.

39. Verfahren zur Herstellung eines Konjugats nach Anspruch 24 mit der allgemeinen Formel CIV, wobei das Verfahren **gekennzeichnet ist durch**
Kondensation eines Reagenzes der allgemeinen Formel 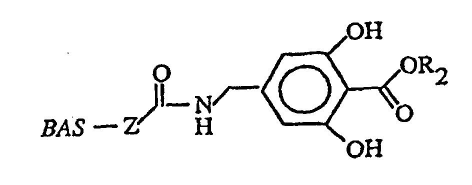 mit einem Hydroxylaminderivat der allgemeinen Formel NH₂OR₃, worin die Gruppe R₂ ausgewählt ist aus einem Alkyl und einer Methylengruppe, die einen Rest mit einem negativen Dipolmoment trägt.

40. Verfahren nach Anspruch 39, worin die Gruppe R₃ vorzugsweise ausgewählt ist aus H, CH₃, CH₂CH₃, CH₂CN, CH₂COOH, CH₂CONH₂, CH₂CH₂OH und CH₂OCH₃.

41. Verfahren nach Anspruch 39, worin die Gruppe Z vorzugsweise fiir eine unverzweigte Alkylkette der allgemeinen Formel (CH₂)ₙ steht, worin n für 1 bis 6 steht.

42. Verbindung, die umfaßt 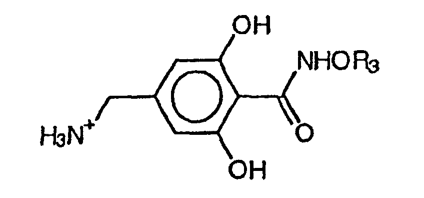 worin R₃ ausgewählt ist aus H, einem Alkyl und einer Alkyl- oder Methylengruppe, die einen Rest mit einem negativen Dipolmoment trägt.

43. Verfahren zur Herstellung der Verbindung nach Anspruch 42, wobei das Verfahren **gekennzeichnet ist durch** Kondensation eines Methyl-2,6-dihydroxy-4-methylbenzoats der allgemeinen Formel 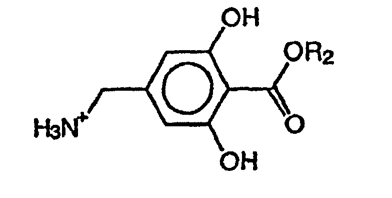 worin R₂ für ein Alkyl steht, mit einer geeigneten Gruppe Q zum Schutz des Aminrests 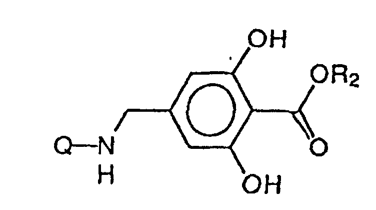 Kondensation des Benzoatesters mit geeigneten Gruppen Q', um die Reaktivität des Ringsystems zu verringern 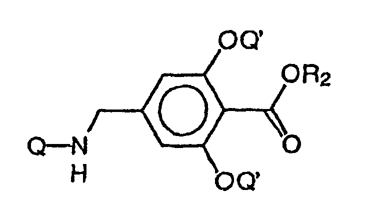 Hydrolyse des Benzoatesters unter Bildung der Salicylsäure 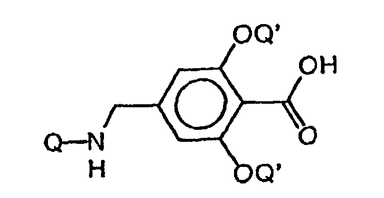 Kondensation der Säure mit einem Hydroxylaminderivat der allgemeinen Formel NH₂OR₃, worin R₃ ausgewählt ist aus H. einem Alkyl und einer Methylen- oder Ethylengruppe, die einen Rest mit einem negativen Dipolmoment trägt 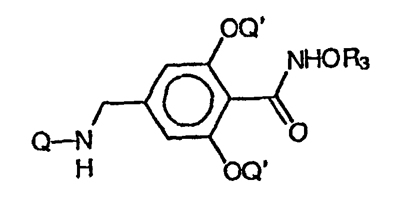 und Entfernung der Schutzgruppen Q und Q'.

## Revendications

1. Réactif répondant à la formule générale de Formule Générale CI : 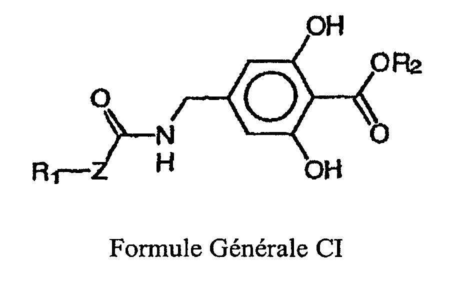 dans laquelle le groupe Z comprend un espaceur choisi entre un espaceur saturé ou insaturé ayant jusqu'à 6 équivalents carbonés en longueur, une chaîne non ramifiée saturée ou insaturée ayant environ 6 à 18 équivalents carbonés en longueur, avec au moins un des groupements amide et disulfure intermédiaires, et une chaîne polyéthylèneglycol d'environ 3 à 12 équivalents carbonés en longueur ;
dans laquelle le groupe R₁ est un groupement électrophile ou nucléophile approprié à une réaction du réactif avec une entité bioactive choisie dans le groupe constitué par des groupements acrylamide, amino, bromo, dithiopyridyle, bromoacétamide, hydrazide, ester de N-hydroxysuccinimide, ester de N-hydroxysulfosuccinimide, ester imidate, imidazolide, iodo, iodoacétamide, maléimide et thiol ; et
dans laquelle le groupe R₂ est un groupe alkyle ou méthylène portant un groupement ayant un moment dipolaire négatif.

2. Réactif selon la revendication 1, dans lequel le groupe R₂ est de préférence choisi entre l'un des groupes suivants : CH₃, CH₂CH₃, CH₂CN, CH₂COOH, CH₂CONH₂ et CH₂OCH₃.

3. Réactif selon la revendication 1, dans lequel le groupe Z est de préférence une chaîne alkyle non ramifiée de formule générale (CH₂)ₙ, dans laquelle n = 1 à 6.

4. Réactif selon la revendication 1, répondant à la formule : 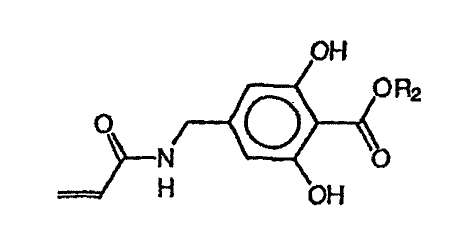 dans laquelle le groupe R₂ est un groupe alkyle ou méthylène portant un groupement ayant un moment dipolaire négatif.

5. Conjugué du réactif selon la revendication 1, dans lequel R₁ est une entité biologiquement active (*BAS*), ledit conjugué répondant à la formule générale de Formule Générale CII :

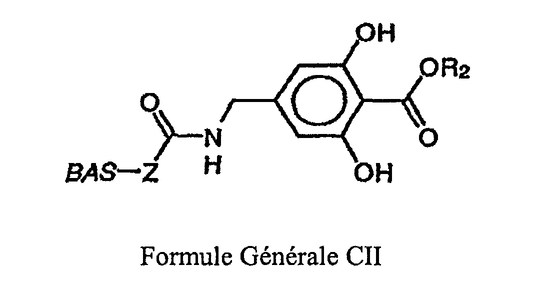

6. Conjugué selon la revendication 5, dans lequel le groupe R₂ est de préférence choisi entre l'un des groupes suivants : CH₃, CH₂CH₃, CH₂CN, CH₂COOH, CH₂CONH₂ et CH₂OCH₃.

7. Conjugué selon la revendication 5, dans lequel le groupe Z est de préférence une chaîne alkyle non ramifiée de formule générale (CH₂)ₙ, dans laquelle n = 1 à 6.

8. Conjugué selon la revendication 5, répondant à la formule générale : 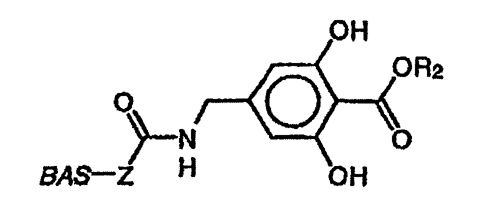 dans laquelle R₂ est un groupe CH₃.

9. Composé comprenant : 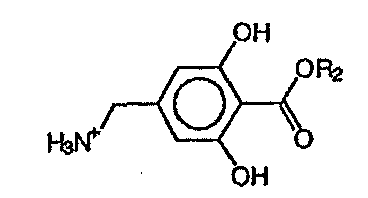 où R₂ représente un groupe alkyle ou méthylène portant un groupement ayant un moment dipolaire négatif.

10. Composé selon la revendication 9, dans lequel le groupe R₂ est de préférence choisi entre l'un des groupes suivants: CH₃, CH₂CH₃, CH₂CN, CH₂COOH, CH₂CONH₂ et CH₂OCH₃.

11. Procédé de préparation d'un réactif selon la revendication 9, le procédé comprenant :
la condensation du 2,6-dihydroxy-4-méthylbenzoate de méthyle avec un groupe protecteur Q approprié pour modifier la réactivité du système cyclique : 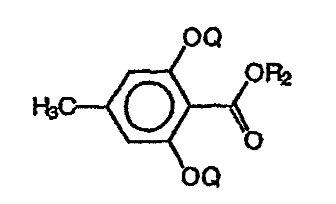 où R₂ représente un groupe alkyle,
l'halogénation de l'ester benzoate d'alkyle pour donner un halogénure de benzyle dans lequel le groupe Y est un halogène : 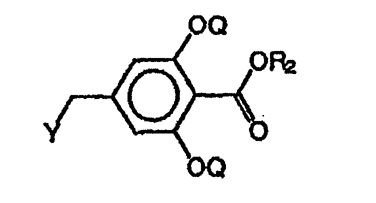
l'alkylation de l'halogénure de benzyle avec un sel consistant en un azoture pour donner un azoture de benzyle : 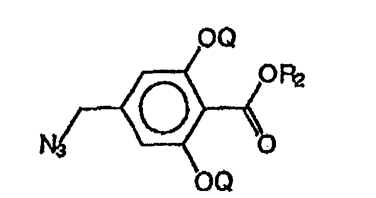
et l'hydrogénation de l'azoture de benzyle pour donner la benzylamine correspondante et pour éliminer les groupes protecteurs Q :

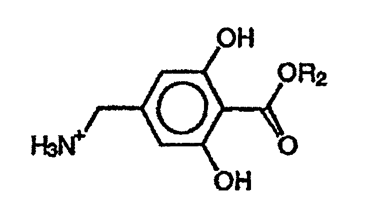

12. Procédé selon la revendication 11, dans lequel le groupe protecteur Q est un groupe acétyle.

13. Procédé selon la revendication 11, comprenant en outre :
la condensation de la 2,6-dihydroxybenzylamine avec un acide carboxylique activé pour donner le réactif de Formule Générale CI selon la revendication 1 :

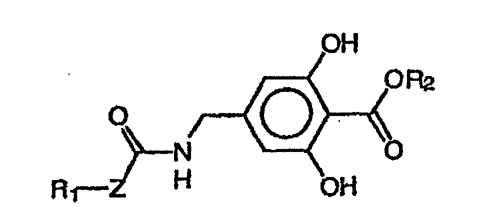

14. Procédé selon la revendication 13, comprenant en outre l'étape de condensation du réactif avec une entité bioactive pour donner le réactif de Formule Générale CII : 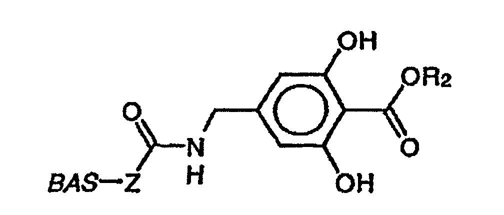 dans laquelle *BAS* est une entité biologiquement active.

15. Procédé de préparation d'un réactif de Formule Générale CI : 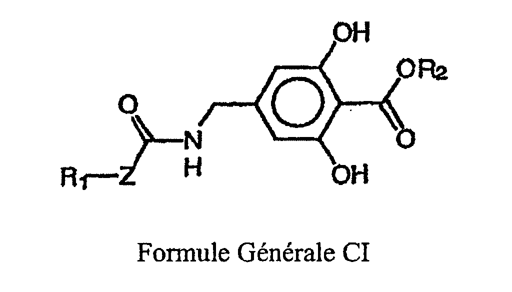 dans laquelle le groupe Z comprend un espaceur choisi entre un espaceur saturé et insaturé ayant jusqu'à 6 équivalents carbonés en longueur, une chaîne non ramifiée saturée ou insaturée ayant environ 6 à 18 équivalents carbonés en longueur avec des groupements amide et disulfure intermédiaires, et une chaîne polyéthylèneglycol d'environ 3 à 12 équivalents carbonés en longueur ;
dans laquelle le groupe R₁ est un groupement électrophile ou nucléophile approprié à une réaction du réactif avec une entité bioactive choisie dans le groupe constitué par des groupements acrylamide, amino, bromo, dithiopyridyle, bromoacétamide, hydrazide, ester de N-hydroxysuccinimide, ester de N-hydroxysulfosuccinimide, ester imidate, imidazolide, iodo, iodoacétamide, maléimide et thiol ; et
dans laquelle le groupe R₂ est un groupe alkyle ou méthylène portant un groupement ayant un moment dipolaire négatif, le procédé comprenant :
la condensation du 2,6-dihydroxy-4-méthylbenzoate avec un groupe protecteur Q approprié pour modifier la réactivité du système cyclique : 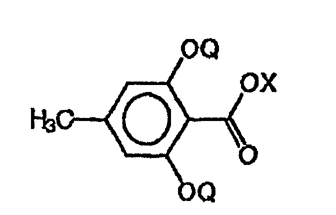 où le groupe X est un groupe alkyle ayant environ 1 à 3 atomes de carbone,
l'halogénation du 2,6-dihydroxy-4-méthylbenzoate pour donner un halogénure de benzyle dans lequel le groupe Y est un halogène : 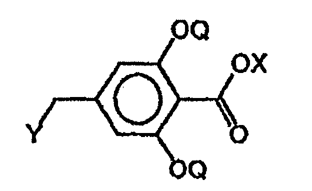
l'alkylation de l'halogénure de benzyle avec un sel consistant en un azoture pour donner l'azoture de benzyle : 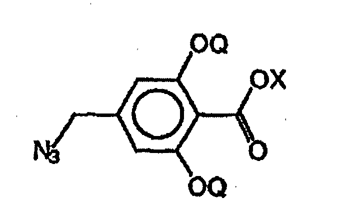
l'hydrogénation de l'azoture de benzyle et l'élimination des groupes protecteurs Q pour donner une benzylamine : 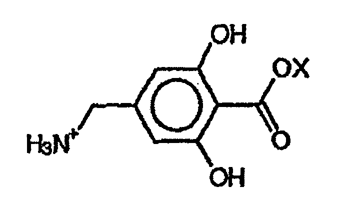
la condensation de la benzylamine avec un groupe protecteur Q' approprié pour protéger le groupement amine d'une participation aux réactions ultérieures, pour donner un aminométhylbenzoate d'alkyle : 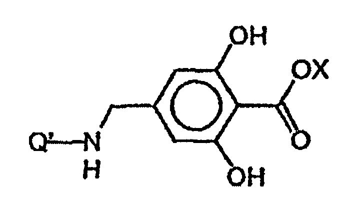
l'hydrolyse de l'alkylester pour donner un acide benzoïque : 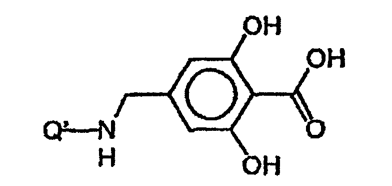
l'alkylation de l'acide benzoïque pour donner un ester : 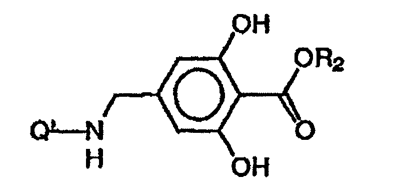 où le groupe R₂ est choisi entre un groupe alkyle et un groupe méthylène portant un groupement ayant un moment dipolaire négatif,
l'élimination du groupe protecteur Q' pour donner une benzylamine : 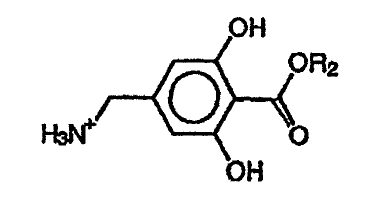
la condensation de la benzylamine avec un acide carboxylique activé pour donner le réactif.

16. Procédé selon la revendication 15, dans lequel le groupe R₂ est choisi entre l'un des groupes suivants : CH₃, CH₂CH₃, CH₂CN, CH₂COOH, CH₂CONH₂ et CH₂OCH₃.

17. Procédé selon la revendication 17, dans lequel le groupe protecteur Q est un groupe acétyle.

18. Procédé selon la revendication 15, dans lequel le groupe protecteur Q' est un groupe N-(tert-butoxycarbonyle).

19. Procédé selon la revendication 15, comprenant l'étape de condensation du réactif de Formule Générale CI selon la revendication 1 avec une entité bioactive pour donner le réactif de Formule Générale CII: 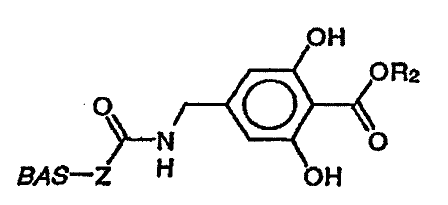 dans laquelle *BAS* est une entité biologiquement active.

20. Réactif répondant à la formule générale de Formule Générale CIII : 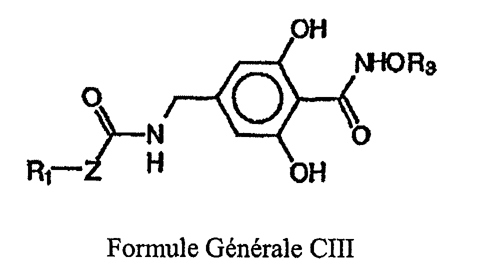 dans laquelle le groupe Z comprend un espaceur choisi entre un espaceur saturé ou insaturé ayant jusqu'à 6 équivalents carbonés en longueur, une chaîne non ramifiée saturée ou insaturée ayant environ 6 à 18 équivalents carbonés en longueur, avec au moins un des groupements amide et disulfure intermédiaires, et une chaîne polyéthylèneglycol d'environ 3 à 12 équivalents carbonés en longueur ;
dans laquelle le groupe R₃ est choisi entre H, un groupe alkyle et un groupement méthylène ou éthylène portant un substituant ayant un moment dipolaire négatif ; et
dans laquelle le groupe R₁ est un groupement électrophile ou nucléophile approprié à une réaction du réactif avec une entité bioactive choisie dans le groupe constitué par des groupements acrylamide, amino, bromo, dithiopyridyle, bromoacétamide, hydrazide, ester de N-hydroxysuccinimide, ester de N-hydroxysulfosuccinimide, ester imidate, imidazolide, iodo, iodoacétamide, maléimide et thiol.

21. Réactif selon la revendication 20, dans lequel le groupe R₃ est de préférence choisi entre H, des groupes CH₃, CH₂CH₃, CH₂CN, CH₂COOH, CH₂CONH₂, CH₂CH₂OH et CH₂OCH₃.

22. Réactif selon la revendication 20, dans lequel le groupe Z est de préférence une chaîne alkyle non ramifiée de formule générale (CH₂)ₙ, dans laquelle n = 1 à 6.

23. Réactif selon la revendication 20, dans lequel R₁ est un groupement éthylène répondant à la formule générale :

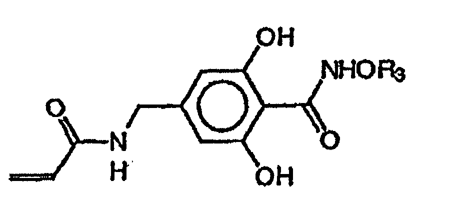

24. Conjugué d'un réactif selon la revendication 20, ledit conjugué répondant à la formule générale de Formule Générale CIV : 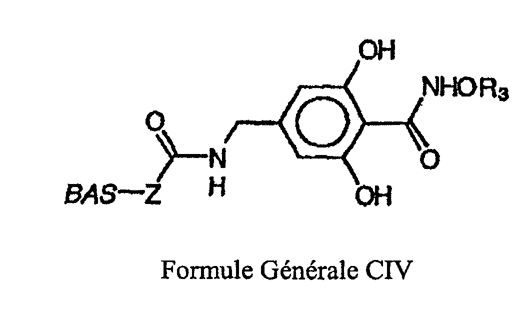 dans laquelle *BAS* est une entité biologiquement active.

25. Conjugué selon la revendication 24, dans lequel le groupe R₃ est de préférence choisi entre H et l'un des groupes suivants : CH₃, CH₂CH₃, CH₂CN, CH₂COOH, CH₂CONH₂, CH₂CH₂OH et CH₂OCH₃.

26. Conjugué selon la revendication 24, dans lequel le groupe Z est de préférence une chaîne alkyle non ramifiée de formule générale (CH₂)ₙ, dans laquelle n = 1 à 6.

27. Conjugué selon la revendication 24, répondant à la formule générale : 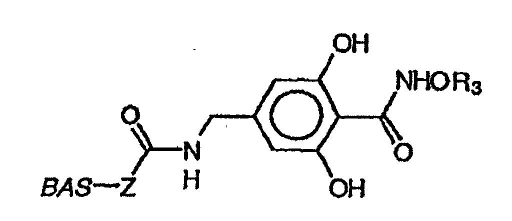 dans laquelle le groupe R₃ représente H.

28. Bioconjugué du conjugué selon la revendication 24, répondant à la formule générale de Formule Générale CVI : 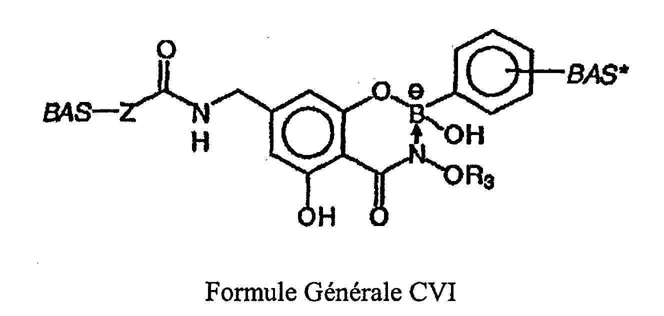 dans laquelle *BAS** est une entité biologiquement active.

29. Bioconjugué selon la revendication 28, dans lequel le groupe R₃ est de préférence choisi entre H et l'un des groupes suivants : CH₃, CH₂CH₃, CH₂CN, CH₂COOH, CH₂CONH₂, CH₂CH₂OH et CH₂OCH₃.

30. Bioconjugué selon la revendication 28, dans lequel le groupe Z est de préférence une chaîne alkyle non ramifiée de formule générale (CH₂)ₙ, dans laquelle n = 1 à 6.

31. Bioconjugué selon la revendication 28, dans lequel *BAS* et *BAS** sont des entités bioactives différentes.

32. Procédé de préparation du réactif de Formule Générale CIV selon la revendication 24, comprenant l'étape de condensation du réactif de Formule Générale CIII selon la revendication 20 avec une entité bioactive pour donner le réactif de Formule Générale CIV.

33. Procédé selon la revendication 32, dans lequel le groupe R₃ est de préférence choisi entre H et l'un des groupes suivants : CH₃, CH₂CH₃, CH₂CN, CH₂COOH, CH₂CONH₂, CH₂CH₂OH et CH₂OCH₃.

34. Procédé selon la revendication 32, dans lequel le groupe Z est de préférence une chaîne alkyle non ramifiée de formule générale (CH₂)ₙ, dans laquelle n = 1 à 6.

35. Procédé de préparation du bioconjugué de Formule Générale CVI selon la revendication 28,
ledit procédé comprenant l'étape consistant à complexer le conjugué selon la revendication 6 avec un second conjugué comprenant une deuxième entité biologiquement active et un acide phénylborique.

36. Procédé selon la revendication 35, dans lequel le groupe R₃ est de préférence choisi entre H et l'un des groupes suivants : CH₃, CH₂CH₃, CH₂CN, CH₂COOH, CH₂CONH₂, CH₂CH₂OH et CH₂OCH₃.

37. Procédé selon la revendication 35, dans lequel le groupe Z est de préférence une chaîne alkyle non ramifiée de formule générale (CH₂)ₙ, dans laquelle n = 1-6.

38. Procédé selon la revendication 35, dans lequel *BAS* et *BAS** sont des entités bioactives différentes.

39. Procédé de préparation d'un conjugué selon la revendication 24 répondant à la formule générale de Formule Générale CIV,
le procédé comprenant :
la condensation d'un réactif de formule générale : 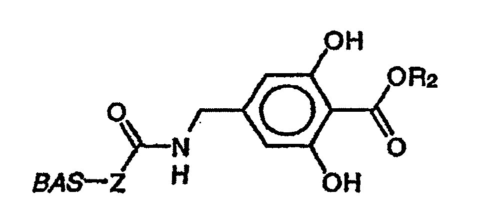 avec un dérivé d'hydroxylamine de formule générale NH₂OR₃, dans laquelle le groupe R₂ est choisi entre un groupe alkyle et un groupe méthylène portant un groupement ayant un moment dipolaire négatif.

40. Procédé selon la revendication 39, dans lequel le groupe R₃ est de préférence choisi entre H et l'un des radicaux suivants : CH₃, CH₂CH₃, CH₂CN, CH₂COOH, CH₂CONH₂, CH₂CH₂OH et CH₂OCH₃.

41. Procédé selon la revendication 39, dans lequel le groupe Z est de préférence une chaîne alkyle non ramifiée de formule générale (CH₂)ₙ, dans laquelle n = 1 à 6.

42. Composé comprenant : 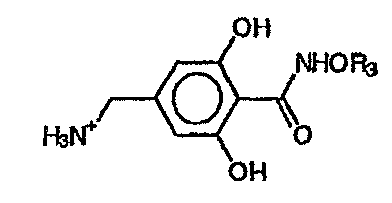 où le groupe R₃ est choisi entre H, un groupe alkyle et un groupe alkyle ou méthylène portant un groupement ayant un moment dipolaire négatif.

43. Procédé de préparation du composé selon la revendication 42, le procédé comprenant :
la condensation du 2,6-dihydroxy-4-méthylbenzoate de méthyle de formule générale : 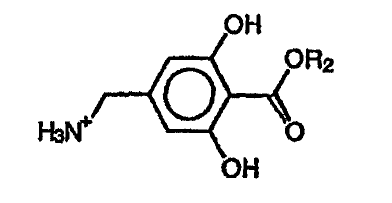 dans laquelle R₂ est un groupe alkyle, avec un groupe Q approprié pour protéger le groupement amine : 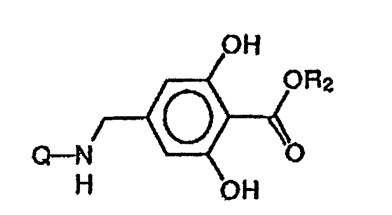
la condensation de l'ester benzoate avec des groupes Q' appropriés pour réduire la réactivité du système cyclique : 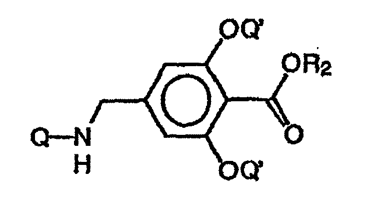
l'hydrolyse de l'ester benzoate pour donner l'acide salicylique : 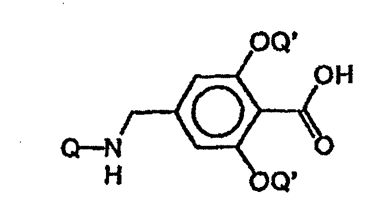
la condensation de l'acide avec un dérivé d'hydroxylamine de formule générale NH₂OR₃, dans laquelle R₃ est choisi entre H, un groupe alkyle, et un groupe méthylène ou éthylène portant un groupement ayant un moment dipolaire négatif : 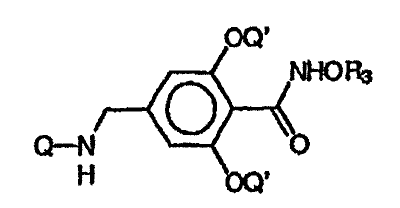
et l'élimination des groupes protecteurs Q et Q'.
